# EUROPEAN PATENT APPLICATION

(11) **EP 4 693 301 A2**
(43) Date of publication of application: **11.02.2026**
(21) Application number: 25224089.0
(22) Date of filing: 23.12.2022
(51) Int. Cl.: G16B 40/20

(54) **MACHINE LEARNING MODEL FOR RECALIBRATING NUCLEOTIDE BASE CALLS CORRESPONDING TO TARGET VARIANTS**

(30) Priority: 28.12.2021 US 202117563934
(62) Divisional of application: 22857067.7
(71) Applicant: ILLUMINA, INC., San Diego, CA 92122 (US)
(72) Inventor: PARNABY, Gavin, San Diego, 92122 (US)
(74) Representative: Robinson, David Edward Ashdown

(57) **Abstract**

This disclosure describes methods, non-transitory computer readable media, and systems that can utilize a machine learning model to recalibrate nucleotide base calls (e.g., variant calls) of a call generation model. For instance, the disclosed systems can train and utilize a call recalibration machine learning model to generate a set of predicted variant call classifications based on sequencing metrics associated with a sample nucleotide sequence. Leveraging the set of variant call classifications, the disclosed systems can further update or modify nucleotide base calls (e.g., variant calls) corresponding to genomic coordinates, such as multiallelic genomic coordinates, haploid genomic coordinates, and genomic coordinates indicated (by the call generation model) to exhibit homozygous reference genotypes.

## Description

### CROSS-REFERENCE TO RELATED APPLICATIONS

This application claims the benefit of, and priority to, U.S. Application No. 17/563,934, entitled "MACHINE-LEARNING MODEL FOR RECALIBRATING NUCLEOTIDE BASE CALLS CORRESPONDING TO TARGET VARIANTS," filed December 28, 2021, the contents of which are hereby incorporated by reference in their entirety.

### BACKGROUND

In recent years, biotechnology firms and research institutions have improved hardware and software for sequencing nucleotides and determining nucleotide base calls (e.g., variant calls) for genomic samples. For instance, some existing nucleotide base sequencing platforms determine individual nucleotide bases within sequences by using conventional Sanger sequencing or by using sequencing-by-synthesis (SBS) methods. When using SBS, existing platforms can monitor many thousands of nucleic acid polymers being synthesized in parallel to predict nucleotide base calls from a larger base call dataset. For instance, a camera in many SBS platforms captures images of irradiated fluorescent tags incorporated into oligonucleotides for determining the nucleotide base calls. After capturing such images, existing SBS platforms send base call data (or image data) to a computing device to apply sequencing data analysis software that determines a nucleotide base sequence for a nucleic acid polymer. In certain cases, some prior systems further utilize a variant caller to identify variants, such as single nucleotide polymorphisms (SNPs), insertions or deletions (indels), or other variants within a sample's nucleic acid sequence.

Despite these recent advances in sequencing and variant calling, existing nucleotide base sequencing platforms and sequencing data analysis software (together and hereinafter, existing sequencing systems) often include variant callers that inaccurately determine nucleotide base calls (and/or corresponding variant calls). For example, existing sequencing systems either inaccurately determine-or are incapable of determining-nucleotide base calls for multiallelic genomic coordinates. Indeed, for regions of a nucleotide sequence, such as multiallelic regions, that are more challenging than biallelic regions, some existing systems struggle to (or cannot) accurately determine genotypes when alleles cover or correspond to a given genomic coordinate. For instance, some machine learning based sequencing systems struggle to determine genotypes for multiallelic coordinates because training data is largely biallelic data. Thus, in the case of a pileup or a large insertion, existing sequencing systems often fail to correctly determine nucleotide base calls and/or a genotype from multiple possible alleles at the given genomic coordinate.

In addition, existing sequencing systems inaccurately determine nucleotide base calls (e.g., variant calls) for haploid genomic coordinates within a genomic sample or other nucleotide sequence. For instance, many existing sequencing systems inaccurately determine nucleotide base calls within sex chromosomes, often due to the sparsity or complete lack of good haploid training data. Specifically, existing sequencing systems often learn parameters for determining nucleotide base calls exclusively from unmodified diploid data (e.g., PrecisionFDA truth data from the PrecisionFDA Truth Challenge, described at https://precision.fda.gov/challenges/truth) and lack models or training to identify nucleotide bases or genotypes for coordinates other than diploid coordinates. Consequently, many of these existing sequencing systems cannot accurately determine nucleotide base calls or variant calls for haploid genomic coordinates.

Further, in some circumstances, existing sequencing systems apply a variant caller that inaccurately identifies excessive numbers of false negative variant calls. For instance, existing sequencing systems sometimes determine a genomic coordinate exhibits a homozygous reference genotype (and therefore not include a variant) when, in fact, the coordinate includes a variant. Indeed, existing variant callers achieve a certain level of accuracy but, due to their limitations, still leave room for improvement in recovering false negative variant calls. To illustrate the impact of such inaccuracy, a variant call identifying a particular single nucleotide polymorphism (SNP) in the hemoglobin beta (*HBB)* gene can have significant implications. When a variant caller identifies an SNP at rs344 on chromosome 11, for instance, the variant caller can either correctly identify the genetic cause of sickle cell anemia or miss the cause of the disease. As a further example, a variant call that correctly or incorrectly identifies the deletion of one or more copies of hemoglobin subunit alpha 1 (*HbA1*) or hemoglobin subunit alpha 2 (*HbA2*) genes can result in either correctly identifying a genetic cause of an inherited blood disorder or miss the gene deletion entirely.

As a contributing factor to the aforementioned inaccuracies, many existing sequencing systems leverage only limited sets of data in determining nucleotide base calls. For instance, existing sequencing systems frequently rely exclusively on information extracted directly from nucleotide reads of a sample sequence, such as read depth, mismatch counts, sequence alignment scores, and mapping quality, to determine nucleotide base calls. While sequence information from nucleotide reads can provide valuable insight for determining nucleotide base calls, existing sequencing systems that solely rely on these data can underperform when determining nucleotide base calls. Indeed, some existing sequencing systems that rely on raw sequence data incorrectly determine SNPs, indels, or other variants in a genomic sample sequence in comparison to more complex models. Indeed, existing sequencing systems frequently identify false negative variants or false positive variants in the Truth Challenges of the U.S. Food and Drug Administration (FDA), and reliable haploid data is often difficult to acquire for testing or training a variant caller.

**In** addition to inaccurately determining variant calls, some existing sequencing systems also inefficiently expend computing resources with overly complex models. Specifically, the variant callers of some existing sequencing systems are computationally expensive and slow. Indeed, some existing sequencing systems utilize variant callers with a deep learning architecture or some other neural network architecture that require extensive computational resources (e.g., computing time, processing power, and memory) to train and apply. For example, some existing sequencing systems utilize deep learning architectures that, even after training, take many hours across multiple computing devices to generate nucleotide base calls for a single sample sequence.

As an added drawback of existing sequencing systems with complex networks, many such systems utilize model architectures that render sequence data uninterpretable. More specifically, some existing deep neural networks transform and manipulate the sequence data many times over, changing from one vector to another across the various layers and neurons, as the basis for generating a variant call. In many cases, the internal data of these deep neural networks is uninterpretable and impossible to utilize in any way outside of the neural network architecture itself.

### SUMMARY

This disclosure describes embodiments of methods, non-transitory computer readable media, and systems that can utilize a machine learning model to recalibrate nucleotide base calls (e.g., variant calls) of a call generation model. For example, the disclosed systems can train and utilize a call recalibration machine learning model to generate a set of classification predictions (e.g., variant call classifications) to improve nucleotide base calls in specific scenarios, such as generating nucleotide base calls for multiallelic coordinates, haploid coordinates, and/or coordinates incorrectly identified by existing sequencing systems as exhibiting homozygous reference genotypes. As disclosed, the disclosed systems can (i) determine sequencing metrics for a particular genomic coordinate, such as a multiallelic coordinate, a haploid coordinate, or an incorrectly identified homozygous reference coordinate and (ii) utilize a call recalibration machine learning model to generate classification predictions for updating or recalibrating an initial nucleotide base call for the genomic coordinate. After recalibrating, the disclosed systems can output the updated or recalibrated nucleotide base call as a final nucleotide base call (e.g., a final variant call) in a variant call file or other base call output file.

By utilizing a call recalibration machine learning model to update sequencing metrics for generating nucleotide base calls, the disclosed systems can improve accuracy, efficiency, and speed over existing sequencing systems. As described further below, for instance, the disclosed call recalibration machine learning model determines variant calls with better accuracy than conventional hidden Markov model (HMM)-based or probabilistic-based variant callers and more complex neural networks (e.g., deep neural network-base variant callers) for variant calling at a multiallelic coordinate, a haploid coordinate, or an incorrectly identified homozygous reference coordinate. The disclosed call recalibration machine learning model also determines variant calls at such genomic coordinates with faster computing times than complex neural networks. Additionally, the disclosed systems can improve interpretability of factors impacting accurate variant calls at such genomic coordinates in comparison to complex neural networks by utilizing a call recalibration machine learning model that processes data in an accessible, interpretable format. Indeed, because of the improved interpretability of the disclosed systems, in some embodiments, the disclosed systems can generate and provide a visualization of various contribution measures associated with individual sequencing metrics to visually depict respective measures of impact that the sequencing metrics have on a resultant nucleotide base call.

### BRIEF DESCRIPTION OF THE DRAWINGS

The detailed description refers to the drawings briefly described below.
FIG. 1 illustrates a block diagram of a sequencing system including a call recalibration system in accordance with one or more embodiments.
FIG. 2 illustrates an overview of the call recalibration system generating a nucleotide base call utilizing the call recalibration system in accordance with one or more embodiments.
FIGS. 3A-3B illustrate the call recalibration system generating nucleotide base calls for multiallelic genomic coordinates in accordance with one or more embodiments.
FIGS. 4A-4B illustrate the call recalibration system generating nucleotide base calls for haploid genomic coordinates in accordance with one or more embodiments.
FIG. 5 illustrates the call recalibration system generating variant calls for homozygous reference genomic coordinates in accordance with one or more embodiments.
FIGS. 6A-6C illustrate the call recalibration system generating or determining sequencing metrics in accordance with one or more embodiments.
FIG. 7 illustrates the call recalibration system generating variant call classifications and recalibrating a nucleotide base call utilizing a call recalibration machine learning model in accordance with one or more embodiments.
FIG. 8 illustrates an example process for the call recalibration system training a call recalibration machine learning model in accordance with one or more embodiments.
FIG. 9 illustrates an example contribution-measure interface displayed on a client device in accordance with one or more embodiments.
FIGS. 10A-10B illustrate graphs and tables depicting accuracy improvements associated with the call recalibration system for diploid coordinates in accordance with one or more embodiments.
FIGS. 11A-11B illustrate a graphs and tables depicting accuracy improvements associated with the call recalibration system for haploid coordinates in accordance with one or more embodiments.
FIG. 12 illustrates a flowchart of a series of acts for generating nucleotide base calls associated with multiallelic genomic coordinates in accordance with one or more embodiments.
FIG. 13 illustrates a flowchart of a series of acts for generating nucleotide base calls associated with haploid genomic coordinates in accordance with one or more embodiments.
FIG. 14 illustrates a flowchart of a series of acts for generating variant calls associated with homozygous reference genomic coordinates in accordance with one or more embodiments.
FIG. 15 illustrates a block diagram of an example computing device for implementing one or more embodiments of the present disclosure.

### DETAILED DESCRIPTION

This disclosure describes embodiments of a call recalibration system that generates and recalibrates nucleotide base calls (e.g., variant calls) for a sample nucleotide sequence utilizing a call recalibration machine learning model. In particular, the call recalibration system can utilize a call recalibration machine learning model to update, recalibrate, or modify an initial nucleotide base call generated by a call generation model. For example, the call recalibration system can recalibrate the initial nucleotide base call to improve its accuracy by utilizing a call recalibration machine learning model to update various call metrics, such as a call quality, a genotype associated with the call, a genotype quality associated with the genotype, Phred-scaled Likelihood (PL), and/or other metrics with corresponding fields. By utilizing the call recalibration machine learning model to update metrics, the call recalibration system can improve the accuracy of nucleotide base calls at particular genomic coordinates, such as multiallelic coordinates, haploid coordinates, and coordinates falsely determined (in an initial call or by an existing sequencing system) to exhibit homozygous reference genotypes.

As just mentioned, in certain implementations, the call recalibration system improves nucleotide base calls and corresponding variant calls for multiallelic coordinates of a sample nucleotide sequence. To facilitate generating multiallelic nucleotide base calls, in some embodiments, the call recalibration system utilizes a call recalibration machine learning model that is specialized and adaptable to generate nucleotide base calls for both biallelic and multiallelic coordinates. For instance, the call recalibration system can generate, from sequencing metrics associated with a multiallelic genomic coordinate, a set of variant call classifications that includes a probability of a homozygous reference genotype at the multiallelic genomic coordinate (i.e., a reference probability), a probability of a genotype error at the multiallelic genomic coordinate (i.e., a differing genotype probability), and a probability of a correct variant call genotype at the multiallelic genomic coordinate (i.e., a correct variant probability). The call recalibration system can further determine a final nucleotide base call for the multiallelic genomic coordinate from the set of variant call classifications. Additional detail regarding generating calls for multiallelic coordinates is provided below with reference to the figures.

As mentioned, in one or more embodiments, the call recalibration system improves nucleotide base calls and corresponding variant calls for haploid genomic coordinates of a sample nucleotide sequence. In particular, the call recalibration system can utilize a call recalibration machine learning model adapted to determine haploid genotypes based on diploid data. For instance, the call recalibration system can train a call recalibration machine learning model by modifying diploid data (e.g., diploid sequencing metrics) to simulate haploid data (e.g., haploid sequencing metrics). In addition, the call recalibration system can utilize the trained call recalibration machine learning model to generate three outputs for a given genomic coordinate: (i) a first confidence score for a homozygous reference genotype (0/0), (ii) a second confidence score for a heterozygous genotype (0/1), and (iii) a third confidence score for a homozygous alternate genotype (1/1).

The call recalibration system can further prune or remove the second confidence score (e.g., the 0/1 confidence score) and can utilize a softmax model or layer to normalize across the other two confidence scores and convert the confidence scores to haploid probabilities. Utilizing the softmax model or layer, the call recalibration system can thus determine: (i) from the homozygous reference confidence score (0/0), a haploid reference probability (0) and (ii) from the homozygous alternate confidence score (1/1), a haploid alternate probability (1). Additional detail regarding generating calls for haploid coordinates is provided below with reference to the figures.

As further mentioned above, the call recalibration system improves nucleotide base calls and corresponding variant calls for genomic coordinates of a sample nucleotide sequence that are determined to exhibit homozygous reference genotypes. More specifically, the call recalibration system can recover false negative variant calls for genomic coordinates that are initially determined as exhibiting homozygous reference genotypes (e.g., as determined by a call generation model) when, in fact, the genotypes of these coordinates are not homozygous with respect to the reference sequence. As opposed to existing sequencing systems that filter out data associated with homozygous reference coordinates, the call recalibration system can determine sequencing metrics for such homozygous reference coordinates and can utilize a call recalibration machine learning model to generate variant call classifications from the sequencing metrics. Further, the call recalibration system can generate final nucleotide base calls for the homozygous reference coordinates based on the variant call classifications, changing a variant call that would have indicated a homozygous reference genotype to indicating a different genotype (and thereby recovering false negative variant calls). Additional detail regarding correcting or updating variant calls for genomic coordinates that would have been incorrectly identified as exhibiting homozygous reference genotypes is provided below with reference to the figures.

As mentioned above, in some embodiments, the call recalibration system can more generally utilize a machine learning model to generate variant call classifications based on sequencing metrics for nucleotide base calls corresponding to genomic coordinates. To generate such classifications, the call recalibration system extracts or determines sequencing metrics from a sample nucleotide sequence. For example, the call recalibration system determines sequencing metrics from nucleotide base calls of nucleotide reads from a sample nucleotide sequence. Indeed, in some cases, the call recalibration system generates or determines a set of initial nucleotide base calls from nucleotide reads captured or determined via fluorescent imaging of a sample nucleotide sequence (e.g., at a particular genomic coordinate). From the read-based nucleotide base calls, in some embodiments, the call recalibration system determines or extracts various sequencing metrics (e.g., sequencing metrics of various types obtained from reads and/or from different components of a call generation model).

To elaborate, in certain implementations, the call recalibration system determines different types of sequencing metrics associated with different sources. For example, the call recalibration system determines read-based sequencing metrics including metrics derived from nucleotide reads of the sample nucleotide sequence. In addition, the call recalibration system determines externally sourced sequencing metrics identified from one or more external databases that indicate various nucleotide attributes, mapping challenges, and genomic sequences associated with sequencing biases. Further, the call recalibration system determines call model generated sequencing metrics generated via a variant caller or other call generation model, such as variables internal to the call recalibration system that are not accessible to other systems or parties (e.g., proprietary quality scores, base contexts, read filtering, proprietary hypothesis scores, and other metrics). Indeed, in some cases, the call recalibration system determines call model generated sequencing metrics in the form of variant calling sequencing metrics and mapping-and-alignment sequencing metrics, where each type is extracted by different components of the call generation model.

As further mentioned, in certain implementations, the call recalibration system generates a set of predicted classifications from the sequencing metrics for modifying or improving a nucleotide base call or variant call data or fields associated with a nucleotide base call. More specifically, the call recalibration system utilizes a call recalibration machine learning model to generate, from the sequencing metrics, a set of three variant call classifications that impact or reflect the accuracy of identifying a variant at a particular genomic coordinate (e.g., a genomic coordinate corresponding to nucleotide base calls of nucleotide reads from a sample nucleotide sequence). Depending on the circumstances, the call recalibration system can utilize the call recalibration machine learning model to, for example, generate different variant call classifications for multiallelic coordinates than for haploid coordinates or would-be-false homozygous reference coordinates.

For instance, when generating variant call classifications for a multiallelic genomic coordinate, the call recalibration system can utilize the call recalibration machine learning model to generate a set including: (i) a reference probability of a homozygous reference genotype at the multiallelic genomic coordinate, (ii) a differing genotype probability of a genotype error at the multiallelic genomic coordinate, and (iii) a correct variant probability of a correct variant call genotype at the multiallelic genomic coordinate. As another example, for haploid coordinates, the call recalibration system can utilize the call recalibration machine learning model to generate a set of variant call classifications including: (i) a first genotype probability of a first genotype at the genomic coordinate and (ii) a second genotype probability of a second genotype at the genomic coordinate. Further, for would-be homozygous reference coordinates, the call recalibration system can utilize the call recalibration machine learning model to generate a set of variant call classifications including: (i) a false positive classification (e.g., a probability that a nucleotide base call is a false positive variant), (ii) a genotype error classification (e.g., a heterozygous genotype classification indicating a probability of identifying a correct alt allele but with a genotype error-e.g., 0/1 instead of 1/1 or 1/1 instead of 0/1- or a probability of incorrectly identifying a genotype of a nucleotide base call), and a (iii) true-positive classification (e.g., homozygous alternate classification indicating a probability that a nucleotide base call or a genotype call is a true positive variant). In some cases, the variant call classifications accordingly represent intermediate scoring metrics associated with a variant caller.

From the variant call classifications, the call recalibration system can further modify or update metrics for one or more final nucleotide base calls for a genomic coordinate (e.g., final nucleotide base calls that indicates a variant call or a non-variant call). For example, the call recalibration system utilizes the variant call classifications to update data fields within a digital call file (e.g., a variant call format file or other base call output file) that indicates or represents a final nucleotide base call and/or a variant call. Indeed, as mentioned above, in some embodiments, the call recalibration system utilizes a call generation model to generate or determine a final nucleotide base call from the sequencing metrics for the genomic coordinate.

Additionally, the call recalibration system can utilize the variant call classifications to update a nucleotide base call and/or a variant call for improved accuracy. In certain implementations, the call recalibration system updates nucleotide base calls for specific genomic coordinates, such as multiallelic genomic coordinates, haploid genomic coordinates, and/or would-be falsely identified homozygous reference coordinates (i.e., genomic coordinates that previously or would have been falsely identified by a variant caller to exhibit homozygous reference genotypes). Indeed, in some embodiments, the call recalibration system utilizes (i) the call generation model to generate an initial nucleotide base call and (ii) the call recalibration machine learning model to modify data fields corresponding to a variant call file for the nucleotide base call. In some cases, the call recalibration system further modifies the nucleotide base call based on one or more of the data fields and generates a variant call file with the modified nucleotide base call. In certain embodiments, the call recalibration system can generate the variant call classifications utilizing the call recalibration machine learning model while also utilizing the call generation model to generate the nucleotide base call based on the variant call classifications.

By contrast, in some cases, the call recalibration system determines a final nucleotide base call or a variant call for a genomic coordinate based on both sequencing metrics for a call generation model and variant call classifications from the call recalibration machine learning model-without an initial nucleotide base call (e.g., an initial variant call) from the call generation model. For example, the call generation model may not output an initial nucleotide base call, but may instead evaluate the genomic coordinate and generate sequencing metrics that the call recalibration machine learning model can then use to generate a variant call in combination with the call generation model. In some embodiments, the call generation model may output a final variant call that accounts for the variant call classifications from the call recalibration machine learning model (without generating an initial variant call that is updated). By contrast, in certain cases, the call generation model may initially determine a confidence or quality corresponding to a potential variant call fails to satisfy a threshold for including in a variant call file but (after accounting for variant call classifications that updates a base call quality metric) determine to include a variant call in the variant call file. As a result of implementing the call recalibration machine learning model and the call generation model in this way, the call recalibration system recovers false negative calls, fixes variant genotype errors, and/or removes false positive calls initially made by the call generation model.

In one or more embodiments, the call recalibration system further determines contribution measures associated with one or more of the sequencing metrics. In particular, the call recalibration system determines measures of impact or influence that each sequencing metric or a subset of sequencing metrics has on a final nucleotide base call. For example, some metrics may be more heavily weighted than others in determining a call at one genomic coordinate versus another. Indeed, due to the accessibility and interpretability of the call generation model and the call recalibration machine learning model, the call recalibration system can access internal sequencing metrics used to generate a nucleotide base call and can determine their respective contribution measures in ultimately determining which metrics are causing or driving the recalibration of the final nucleotide base calls (or variant calls). In some cases, the call recalibration system further generates and provides a visualization of the contribution measures for display on a client device.

As suggested above, the call recalibration system provide several advantages, benefits, and/or improvements over existing sequencing systems, including variant callers and other sequencing data analysis software. For instance, the call recalibration system generates more accurate nucleotide base calls and/or variant calls than existing sequencing systems. While some existing sequencing systems are either incapable of generating, or inaccurately generating, nucleotide base calls for multiallelic coordinates, in some embodiments, the call recalibration system generates more accurate calls for multiallelic genomic coordinates. Specifically, the call recalibration system can utilize or adapt a call recalibration machine learning model with parameters trained or tuned to generate a set of variant call classifications specific to multiallelic genomic coordinates. From the set of variant call classifications, the call recalibration system can further generate one or more final nucleotide base calls for a multiallelic genomic coordinate to indicate a genotype of the multiallelic coordinate, indicate whether the genotype is a variant with respect to a reference sequence, and/or indicate whether the genotype is correct (e.g., a genotype quality metric in GQ field indicating a likelihood or probability that a genotype is correct). Similarly, from the set of variant call classifications, the call recalibration system can also improve accuracy of quality fields and other fields, such as PL.

In some embodiments, the call recalibration system generates more accurate nucleotide base calls and/or variant calls for haploid coordinates of a sample nucleotide sequence, as compared to an existing sequencing system. Unlike some existing sequencing systems that cannot recalibrate nucleotide base calls for haploids, the call recalibration system can utilize a call recalibration machine learning model adaptable to haploid regions of a sample nucleotide sequence. In certain cases, the call recalibration system learns parameters for the call recalibration machine learning model by adapting diploid data to simulate haploid data. Additionally, the call recalibration system can generate nucleotide base calls for haploid coordinates by pruning, for a particular genomic coordinate, a particular machine learning output (e.g., confidence score) of the call recalibration machine learning model not pertinent to haploid calls and by normalizing across the remaining two outputs (e.g., confidence scores). By pruning and normalizing outputs compatible with diploid data to outputs compatible with haploid data, the call recalibration system can determine probabilities indicating a haploid reference genotype and a haploid alternate genotype at the coordinate.

In one or more embodiments, the call recalibration system generates more accurate nucleotide base calls and/or variant calls for (would-be-falsely identified) homozygous reference coordinates of a sample nucleotide sequence, as compared to an existing sequencing system. For instance, some existing sequencing systems generate an inordinate number of false negative variant calls by incorrectly identifying certain genomic coordinates as exhibiting homozygous reference genotypes when, in actuality, their genotypes are not homozygous reference. By contrast, the call recalibration system identifies fewer false negative variant calls (or recovers more false negative variant calls) by determining sequencing metrics for genomic coordinates indicated to exhibit homozygous reference genotypes and utilizing a call recalibration machine learning model to generate variant call classifications for these coordinates. The call recalibration system can further generate one or more final nucleotide base calls from the variant call classifications of the homozygous reference coordinates.

The call recalibration system improves upon the accuracies of existing sequencing systems (e.g., in each of the scenarios described above) by removing large numbers of false positive variant calls and/or recovering large numbers of false negative variant calls utilizing the call recalibration machine learning model. By editing an initial nucleotide base call or generating a final nucleotide base call based on variant call classifications from the call recalibration machine learning model, the call recalibration system can use unique machine learning outputs to recalibrate base calls with better accuracy than existing variant callers or existing machine learning models. For instance, the call recalibration system utilizes the call recalibration machine learning model to generate variant call classifications from both internal (e.g., proprietary and model-specific) and external sequencing metrics, which results in recovering variant nucleotide base calls that were previously filtered out and/or removing non-variant nucleotide base calls that were previously not filtered out.

To accomplish the aforementioned improved accuracies, as indicated, the call recalibration system utilizes an improved and unique machine learning model-the call recalibration machine learning model-that is trained to perform new applications. Unlike existing variant callers that generate nucleotide base calls from general sequencing data (without any particular emphasis on one genomic coordinate or another), the call recalibration system utilizes a unique call recalibration machine learning model that generates specific variant call classifications for specific scenarios, such as multiallelic genomic coordinates, haploid genomic coordinates, and false homozygous reference coordinates. In some cases, the call recalibration system utilizes the call recalibration machine learning model to update a nucleotide base call generated by a call generation model from the same (or a subset of the same) metrics used by the call recalibration machine learning model to generate the variant call classifications.

Contributing at least in part to the improved accuracy, the call recalibration system exhibits improved flexibility over existing sequencing systems. For example, while many existing sequencing systems are limited to application at certain genomic coordinates and/or are incompatible with other genomic coordinates, in some embodiments, the call recalibration system flexibly adapt to many of these previously incompatible coordinates. Specifically, unlike some existing sequencing systems, the call recalibration system can generate nucleotide base calls and/or variant calls for multiallelic genomic coordinates, haploid genomic coordinates, and false homozygous reference genomic coordinates.

As another example of improved flexibility, as mentioned above, existing sequencing systems sometimes utilize variant callers that rely exclusively on internal sequencing metrics for particular base calls to generate a nucleotide base call-without re-engineering or modifying such internal sequencing metrics or analyzing externally sourced sequencing metrics relevant to the genomic coordinates of corresponding nucleotide base calls. By contrast, in some embodiments, the call recalibration system generates and manipulates both external and internal sequencing metrics. Indeed, in some cases, the call recalibration system determines call model generated sequencing metrics from variant caller components and mapping-and-alignment components of a call generation model by combining Bayesian probabilistic models with machine learning techniques in an efficient manner. In addition, the call recalibration system utilizes a call recalibration machine learning model to generate an updated nucleotide base call (e.g., from variant call classifications) from one or more sequencing metrics.

In addition to improved accuracy and flexibility, in certain embodiments, the call recalibration system improves efficiency and speed. As noted above, some existing sequencing systems utilize computationally expensive, slow neural network architectures (e.g., deep learning architectures such as convolutional neural networks) that require many hours (e.g., 5-8 hours with multiple processors executing on a server) and large amounts of computational resources to even implement and generate a file with variant calls from a sequencing run. Such deep learning architectures can further require several days (or weeks) to train. Conversely, the call recalibration system utilizes comparatively lightweight, fast architectures for both the call generation model and the call recalibration machine learning model. Indeed, contrasting with the many hours across multiple processors required by existing sequencing systems, the call recalibration system, in many cases, requires under 30 minutes (for both the call generation model and the call recalibration machine learning model together) of runtime on a single field-programmable-gate array or a single processor to generate nucleotide base calls for a sample nucleotide sequence. Thus, the call recalibration system is far faster and less computationally expensive than many deep learning approaches to variant calling. Not only are the models of the call recalibration system faster and less computationally expensive to implement, but the models of the call recalibration system are also much faster and less computationally expensive to train than many existing deep-learning-based systems.

As part of the improved speed and efficiency, in some embodiments, the call recalibration system recalibrates nucleotide base calls on a call-by-call basis as each call is processed by the call generation model. Indeed, the call recalibration system can generate variant call classifications for recalibrating a nucleotide base call (e.g., utilize the call recalibration machine learning model) while also generating the nucleotide base call from the variant call classifications along with one or more sequencing metrics. In some embodiments, the call recalibration system utilizes the call generation model in parallel with the call recalibration machine learning model to contemporaneously generate an initial nucleotide base call and variant call classifications for modifying or recalibrating the initial nucleotide base call.

As a further advantage over existing sequencing systems, in certain implementations, the call recalibration system can identify or facilitate changes to individual metrics that affect the accuracy of nucleotide base calls. While the neural network architectures of many existing sequencing systems render any interpretation of internal model data impossible with latent features, the call recalibration system utilizes model architectures that facilitate interpretation of the effect of individual sequencing metrics. More specifically, in some cases, the call recalibration system utilizes a call generation model and a call recalibration machine learning model that enable extraction and analysis of individual sequencing metrics used throughout the process of generating a nucleotide base call. Indeed, the call recalibration system can determine respective contribution measures for sequencing metrics involved in determining a nucleotide base call at a particular genomic coordinate.

As suggested by the foregoing discussion, this disclosure utilizes a variety of terms to describe features and benefits of the call recalibration system. Additional detail is hereafter provided regarding the meaning of these terms as used in this disclosure. As used in this disclosure, for instance, the term "sample nucleotide sequence" or "sample sequence" refers to a sequence of nucleotides isolated or extracted from a sample organism (or a copy of such an isolated or extracted sequence). In particular, a sample nucleotide sequence includes a segment of a nucleic acid polymer that is isolated or extracted from a sample organism and composed of nitrogenous heterocyclic bases. For example, a sample nucleotide sequence can include a segment of deoxyribonucleic acid (DNA), ribonucleic acid (RNA), or other polymeric forms of nucleic acids or chimeric or hybrid forms of nucleic acids noted below. More specifically, in some cases, the sample nucleotide sequence is found in a sample prepared or isolated by a kit and received by a sequencing device.

As further used herein, the term "nucleotide base call" (or sometimes simply "call") refers to a determination or prediction of a particular nucleotide base (or nucleotide base pair) for a genomic coordinate of a sample genome or for an oligonucleotide during a sequencing cycle. In particular, a nucleotide base call can indicate (i) a determination or prediction of the type of nucleotide base that has been incorporated within an oligonucleotide on a nucleotide-sample slide (e.g., read-based nucleotide base calls) or (ii) a determination or prediction of the type of nucleotide base that is present at a genomic coordinate or region within a sample genome, including a variant call or a non-variant call in a digital output file. In some cases, for a nucleotide read, a nucleotide base call includes a determination or a prediction of a nucleotide base based on intensity values resulting from fluorescent-tagged nucleotides added to an oligonucleotide of a nucleotide-sample slide (e.g., in a well of a flow cell). Alternatively, a nucleotide base call includes a determination or a prediction of a nucleotide base to chromatogram peaks or electrical current changes resulting from nucleotides passing through a nanopore of a nucleotide-sample slide. By contrast, a nucleotide base call can also include an initial or final prediction of a nucleotide base at a genomic coordinate of a sample genome for a variant call file or other base call output file-based on nucleotide reads corresponding to the genomic coordinate. Accordingly, a nucleotide base call can include a base call corresponding to a genomic coordinate and a reference genome, such as an indication of a variant or a non-variant at a particular location corresponding to the reference genome. Indeed, a nucleotide base call can refer to a variant call, including but not limited to, a single nucleotide polymorphism (SNP), an insertion or a deletion (indel), or base call that is part of a structural variant. By using nucleotide base call, a sequencing system determines a sequence of a nucleic acid polymer. For example, a single nucleotide base call can comprise an adenine call, a cytosine call, a guanine call, or a thymine call for DNA (abbreviated as A, C, G, T) or a uracil call (instead of a thymine call) for RNA (abbreviated as U).

Relatedly, as used herein, the term "nucleotide read" refers to an inferred sequence of one or more nucleotide bases (or nucleotide base pairs) from all or part of a sample nucleotide sequence. In particular, a nucleotide read includes a determined or predicted sequence of nucleotide base calls for a nucleotide fragment (or group of monoclonal nucleotide fragments) from a sequencing library corresponding to a genome sample. For example, the call recalibration system determines a nucleotide read by generating nucleotide base calls for nucleotide bases passed through a nanopore of a nucleotide-sample slide, determined via fluorescent tagging, or determined from a well in a flow cell.

As noted above, in some embodiments, the call recalibration system determines sequencing metrics for nucleotide base calls of nucleotide reads. As used herein, the term "sequencing metric" refers to a quantitative measurement or score indicating a degree to which an individual nucleotide base call (or a sequence of nucleotide base calls) aligns, compares, or quantifies with respect to a genomic coordinate or genomic region of a reference genome, with respect to nucleotide base calls from nucleotide reads, or with respect to external genomic sequencing or genomic structure. For instance, a sequencing metric includes a quantitative measurement or score indicating a degree to which (i) individual nucleotide base calls align, map, or cover a genomic coordinate or reference base of a reference genome; (ii) nucleotide base calls compare to reference or alternative nucleotide reads in terms of mapping, mismatch, base call quality, or other raw sequencing metrics; or (iii) genomic coordinates or regions corresponding to nucleotide base calls demonstrate mappability, repetitive base call content, DNA structure, or other generalized metrics.

Relatedly, the term "diploid sequencing metric" refers to a sequencing metric determined for a nucleotide base call at a diploid genomic coordinate. For example, a diploid sequencing metric includes a sequencing metric for a particular genomic coordinate of a nucleotide sequence from (or is indicated to be from) a diploid chromosome or a diploid nucleotide sequence (e.g., with two alleles at genomic regions corresponding to the genomic coordinate). Additionally, the term "haploid sequencing metric" refers to a sequencing metric determined for a nucleotide base call at a haploid genomic coordinate. For example, a haploid sequencing metric includes a sequencing metric for a particular genomic coordinate of a nucleotide sequence from (or is indicated to be from) a haploid chromosome or a haploid nucleotide sequence (e.g., with a single allele at a genomic region corresponding to the genomic coordinate).

As further used herein, the term "genomic coordinate" (or sometimes simply "coordinate") refers to a particular location or position of a nucleotide base within a genome (e.g., an organism's genome or a reference genome). In some cases, a genomic coordinate includes an identifier for a particular chromosome of a genome and an identifier for a position of a nucleotide base within the particular chromosome. For instance, a genomic coordinate or coordinates may include a number, name, or other identifier for a chromosome (e.g., chrl or chrX) and a particular position or positions, such as numbered positions following the identifier for a chromosome (e.g., chr1:1234570 or chr1:1234570-1234870). Further, in certain implementations, a genomic coordinate refers to a source of a reference genome (e.g., mt for a mitochondrial DNA reference genome or SARS-CoV-2 for a reference genome for the SARS-CoV-2 virus) and a position of a nucleotide base within the source for the reference genome (e.g., mt:16568 or SARS-CoV-2:29001). By contrast, in certain cases, a genomic coordinate refers to a position of a nucleotide base within a reference genome without reference to a chromosome or source (e.g., 29727).

Relatedly, as used herein, the term "multiallelic genomic coordinate" refers to a genomic coordinate associated with three or more alleles. For example, a multiallelic genomic coordinate includes a genomic coordinate of a nucleotide sequence where nucleotide reads indicate three or more possible alleles corresponding to the coordinate, such as a reference allele, a first alternate allele, a second alternate allele, and so forth. In some cases, a multiallelic genomic coordinate corresponds to a genomic coordinate where a read pileup occurs or where an insertion occurs. For instance, a multiallelic genomic coordinate can exhibit a multiallelic genotype, such as a 1/2 genotype, where the first allele at the coordinate corresponds to an allele from a first alternate nucleotide sequence and the second allele corresponds to an allele from a second alternate nucleotide sequence.

As mentioned above, in some embodiments, the call recalibration system generates nucleotide base calls for haploid genomic coordinates, or genomic coordinates within a haploid nucleotide sequence. As used herein, the term "haploid nucleotide sequence" refers to a sequence of one or more nucleotide bases from a haploid chromosome (e.g., sex chromosome in males) or a single chromosome without a counterpart chromosome. For instance, a haploid nucleotide sequence can include a haploid region of a sample nucleotide sequence in which each of the genomic coordinate cover a nucleotide base from a haploid chromosome or a single chromosome without a counterpart chromosome. Thus, a haploid coordinate within a haploid nucleotide sequence has a haploid genotype, such as a haploid reference genotype (0) or a haploid alternate genotype (1).

Other coordinates within a nucleotide sequence can exhibit different genotypes. For example, a "homozygous reference genotype" refers to a genotype where both nucleotide bases at a given coordinate of a sample nucleotide sequence match a reference nucleotide base of a reference sequence or a reference genome (represented as 0/0). As another example, a "homozygous alternate genotype" refers to a genotype at a given coordinate where both nucleotide bases differ from a reference nucleotide base of a reference sequence or a reference genome (represented as 1/1). As a further example, a "heterozygous genotype" refers to a genotype where the nucleotide bases at a given coordinate are not the same. In some cases, a heterozygous genotype includes a genotype in which one nucleotide base matches a reference nucleotide base and the other nucleotide base differs from the reference nucleotide base (represented as 0/1 or 1/0). For multiallelic genomic coordinates, genotypes can exhibit nucleotide bases from more than one alternate nucleotide base differing from a reference nucleotide base of a reference genome. For instance, a multiallelic heterozygous genotype can be represented as 1/2, where one nucleotide base call matches a first alternate nucleotide base differing from a reference nucleotide base and the other nucleotide base call matches a second alternate nucleotide base differing from the reference nucleotide base.

As noted above, a genomic coordinate includes a position within a reference genome. Such a position may be within a particular reference genome. As used herein, the term "reference genome" refers to a digital nucleic acid sequence assembled as a representative example (or representative examples) of genes and other genetic sequences of an organism. Regardless of the sequence length, in some cases, a reference genome represents an example set of genes or a set of nucleic acid sequences in a digital nucleic acid sequenced determined by scientists as representative of an organism of a particular species. For example, a linear human reference genome may be GRCh38 or other versions of reference genomes from the Genome Reference Consortium. As a further example, a reference genome may include a reference graph genome that includes both a linear reference genome and paths representing nucleic acid sequences from ancestral haplotypes, such as Illumina DRAGEN Graph Reference Genome hg19.

In some embodiments, the call recalibration system determines various types of sequencing metrics from different sources, such as read-based sequencing metrics, externally sourced sequencing metrics, and call model generated sequencing metrics. As used herein, the term "read-based sequencing metrics" refers to sequencing metrics derived from nucleotide reads of a sample nucleotide sequence. For example, read-based sequencing metrics include sequencing metrics determined by applying statistical tests to detect differences between a reference sequence and nucleotide reads. For example, read-based sequencing metrics can include a comparative-mapping-quality-distribution metric that indicates a comparison between mapping qualities or a comparative-mismatch-count metric that indicates a comparison between mismatch counts.

By contrast, "externally sourced sequencing metrics" refer to sequencing metrics identified or obtained from one or more external databases. For example, externally sourced sequencing metrics include metrics relating to mappability of nucleotides, replication timing, or DNA structure that are available outside of the call recalibration system.

Further, "call model generated sequencing metrics" refer to internal, model-specific sequencing metrics generated or extracted by a call generation model. For example, call model generated sequencing metrics include variant calling sequencing metrics extracted or determined via variant caller components of a call generation model and mapping-and-alignment sequencing metrics extracted or determined via mapping-and-alignment components of a call generation model. As indicated above, call model generated sequencing metrics can include alignment metrics that quantify a degree to which sample nucleic acid sequences align with genomic coordinates of an example nucleic acid sequence, such as deletion-size metrics or mapping-quality metrics. Further, call model generated sequencing metrics can include depth metrics that quantify the depth of nucleotide base calls for sample nucleic acid sequences at genomic coordinates of an example nucleic acid sequence, such as forward-reverse-depth metrics or normalized-depth metrics. Call model generated sequencing metrics can also include call-quality metrics that quantify a quality or accuracy of nucleotide base calls, such as nucleotide base call quality metrics, callability metrics, or somatic-quality metrics.

As used herein, the term "base call quality metric" refers to a specific score or other measurement indicating an accuracy of a nucleotide base call. In particular, a base call quality metric comprises a value indicating a likelihood that one or more predicted nucleotide base calls for a genomic coordinate contain errors. For example, in certain implementations, a base call quality metric can comprise a Q score (e.g., a Phred quality score) predicting the error probability of any given nucleotide base call. To illustrate, a quality score (or Q score) may indicate that a probability of an incorrect nucleotide base call at a genomic coordinate is equal to 1 in 100 for a Q20 score, 1 in 1,000 for a Q30 score, 1 in 10,000 for a Q40 score, etc.

Relatedly, as used herein, the term "re-engineered sequencing metrics" refers to sequencing metrics that have been updated, modified, augmented, refined, or re-engineered to measure or compare nucleotide base calls (e.g., nucleotide base calls for reads or variant calls) with respect to other nucleotide base calls, a standard or reference, or for targeted for a particular objective or task. For example, re-engineered sequencing metrics can include modifications to, or combinations of, raw sequencing metrics. In some embodiments, for instance, the call recalibration system generates one or more of the read-based sequencing metrics, the externally sourced sequencing metrics, and/or the call model generated sequencing metrics as re-engineered sequencing metrics. In some cases, re-engineered sequencing metrics refer to sequencing metrics that are generated by the call recalibration system and are therefore proprietary or internal to the call recalibration system and not available to third-party systems. Example re-engineered sequencing metrics include a comparative-mapping-quality-distribution metric indicating a comparison between mapping quality distributions associated with a reference sequence and alternatives supporting nucleotide reads or a comparative-base-quality metric indicating comparisons between base qualities of a reference sequence and alternative supporting nucleotide reads.

As suggested above, the call recalibration system can utilize a machine learning model to modify sequencing metrics and update a nucleotide base call. As used herein, the term "machine learning model" refers to a computer algorithm or a collection of computer algorithms that automatically improve for a particular task through experience based on use of data. For example, a machine learning model can utilize one or more learning techniques to improve in accuracy and/or effectiveness. Example machine learning models include various types of decision trees, support vector machines, Bayesian networks, or neural networks. In some cases, the call recalibration machine learning model is a series of gradient boosted decision trees (e.g., XGBoost algorithm), while in other cases the call recalibration machine learning model is a random forest model, a multilayer perceptron, a linear regression, a support vector machine, a deep tabular learning architecture, a deep learning transformer (e.g., self-attention-based-tabular transformer), or a logistic regression.

In some cases, the call recalibration system utilizes a call recalibration machine learning model to modify or update a nucleotide base call based on sequencing metrics. As used herein, the term "call recalibration machine learning model" refers to a machine learning model that generates variant call classifications. For example, in some cases, the call recalibration machine learning model is trained to generate variant call classifications indicating various probabilities or predictions for variant calls based on the sequencing metrics. Accordingly, in some cases, a call recalibration machine learning model a variant call recalibration machine learning model. In certain embodiments, a call recalibration machine learning model includes multiple sub-models or operates in tandem with another call recalibration machine learning model. For instance, a first call recalibration machine learning model (e.g., an ensemble of gradient boosted trees) generates a first set of variant call classifications and a second call recalibration machine learning model (e.g., a random forest) generates a second set of variant call classifications.

Relatedly, the term "variant call classification" refers to a predicted classification from a call recalibration machine learning model that indicates a probability, score, or other quantitative measurement associated with some aspect of a nucleotide base call based on one or more sequencing metrics. A variant call classification can include a specialized prediction depending on the application of a call recalibration machine learning model. In embodiments for generating nucleotide base calls (or variant calls) for multiallelic genomic coordinates, variant call classifications can include: (i) a reference probability of a homozygous reference genotype at a multiallelic genomic coordinate, (ii) a differing genotype probability of a genotype error at a multiallelic genomic coordinate, and (iii) a correct variant probability of a correct variant call genotype at a multiallelic genomic coordinate.

In embodiments for generating nucleotide base calls (or variant calls) for a haploid genomic coordinate, variant call classifications can include: (i) a first genotype probability of a first genotype at the genomic coordinate and (ii) a second genotype probability of a second genotype at the genomic coordinate. As suggested above, the first genotype probability can be a probability that a genotype at a genotype coordinate is a haploid reference genotype, and the second genotype probability can be a probability that a genotype at the genotype coordinate is a haploid alternate genotype. In these or other embodiments, such as embodiments for generating nucleotide base calls (or variant calls) for genomic coordinates indicated to exhibit homozygous reference genotypes, variant call classifications can include: (i) a false positive classification or a homozygous reference classification indicating a probability that a nucleotide base call is a false positive or a homozygous reference genotype, respectively; (ii) a genotype error classification or a heterozygous genotype classification indicating a probability that a genotype (e.g., an indication of a heterozygous or homozygous genotype for a variant call at a particular location) is incorrect or a heterozygous genotype, respectively; and/or (iii) a true-positive classification or a homozygous alternate classification indicating a probability that a nucleotide base call is a true positive or a homozygous alternate genotype, respectively. In some cases, the variant call classifications accordingly represent intermediate scoring metrics and/or a predicted probability that a genotype for a nucleotide base call is accurate.

As mentioned, in some embodiments, the call recalibration machine learning model can be a neural network. The term the term "neural network" refers to a machine learning model that can be trained and/or tuned based on inputs to determine classifications or approximate unknown functions. For example, a neural network includes a model of interconnected artificial neurons (e.g., organized in layers) that communicate and learn to approximate complex functions and generate outputs (e.g., generated digital images) based on a plurality of inputs provided to the neural network. In some cases, a neural network refers to an algorithm (or set of algorithms) that implements deep learning techniques to model high-level abstractions in data. For example, a neural network can include a convolutional neural network, a recurrent neural network (e.g., an LSTM), a graph neural network, a self-attention transformer neural network, or a generative adversarial neural network.

As noted above, the call recalibration system can generate variant call classifications that indicate or reflect a likelihood of identifying a variant at a genomic coordinate. As used herein, the term "variant" refers to a nucleotide base or multiple nucleotide bases that do not align with, differs from, or varies from a corresponding nucleotide base (or nucleotide bases) in a reference sequence or a reference genome. For example, a variant includes a SNP, an indel, or a structural variant that indicates nucleotide bases in a sample nucleotide sequence that differ from nucleotide bases in corresponding genomic coordinates of a reference sequence. Along these lines, a "variant nucleotide base call" (or simply "variant call") refers to a nucleotide base call comprising a variant at a particular genomic coordinate. Conversely, a "non-variant nucleotide base call" (or simply "non-variant call") refers to a nucleotide base call comprising a non-variant at a genomic coordinate.

As mentioned, in some embodiments, the call recalibration system modifies data fields corresponding to a variant call file. As used herein, the term "variant call file" refers to a digital file that indicates or represents one or more nucleotide base calls (e.g., variant calls) compared to a reference genome along with other information pertaining to the nucleotide base calls (e.g., variant calls). For example, a variant call format (VCF) file refers to a text file format that contains information about variants at specific genomic coordinates, including meta-information lines, a header line, and data lines where each data line contains information about a single nucleotide base call (e.g., a single variant). As described further below, the call recalibration system can generate different versions of variant call files, including a pre-filter variant call file comprising variant nucleotide base calls that either pass or fail a quality filter for base call quality metrics or a post-filter variant call file comprising variant nucleotide base calls that pass the quality filter but excludes variant nucleotide base calls that fail the quality filter.

In some embodiments, the call recalibration system modifies data fields corresponding to metrics of a nucleotide base call associated with a variant call file, such as fields for call quality, genotype, and genotype quality. As used herein, the term "call quality" when used with respect to a data field in a variant call file refers to a measure or an indication of a likelihood or a probability that a variant exists at a given location. Accordingly, a call quality field (or QUAL field) corresponding to a VCF file may include a base call quality metric, such as a Phred-scaled quality or Q score, representing a probability that a genomic coordinate of a sample genome includes a variant. Similarly, a "genotype quality" when used with respect to a field refers to a likelihood or a probability that a particular predicted genotype for a nucleotide base call is correct.

As noted, in some embodiments, the call recalibration system utilizes a call generation model to generate a nucleotide base call for a genomic coordinate. As used herein, the term "call generation model" refers to a probabilistic model that generates sequencing data from nucleotide reads of a sample nucleotide sequence, including nucleotide base calls and associated metrics. Accordingly, in some cases, a call generation model may be a variant call generation model. For example, in some cases, a call generation model refers to a Bayesian probability model that generates variant calls based on nucleotide reads of a sample nucleotide sequence. Such a model can process or analyze sequencing metrics corresponding to read pileups (e.g., multiple nucleotide reads corresponding to a single genomic coordinate), including mapping quality, base quality, and various hypotheses including foreign reads, missing reads, joint detection, and more. A call generation model may likewise include multiple components, including, but not limited to, different software applications or components for mapping and aligning, sorting, duplicate marking, computing read pileup depths, and variant calling. In some cases, the call generation model refers to the ILLUMINA DRAGEN model for variant calling functions and mapping and alignment functions.

As mentioned above, in certain described embodiments, the call recalibration system generates or determines contribution measures associated with individual sequencing metrics. As used herein, the term "contribution measure" refers to a measure of effect, influence, or impact that a sequencing metric has on a given recalibration of fields for a base call output file (e.g., a variant call file), a given nucleotide base call in a base call output file, or (in particular) a given variant call. For example, a contribution measure indicates how much of a role one sequencing metric plays in determining a nucleotide base call over a different nucleotide base call (and compared to other sequencing metrics).

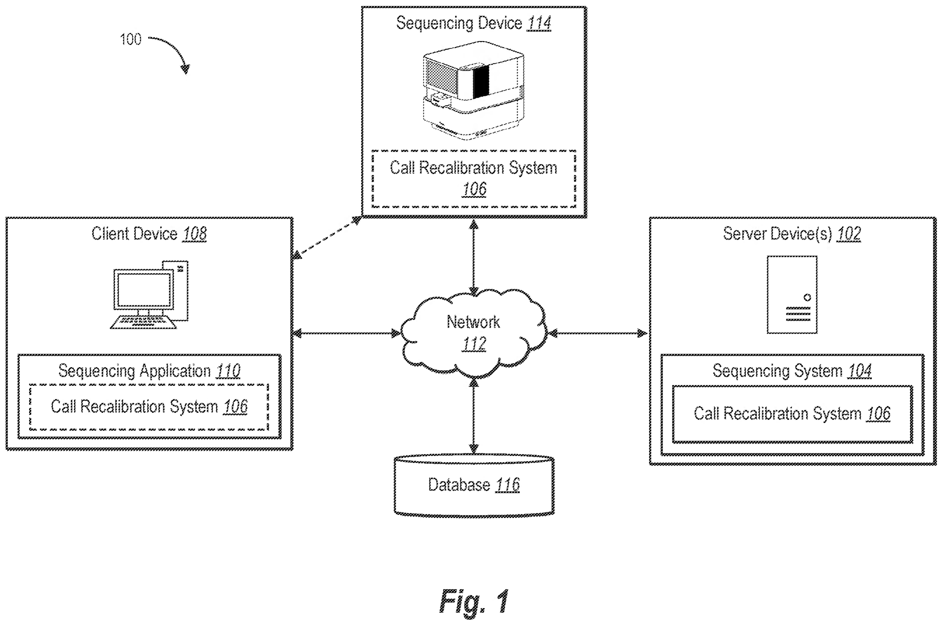

The following paragraphs describe the call recalibration system with respect to illustrative figures that portray example embodiments and implementations. For example, FIG. 1 illustrates a schematic diagram of a system environment (or "environment") 100 in which a call recalibration system 106 operates in accordance with one or more embodiments. As illustrated, the environment 100 includes one or more server device(s) 102 connected to a client device 108 and a sequencing device 114 via a network 112. While FIG. 1 shows an embodiment of the call recalibration system 106, this disclosure describes alternative embodiments and configurations below.

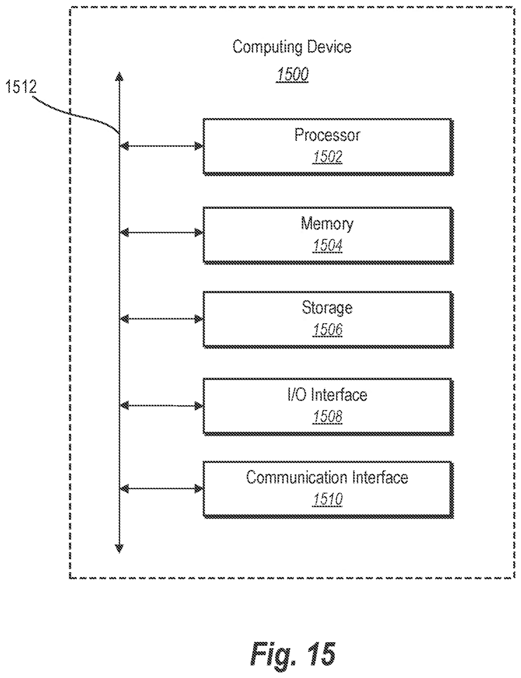

As shown in FIG. 1, the server device(s) 102, the client device 108, and the sequencing device 114 can communicate with each other via the network 112. The network 112 comprises any suitable network over which computing devices can communicate. Example networks are discussed in additional detail below with respect to FIG. 15.

As indicated by FIG. 1, the sequencing device 114 comprises a device for sequencing a nucleic acid polymer. In some embodiments, the sequencing device 114 analyzes nucleic acid segments or oligonucleotides extracted from genomic samples to generate nucleotide reads or other data utilizing computer implemented methods and systems (described herein) either directly or indirectly on the sequencing device 114. More particularly, the sequencing device 114 receives and analyzes, within nucleotide-sample slides (e.g., flow cells), nucleic acid sequences extracted from samples. In one or more embodiments, the sequencing device 114 utilizes SBS to sequence nucleic acid polymers into nucleotide reads. In addition or in the alternative to communicating across the network 112, in some embodiments, the sequencing device 114 bypasses the network 112 and communicates directly with the client device 108.

As further indicated by FIG. 1, the server device(s) 102 may generate, receive, analyze, store, and transmit digital data, such as data for determining nucleotide base calls or sequencing nucleic acid polymers. As shown in FIG. 1, the sequencing device 114 may send (and the server device(s) 102 may receive) call data from the sequencing device 114. The server device(s) 102 may also communicate with the client device 108. In particular, the server device(s) 102 can send data to the client device 108, including a variant call file or other information indicating nucleotide base calls, sequencing metrics, error data, or other metrics associated with a nucleotide base call.

In some embodiments, the server device(s) 102 comprise a distributed collection of servers where the server device(s) 102 include a number of server devices distributed across the network 112 and located in the same or different physical locations. Further, the server device(s) 102 can comprise a content server, an application server, a communication server, a web-hosting server, or another type of server. In some cases, the server device(s) 102 are located at a same physical location as the sequencing device 114.

As further shown in FIG. 1, the server device(s) 102 can include a sequencing system 104. Generally, the sequencing system 104 analyzes call data, such as sequencing metrics received from the sequencing device 114, to determine nucleotide base sequences for nucleic acid polymers. For example, the sequencing system 104 can receive raw data from the sequencing device 114 and can determine a nucleotide base sequence for a nucleic acid segment. In some embodiments, the sequencing system 104 determines the sequences of nucleotide bases in DNA and/or RNA segments or oligonucleotides. In addition to processing and determining sequences for nucleic acid polymers, the sequencing system 104 also generates a variant call file indicating one or more nucleotide base calls and/or variant calls for one or more genomic coordinates.

As just mentioned, and as illustrated in FIG. 1, the call recalibration system 106 analyzes call data, such as sequencing metrics from the sequencing device 114, to determine nucleotide base calls for sample nucleic acid sequences. The call recalibration system 106 includes a call generation model and a call recalibration machine learning model. In some embodiments, the call recalibration system 106 determines sequencing metrics for sample nucleotide sequences. Based on data derived or prepared from the sequencing metrics, the call recalibration system 106 trains and applies a call generation model to determine nucleotide base calls for the sample sequence corresponding to genomic coordinates. The call recalibration system 106 further utilizes a call recalibration machine learning model to generate sets of variant call classifications to update or modify the nucleotide base calls (and/or variant calls). Based on such data, for example, the call recalibration system 106 can update data fields corresponding to a variant call file to update a nucleotide base call and/or a variant call for improved accuracy.

As further illustrated and indicated in FIG. 1, the client device 108 can generate, store, receive, and send digital data. In particular, the client device 108 can receive sequencing metrics from the sequencing device 114. Furthermore, the client device 108 may communicate with the server device(s) 102 to receive a variant call file comprising nucleotide base calls and/or other metrics, such as a call-quality, a genotype indication, and a genotype quality. The client device 108 can accordingly present or display information pertaining to the nucleotide base call within a graphical user interface to a user associated with the client device 108. For example, the client device 108 can present a contribution-measure interface that includes a visualization or a depiction of various contribution measures associated with, or attributed to, individual sequencing metrics with respect to a particular nucleotide base call.

The client device 108 illustrated in FIG. 1 may comprise various types of client devices. For example, in some embodiments, the client device 108 includes non-mobile devices, such as desktop computers or servers, or other types of client devices. In yet other embodiments, the client device 108 includes mobile devices, such as laptops, tablets, mobile telephones, or smartphones. Additional details regarding the client device 108 are discussed below with respect to FIG. 15.

As further illustrated in FIG. 1, the client device 108 includes a sequencing application 110. The sequencing application 110 may be a web application or a native application stored and executed on the client device 108 (e.g., a mobile application, desktop application). The sequencing application 110 can include instructions that (when executed) cause the client device 108 to receive data from the call recalibration system 106 and present, for display at the client device 108, data from a variant call file. Furthermore, the sequencing application 110 can instruct the client device 108 to display a visualization of contribution measures for sequencing metrics of a nucleotide base call.

As further illustrated in FIG. 1, the call recalibration system 106 may be located on the client device 108 as part of the sequencing application 110 or on the sequencing device 114. Accordingly, in some embodiments, the call recalibration system 106 is implemented by (e.g., located entirely or in part) on the client device 108. In yet other embodiments, the call recalibration system 106 is implemented by one or more other components of the environment 100, such as the sequencing device 114. In particular, the call recalibration system 106 can be implemented in a variety of different ways across the server device(s) 102, the network 112, the client device 108, and the sequencing device 114. For example, the call recalibration system 106 can be downloaded from the server device(s) 102 to the client device 108 and/or to the sequencing device 114 where all or part of the functionality of the call recalibration system 106 is performed at each respective device within the environment 100.

As further illustrated in FIG. 1, the environment 100 includes a database 116. The database 116 can store information, such as variant call files, sample nucleotide sequences, nucleotide reads, nucleotide base calls, variant calls, and sequencing metrics. In some embodiments, the server device(s) 102, the client device 108, and/or the sequencing device 114 communicate with the database 116 (e.g., via the network 112) to store and/or access information, such as variant call files, sample nucleotide sequences, nucleotide reads, nucleotide base calls, variant calls, and sequencing metrics. In some cases, the database 116 also stores one or more models, such as a call recalibration machine learning model and/or a call generation model.

Though FIG. 1 illustrates the components of environment 100 communicating via the network 112, in certain implementations, the components of environment 100 can also communicate directly with each other, bypassing the network 112. For instance, and as previously mentioned, in some implementations, the client device 108 communicates directly with the sequencing device 114. Additionally, in some embodiments, the client device 108 communicates directly with the call recalibration system 106. Moreover, the call recalibration system 106 can access one or more databases housed on or accessed by the server device(s) 102 or elsewhere in the environment 100.

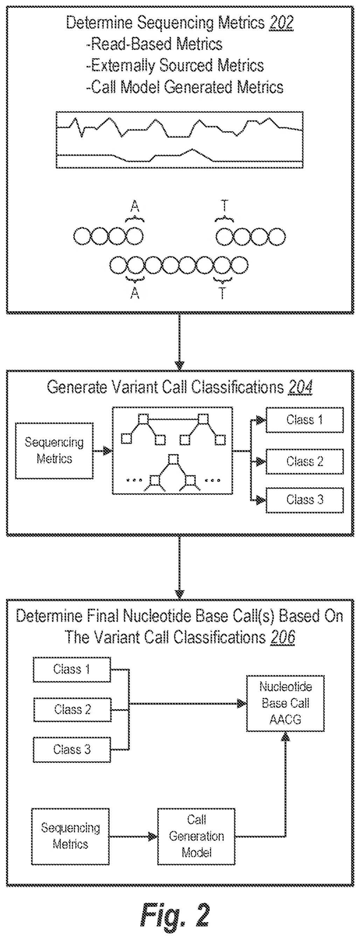

As indicated above, the call recalibration system 106 can determine a nucleotide base call based on one or more variant call classifications. In particular, the call recalibration system 106 can determine variant call classifications from sequencing metrics utilizing a call recalibration machine learning model and can determine or update various metrics associated with a nucleotide base call from the generated variant call classifications. FIG. 2 illustrates an example overview of determining a nucleotide base call based on variant call classifications in accordance with one or more embodiments.

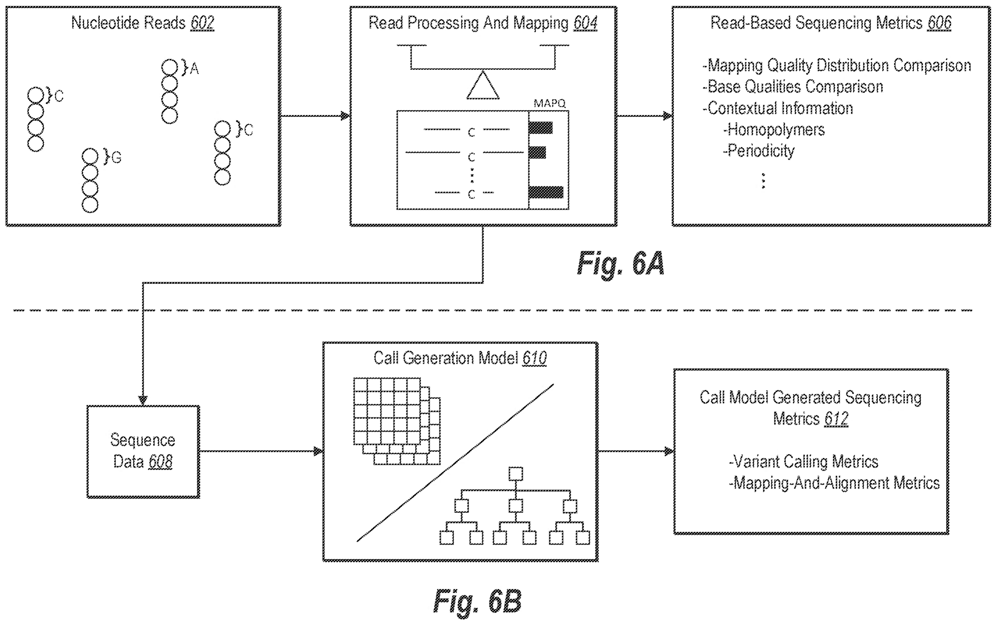

As illustrated in FIG. 2, the call recalibration system 106 performs an act 202 to determine sequencing metrics. In particular, the call recalibration system 106 determines sequencing metrics such as read-based sequencing metrics, externally sourced sequencing metrics, and call model generated sequencing metrics. For example, the call recalibration system 106 determines sequencing metrics that indicate various attributes or data in relation to various nucleotide base calls of nucleotide reads from a sample nucleotide sequence. Additional detail regarding determining the various types of sequencing metrics is provided below with reference to FIGS. 6A-6C.

As further illustrated in FIG. 2, the call recalibration system 106 performs an act 204 to generate variant call classifications. More specifically, the call recalibration system 106 generates (or updates or refines) variant call classifications from sequencing metrics utilizing a call recalibration machine learning model. To elaborate, the call recalibration system 106 utilizes the call recalibration machine learning model to process or analyze one or more sequencing metrics and to generate a set of classifications (e.g., predicted probabilities associated with genotypes). For instance, the call recalibration system 106 generates, utilizing the call recalibration machine learning model, a set of variant call classifications (represented in FIG. 2 as "Class 1," "Class 2," and "Class 3") that indicate certain probabilities associated with a genotype of a corresponding nucleotide base call based on the sequencing metrics.

In some embodiments, the call recalibration system 106 generates different variant call classifications for different applications and/or for different genomic coordinates. For example, the call recalibration system 106 generates a first set of variant call classifications for multiallelic genomic coordinates, generates a second set of variant call classifications for haploid genomic coordinates, and generates a third set of variant call classification for genomic coordinates indicated to exhibit homozygous reference genotypes. In certain embodiments, the call recalibration system 106 generates the same variant call classifications for different applications and/or for different genomic coordinates but utilizes them differently or utilizes different information associated with the variant call classifications. Additional detail regarding generating variant call classifications is provided below with reference to subsequent figures.

As further illustrated in FIG. 2, the call recalibration system 106 also performs an act 206 to determine a final nucleotide base call (or a variant call) based on the variant call classifications. More particularly, the call recalibration system 106 determines or updates a nucleotide base call for a sample nucleotide sequence at a genomic coordinate within a reference genome. To determine or generate the final nucleotide base call, in some embodiments the call recalibration system 106 determines initial nucleotide base calls utilizing a call generation model and edits or updates certain initial nucleotide base calls based on the variant call classifications generated by the call recalibration machine learning model.

To elaborate, the call recalibration system 106 utilizes a call generation model to process or analyze sequencing metrics (e.g., one or more of the same sequencing metrics used to generate the variant call classifications in act 204) to determine a nucleotide base call (e.g., an initial nucleotide base call) from the sequencing metrics. For example, the call recalibration system 106 applies a number of Bayesian probabilistic models or algorithms to derive various probabilities for different nucleotide bases, quality metrics, mapping metrics, joint metrics, and other data occurring within the sample nucleotide sequence to include within a variant call file. From the probabilistic models, the call recalibration system 106 determines a nucleotide base call (e.g., a call indicating a difference or sameness to a reference base from a reference genome) that indicates a predicted nucleotide base for the sample genome at a corresponding genomic coordinate.

As further illustrated in FIG. 2, in certain implementations, the call recalibration system 106 utilizes the initial variant call classifications (e.g., as determined via the act 204) to generate, recalibrate, determine, modify, or augment the nucleotide base call. To elaborate, the call recalibration system 106 utilizes probabilities associated with the variant call classifications to determine or update certain metrics associated with a nucleotide base call. For example, the call recalibration system 106 modifies data fields corresponding to a variant call file for metrics, such as call quality, genotype, and genotype quality (or others as described below).

In some cases, the call recalibration system 106 extrapolates from the variant call classifications to determine metrics corresponding to a variant call file, such as call quality, genotype, and genotype quality associated with the nucleotide base call. For instance, by utilizing a genotype error classification, the call recalibration system 106 can remedy certain errors in or associated with an initial nucleotide base call. Indeed, if the call recalibration system 106 determines a high false positive probability for a nucleotide base call, then the call recalibration system 106 applies the call recalibration machine learning model to function as a variant filter to modify (e.g., reduce) a call quality associated with the nucleotide base call. As another example, the call recalibration system 106 utilizes a genotype error probability to modify a genotype and/or a genotype quality of a nucleotide base call in cases where systems would previously filter out or doubly penalize heterozygous/homozygous (het/hom) errors (e.g., where the system generates a nucleotide base call that is incorrect which further results in missing a nucleotide base call that is correct).

In certain embodiments, the call recalibration system 106 considers a single variant call classification to modify a data field for a nucleotide base call (e.g., a call quality, a genotype, or a genotype quality). In other embodiments, the call recalibration system 106 considers multiple variant call classifications at once (e.g., in a weighted combination) to modify or update one or more data fields for call quality, genotype, and/or genotype quality. Additional detail regarding generating and modifying nucleotide base calls is provided below with reference to subsequent figures.

In one or more implementations, the call recalibration system 106 generates the variant call classifications (e.g., via the act 204) while, or during the process of, determining a nucleotide base call. For example, the call recalibration system 106 simultaneously implements the call recalibration machine learning model and the call generation model to generate a nucleotide base call and variant call classifications for modifying the nucleotide base call. The call recalibration system 106 further modifies data fields corresponding to a variant call file of the nucleotide base call to generate a finalized nucleotide base call (e.g., within a pre-filter or post-filter variant call file). Indeed, the call recalibration system 106 generates the finalized (e.g., recalibrated) nucleotide base call from the variant call classifications as well as sequencing metrics processed by the call generation model (e.g., one or more of the same sequencing metrics used to generate the variant call classifications). As described above, this simultaneous or parallel operation affords the call recalibration system 106 improved computational efficiency and increased speed by recalibrating nucleotide base calls as they are initially generated (rather than performing one operation before the other).

In one or more implementations, the call recalibration system 106 determines a nucleotide base call as part of a SNP, a deletion, an insertion, or a structural variation. For example, the call recalibration system 106 determines a nucleotide base call represent an SNP at a genomic coordinate (e.g., chr1:151863125) by identifying a G in the sample nucleotide sequence where an A exists in the reference sequence. As another example, the call recalibration system 106 determines nucleotide base calls surrounding one or more genomic coordinates (e.g., chr1:49263256) indicate a deletion by identifying a single G in the sample nucleotide sequence where GTAAC exists in the reference sequence.

As a further example, the call recalibration system 106 determines a sequence of nucleotide base calls represent an insertion at a genomic coordinate (e.g., chr1:7602080) by identifying a sequence of TTTCC in the sample nucleotide sequence where a T exists in the reference sequence. Indeed, in some cases, an insertion includes a sequence of nucleotide base calls that replace a single reference base at a genomic coordinate of a reference sequence.

In some embodiments, the call recalibration system 106 sets a quality threshold (e.g., a customized quality threshold) for base call quality metrics at genomic coordinates for a genomic sample (e.g., including one or more of diploid coordinates, haploid coordinates, multiallelic coordinates, and genomic coordinates incorrectly identified as exhibiting homozygous reference genotypes). The base call quality metrics can change significantly between a call generation model and a call recalibration machine learning model. To adjust for the potentially broad range and significant changes of base call quality metrics, the call recalibration system 106 can determine or set a hard filter QUAL threshold for a variant call file output that results in (or corresponds to) a favorable F1 position as a measure of performance (e.g., a favorable trade-off between false positives and false negatives).

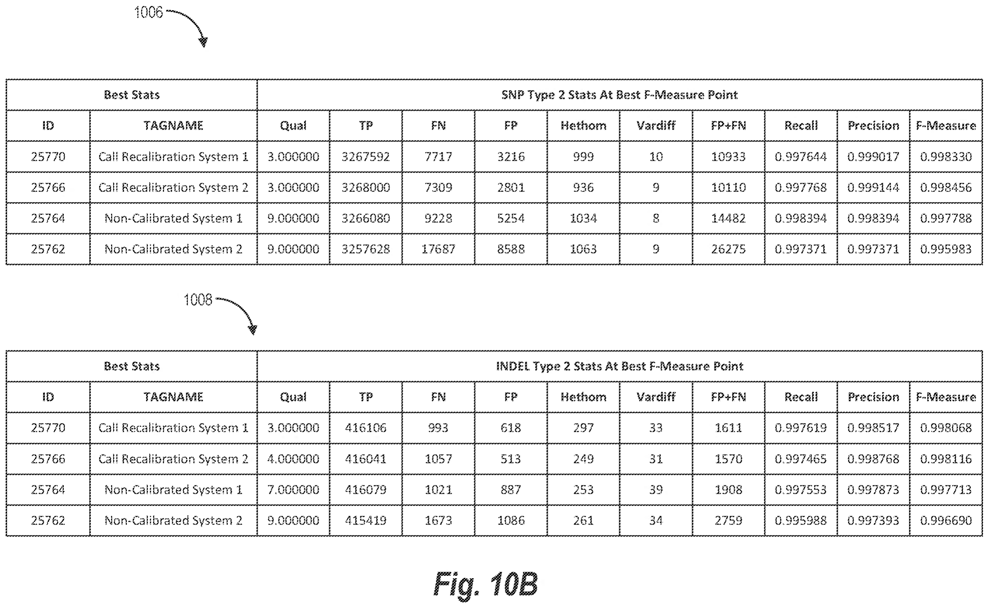

Such a favorable F1 position can include a score or a position with a favorable (e.g., best) tradeoff between precision and recall of calling variants. In some cases, for instance, an F1 position (or an F1 score) is proportional to a combination (e.g., sum) of false positive variants and false negative variants (which means that a favorable F1 score corresponds to a low FP + FN metric). As described below, for instance, FIG. 10B depicts examples of FP+FN metrics based upon which the call recalibration system 106 sets a quality threshold for base call quality metrics at haploid genomic coordinates. In some embodiments, the call recalibration system 106 thus utilizes a quality filter for base call quality metrics that result in the favorable F1 position for the Call Recalibration System 1 or 2 depicted in FIG. 10B.

As indicated above, however, the call recalibration system 106 can set such a quality threshold for base call quality metrics at any or all genomic coordinates resulting in a favorable F1 position when using a call recalibration machine learning model. Indeed, in some embodiments, the call recalibration system 106 generates F1 scores and applies related filtering logic for QUAL scores (as described above) for various genomic coordinates, including haploid coordinates, diploid coordinates, multiallelic coordinates, genomic coordinates incorrectly identified as exhibiting homozygous reference genotypes, or other genomic coordinates.

Thus, in some cases, rather than a call generation model discarding certain variant nucleotide base calls that do not pass a previous quality filter, the call recalibration system 106 executes a pipeline of the following acts: (i) utilizing a call generation model to generate variant nucleotide base calls across various regions or coordinates; (ii) utilizing a call recalibration machine learning model to recalibrate variant nucleotide base calls and corresponding metrics, such as one or more of base call quality metrics, genotype quality metrics, or genotype metrics in corresponding VCF fields; (iii) generating a prefiltered VCF that includes variant nucleotide base calls above a quality threshold either because the call generation model called a variant nucleotide base call at a corresponding genomic coordinate or because the call recalibration machine learning model called a variant nucleotide base call at a genomic coordinate at which the call generation model had determined such as variant nucleotide base call did not pass a previous quality filter; and (iv) utilizing a hard quality threshold filter to select quality variant nucleotide base calls from the prefiltered VCF. Such a hard quality threshold is configured such that the filtered output of the call recalibration system 106 is close to a favorable F1 position (thereby resulting in a post-filter VCF that contains only variant nucleotide base calls satisfying the hard quality threshold). The call recalibration system 106 can change the QUAL threshold depending on whether the call recalibration machine learning model is active or the call generation model (e.g., DRAGEN) is executing without the call recalibration machine learning model.

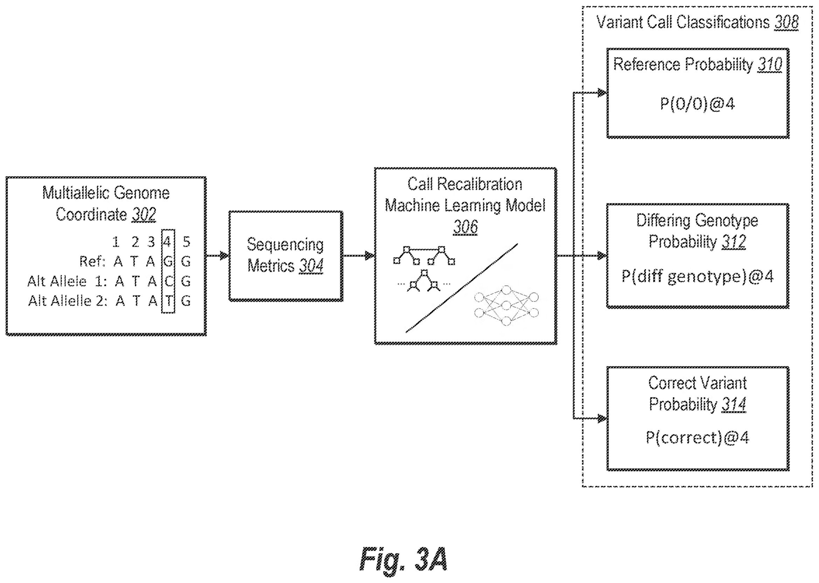

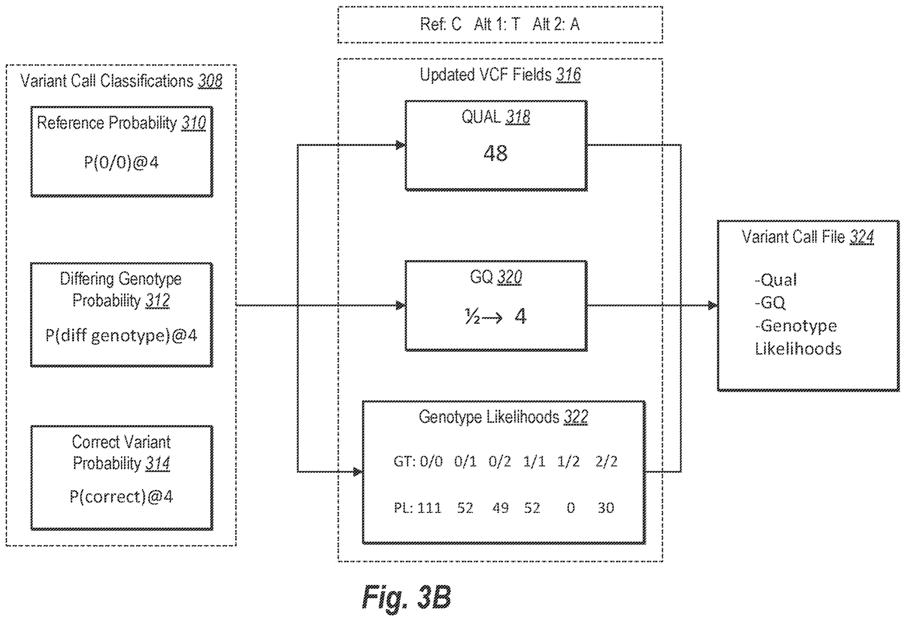

As mentioned above, in certain described embodiments, the call recalibration system 106 generates variant call classifications for multiallelic genomic coordinates. In addition, the call recalibration system 106 generates or updates a variant call file for the multiallelic coordinate based on the variant call classifications. FIGS. 3A-3B illustrate an example flow of the call recalibration system 106 generating a variant call file from variant call classifications of a multiallelic genomic coordinate in accordance with one or more embodiments. For example, FIG. 3A illustrates the call recalibration system 106 generating variant call classifications for multiallelic genomic coordinates in accordance with one or more embodiments. Thereafter, FIG. 3B illustrates the call recalibration system 106 generating a variant call file from variant call classifications in accordance with one or more embodiments.

As illustrated in FIG. 3A, the call recalibration system 106 identifies a multiallelic genomic coordinate 302. For instance, the call recalibration system 106 identifies the multiallelic genomic coordinate 302 from nucleotide base calls corresponding to a sample nucleotide sequence or based on haplotype data corresponding to the multiallelic genomic coordinate 302. In some cases, the call recalibration system 106 identifies the multiallelic genomic coordinate 302 by determining (i) that nucleotide base calls from nucleotide reads covering the genomic coordinate include more than two possible nucleotide base calls from corresponding alleles and (ii) that the nucleotide base calls satisfy one or more threshold sequencing metrics (e.g., a base-call-quality metric of Q30). Additionally or alternatively, in certain embodiments, the call recalibration system 106 identifies the genomic coordinate by from a database comprising a haplotype reference panel correlated with specific genomic coordinates. Based on different haplotype probabilities in the database, the call recalibration system 106 identifies genomic coordinates as probable candidates for multiallelic genomic coordinates. Regardless of the identification method, in some cases, the call recalibration system 106 uses a call generation model (e.g., a variant caller within a call generation model) to identify the multiallelic genomic coordinate 302.

As depicted in FIG. 3A, for instance, the call recalibration system 106 analyzes the nucleotide base sequences corresponding to coordinates 1 through 5 to identify genomic coordinate 4 as the multiallelic genomic coordinate 302. For simplicity of illustration, each of the nucleotide base sequences constitute representatives of different nucleotide reads that map to different alleles and correspond to coordinates 1 through 5. While each of coordinates 1 through 5 have three possible alleles corresponding to them-one from the reference genome, another from a first possible allele "Alternate allele 1," and another from a second possible allele "Alternate allele 2"-only coordinate 4 exhibits three or more different nucleotide base calls from different alleles that could possibly be assigned. Specifically, coordinate 4 could exhibit a G from the reference genome, a C from the first alternate allele, or a T from the second alternate allele.

As further illustrated in FIG. 3A, the call recalibration system 106 determines sequencing metrics 304 for the multiallelic genomic coordinate 302. In particular, the call recalibration system 106 determines sequencing metrics associated with nucleotide reads, generated by a call generation model, or retrieved from an external source. Additional detail regarding determining the sequencing metrics 304 is provided below with specific reference to FIGS. 6A-6C.

Additionally, as shown in FIG. 3A, the call recalibration system 106 utilizes a call recalibration machine learning model 306 to generate variant call classifications 308. Specifically, the call recalibration system 106 utilizes the call recalibration machine learning model 306 to generate a reference probability 310 indicating a probability of a homozygous reference genotype at the multiallelic genomic coordinate 302. To elaborate, the call recalibration system 106 generates a variant call classification that indicates a probability that, based on its sequencing metrics, the multiallelic genomic coordinate 302 exhibits a homozygous genotype with respect to a reference genome. As shown, the reference probability 310 indicates a probability of a homozygous reference genotype (0/0) at coordinate 4 from the sample nucleotide sequence (represented as P(0/0)@4).

In one or more implementations, the call recalibration system 106 also utilizes the call recalibration machine learning model 306 to generate a differing genotype probability 312 indicating a probability of a genotype error at the multiallelic genomic coordinate 302. For instance, the call recalibration system 106 determines a probability that a predicted genotype for the multiallelic genomic coordinate 302 is an incorrect genotype (e.g., a genotype incorrectly identified by a call generation model) or includes an incorrect allele in the predicted genotype. To elaborate, in some cases, the call recalibration system 106 determines a probability that any het/hom error exists at the multiallelic genomic coordinate 302-e.g., where the alternate base is correct but the genotype is wrong-or a probability that the nucleotide base calls represent either the wrong genotype altogether or the wrong allele(s) in the predicted genotype. For example, when determining a probability that a het/hom error exists, the call recalibration system 106 determines a probability that an alternate base call represented as "1" is correct, but the genotype is incorrect, such as a probability of incorrectly determining a 0/1 genotype call (e.g., A/T) instead of a correct 1/1 genotype call (e.g., T/T) (or vice versa when the correct genotype call is 0/1).

By determining the differing genotype probability 312, the call recalibration system 106 can fix inaccuracies of existing sequencing systems where incorrect calls are often indels. In particular, the call recalibration system 106 can more accurately generate nucleotide base calls for genomic coordinates corresponding to indels where existing sequencing systems would determine a nucleotide base call represent an incorrect genotype that represents an incorrect allele resulting from a long inserted or deleted sequence. As shown, the differing genotype probability 312 indicates a probability of a different genotype belonging at coordinate 4 (represented as P(diff genotype)@4).

As further illustrated in FIG. 3A, the call recalibration system 106 utilizes the call recalibration machine learning model 306 to generate a correct variant probability 314 indicating a probability of a correct variant call genotype at the multiallelic genomic coordinate 302. For example, the call recalibration system 106 generates a probability that a predicted genotype for the multiallelic genomic coordinate 302 is correct as determined by a call generation model. As shown, the call recalibration system 106 determines the correct variant probability 314 indicating a probability that the predicted variant call from the call generation model is correct for coordinate 4 (represented as P(correct)@4).

Continuing to FIG. 3B, in some embodiments, the call recalibration system 106 utilizes the variant call classifications 308 to update one or more data fields or variant call file fields ("VCF" fields) associated with a variant call file (e.g., a variant call file 324). For example, the call recalibration system 106 generates updated VCF fields 316 that indicate updated sequencing metrics for a final nucleotide base call. In some cases, the call recalibration system 106 modifies or updates only certain VCF fields and does not update others based on the variant call classifications 308. In other cases, the call recalibration system 106 does not update VCF fields based on the variant call classifications 308. When generating nucleotide base calls for the multiallelic genomic coordinate 302, for instance, the call recalibration system 106 does not update certain fields, such as a genotype (GT) field, based on the variant call classifications 308. Thus, in contrast to biallelic genomic coordinates, in some cases, the call recalibration system 106 does not modify or update a GT field because, in some cases, there is not enough information to determine a new or updated genotype at a multiallelic genomic coordinate.

To illustrate one embodiment, FIG. 3B depicts the call recalibration system 106 generating updated VCF fields 316 for a genotype (GT) of 1/2, where cytosine represents a reference base (shown as "Ref: C") at a multiallelic genomic coordinate for an allele corresponding to the reference genome, adenine represents a first alternate base ("Alt 1: A") at the multiallelic genomic coordinate for a different allele, and thymine represents a second alternate base ("Alt 2: T") at the multiallelic genomic coordinate for yet a different allele. But FIG. 3B merely depicts examples of a possible reference base and possible alternate bases at a multiallelic genomic coordinate. The call recalibration system 106 can generate variant call classifications and modify corresponding metrics in VCF fields for various other reference bases and alternate bases at other multiallelic genomic coordinates.

As further illustrated in FIG. 3B, the call recalibration system 106 generates an updated base call quality (QUAL) field 318. More specifically, the call recalibration system 106 modifies or updates a base call quality metric based on the variant call classifications 308 to indicate an accuracy of a nucleotide base call at the multiallelic genomic coordinate 302. As shown, the updated base call quality field 318 indicates a QUAL score of 48 for a variant at the corresponding genomic coordinate. In this example, the updated base call quality metric (e.g., QUAL score of 48) represents a score for any type of variant at the corresponding multiallelic genomic coordinate. In addition, the call recalibration system 106 generates a modified or updated genotype quality (GQ) field 320. For instance, based on the variant call classifications 308, the call recalibration system 106 generates a modified or updated genotype quality metric indicating a likelihood or a probability that a predicted genotype at the multiallelic genomic coordinate 302 is correct. As shown, for instance, the updated genotype quality field 320 indicates a genotype quality metric for a genotype call with a heterozygous genotype (e.g., a GQ score of 4 for a genotype of 1/2 at a multiallelic genomic coordinate).

In one or more embodiments, the call recalibration system 106 further generates or updates genotype likelihoods 322 and (in some cases) uses the genotype likelihoods 322 to rank alleles. To elaborate, the call recalibration system 106 generates updated genotype likelihoods as the genotype likelihoods 322 by ordering candidate nucleotide base calls at the multiallelic genomic coordinate 302 according to the candidate nucleotide base calls' respective probabilities of belonging at the multiallelic genomic coordinate 302. For example, the call recalibration system 106 determines probabilities associated with a plurality of genotypes where each diploid genotype is composed of a pair of alleles. As another example, the call recalibration system 106 determines relative probabilities associated with a plurality of alleles (e.g., from a reference genome, a first alternate allele, and a second alternate allele) of belonging at the multiallelic genomic coordinate 302 of the sample nucleotide sequence. In some embodiments, the call recalibration system 106 generates metrics for a PHRED-scale Likelihood (PL) field as part of the updated VCF fields 316. For example, the call recalibration system 106 generates metrics for a PL field that can indicate genotypes, such as homozygous reference, heterozygous, and homozygous alternate genotypes (e.g., with PL field nomenclature 9/0/3, respectively).

Indeed, the call recalibration system 106 generates the allele-specific probabilities or likelihoods based on a relative probability of a nucleotide base call corresponding to an allele from a call generation model versus any other (non-reference) genotype identified by the call recalibration machine learning model 306. For instance, in some embodiments, the call recalibration system 106 indicates relative probability scores for each allele corresponding to respective nucleotide base calls in PL fields indicating normalized PHRED-scale likelihoods for genotypes and/or Genotype Likelihood (GL) fields indicating log-scaled likelihoods (e.g., log10-scaled) of data (e.g., sequencing metrics) given a called genotype.

As an example of generating updated genotype likelihoods and modifying certain VCF fields, in some cases, the call recalibration system 106 utilizes the call recalibration machine learning model 306 to generate the variant call classifications 308 as a set of three variant call classifications (whose probabilities sum to 1). In particular, the call recalibration machine learning model 306 may generate the reference probability 310 as 0.1, the differing genotype probability 312 as 0.2, and the correct variant probability 314 as 0.7. Based on the reference probability 310, the differing genotype probability 312, and the correct variant probability 314 in such an example, the call recalibration system 106 generates the updated genotype likelihoods 322 by updating GT=0/0 using the reference probability 310, updating GT=1/2 using the correct variant probability 314, and updating other genotype positions in a PL field using a combination of information from the call recalibration machine learning model 306 and a call generation model. To use such a combination, in some embodiments, the call recalibration system 106 combines (e.g., sums) the probabilities of all of the alternative genotypes (as determined by the call generation model) and scales the combination to match the differing genotype probability 312.

As illustrated in FIG. 3B, the call recalibration system 106 generates genotype likelihoods by determining a normalized PL score for different genotypes (GT). According to the normalized scale of a PL score, a relatively lower score (e.g., PL 0) for a genotype represents a relatively higher likelihood of the genotype being present at a genomic coordinate; and a relatively higher score (e.g., PL 101) for the genotype represents a relatively lower likelihood of the genotype being present at the genomic coordinate. For example, the call recalibration system 106 determines a PL score of 111 for the 0/0 genotype, a PL score of 52 for the 0/1 genotype, a PL score of 49 for the 0/2 genotype, a PL score of 42 for the 1/1 genotype, a PL score of 0 for the 1/2 genotype, and a PL score of 30 for the 2/2 genotype. Accordingly, in FIG. 3B, the PL score of 0 indicates a genotype with the highest likelihood or the selected genotype (e.g., a 1/2 genotype) and the PL score of 111 represents the lowest likelihood (e.g., a 0/0 genotype). Thus, in the example, the order of genotypes according to likelihood (from most likely to least likely) is as follows: 1/2, 2/2, 1/1, 0/2, 0/1, and 0/0.

In some cases, the call recalibration system 106 generates the updated genotype likelihoods 322 as a ranking of a plurality of alleles identified via the call generation model (without utilizing the call recalibration machine learning model 306). In other cases, the call recalibration system 106 utilizes a specialized version of the call recalibration machine learning model 306 that is trained to generate the updated genotype likelihoods 322 based on the variant call classifications 308.

As further illustrated in FIG. 3B, the call recalibration system 106 generates or updates a variant call file 324. The call recalibration system 106 can generate the variant call file 324 to include the updated VCF fields 316, including a base call quality metric, a genotype quality metric, and/or updated genotype likelihoods. As mentioned, in some cases, the call recalibration system 106 updates only certain fields while other fields, such as a genotype (GT) field remain unchanged based on the multiallelic analysis of FIGS. 3A-3B. For instance, the call recalibration system 106 updates the genotype quality field and the based call quality field.

For other data fields such as normalized PHRED-scale likelihoods (PL) for genotypes and posterior genotype probability (GP), the call recalibration system 106 either: (i) maintains the field as-is, (ii) removes the field, or (iii) only updates fields to reflect GQ for the called genotype and *Class* 0 output 0/0. In some cases, the call recalibration system 106 maintains the relative probabilities of other genotypes with respect to the called genotype to ensure consistent updates and that the called genotype is highest. By updating only the values for 0/0 and 1/2, the call recalibration system 106 maintains distances of other genotypes from the called genotype.

Within the variant call file 324, the call recalibration system 106 can include or update one or more final nucleotide base calls (e.g., variant nucleotide base calls) associated with the multiallelic genomic coordinate 302, as determined based on the updated VCF fields 316. Indeed, to generate a final nucleotide base call for the multiallelic genomic coordinate 302, the call recalibration system 106 can predict two nucleotide bases from three or more candidate alleles at the multiallelic genomic coordinate (e.g., according to their respective probabilities).

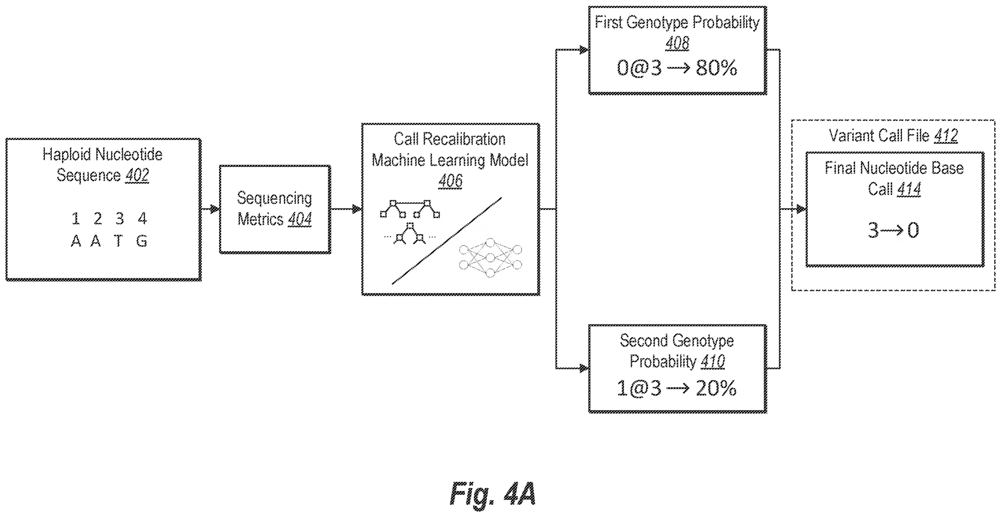

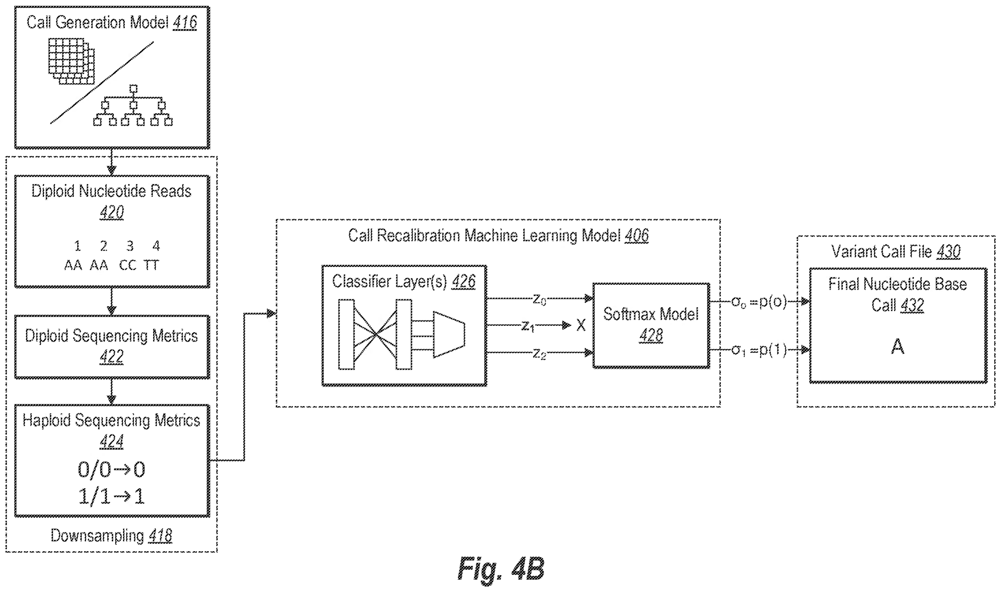

As mentioned, in certain described embodiments, the call recalibration system 106 generates final nucleotide base calls (e.g., variant calls) for genomic coordinates within a haploid nucleotide sequence from a genomic sample. In particular, the call recalibration system 106 determines a haploid genotype for a haploid coordinate of a sample nucleotide sequence and further determines whether the haploid genotype is a variant. FIGS. 4A-4B illustrate generating a final nucleotide base call for a haploid genomic coordinate in accordance with one or more embodiments. For example, FIG. 4A illustrates the call recalibration system 106 generating a final nucleotide base call utilizing a call recalibration machine learning model in accordance with one or more embodiments. Thereafter, FIG. 4B illustrates a process for the call recalibration system 106 training, tuning, testing, and/or applying a call recalibration machine learning model to generate nucleotide base calls for haploid coordinates in accordance with one or more embodiments.

As illustrated in FIG. 4A, the call recalibration system 106 identifies a haploid nucleotide sequence 402. In particular, the call recalibration system 106 identifies the haploid nucleotide sequence 402 as a region of a sample nucleotide sequence that only includes haploids (as opposed to diploids). For example, the call recalibration system 106 determines, via a call generation model, that a region of a sample nucleotide sequence is located on a haploid sex chromosome (e.g., chr:Y). In some cases, the call recalibration system 106 determines or identifies the haploid nucleotide sequence 402 by determining nucleotide base calls for nucleotide reads corresponding to the haploid nucleotide sequence 402 and aligning the nucleotide reads with a reference genome. While the haploid nucleotide sequence 402 is determined from nucleotide base calls for nucleotide reads, for simplicity of illustration, FIG. 4A depicts the nucleobases for the underlying haploid nucleotide sequence 402 at given genomic coordinates. This disclosure describes the process of determining nucleotide base calls for nucleotide reads and corresponding sequencing metrics below with respect to FIGS. 6A-6B. As shown in FIG. 4A, the call recalibration system 106 identifies the haploid nucleotide sequence 402 to include genomic coordinates 1 through 4 based on nucleotide reads, each with a single nucleotide base: 1. A 2. A 3. T 4. G.

As further illustrated in FIG. 4A, the call recalibration system 106 determines sequencing metrics 404 for the haploid nucleotide sequence 402. In particular, the call recalibration system 106 determines read-based sequencing metrics, call model generated sequencing metrics, and/or externally sourced sequencing metrics associated with a particular genomic coordinate within the haploid nucleotide sequence 402. Additional detail regarding determining sequencing metrics is provided below with reference to FIGS. 6A-6C.

Based on the sequencing metrics 404, the call recalibration system 106 utilizes a call recalibration machine learning model 406 (e.g., the call recalibration machine learning model 306) to generate, for a genomic coordinate within the haploid nucleotide sequence 402, a first genotype probability 408 and a second genotype probability 410 based on the sequencing metrics 404. For instance, the call recalibration system 106 generates the first genotype probability 408 indicating a probability that the genomic coordinate exhibits a first genotype (e.g., a haploid reference genotype) and generates the second genotype probability 410 indicating a probability that the genomic coordinate exhibits a second genotype (e.g., a haploid alternate genotype). As used herein, in some cases, the first genotype probability 408 and the second genotype probability 410 are examples of types of variant call classifications.

In some cases, the call recalibration system 106 generates the first genotype probability 408 and the second genotype probability 410 by converting inputs and/or outputs of the call recalibration machine learning model 406 to adapt the model to haploid scenarios. For example, in some cases, the call recalibration system 106 converts certain sequencing metrics or features as inputs of the call recalibration machine learning model 406 from haploid inputs to diploid inputs. More specifically, the call recalibration system 106 converts a haploid reference genotype call generated by a call generation model to a diploid homozygous reference genotype call as an input for the call recalibration machine learning model 406 (e.g., converts a haploid 0 VC GT to a diploid 0/0 GT as an input). In addition, the call recalibration system 106 converts a haploid alternate genotype call generated by the call generation model to a diploid homozygous alternate genotype call as an input for the call recalibration machine learning model 406 (e.g., converts a haploid 1 VC GT to a diploid 1/1 GT as an input). Further, in some cases, the call recalibration system 106 excludes, removes, or ignores a heterozygous genotype call generated by the call generation model as an input for the call recalibration machine learning model 406.

In one or more embodiments, the call recalibration system 106 also (or alternatively) converts outputs of the call recalibration machine learning model 406 from diploid outputs to haploid outputs. For instance, in some cases, the call recalibration system 106 converts from diploid outputs to haploid outputs utilizing a softmax model or layer (e.g., as a layer within the call recalibration machine learning model 406). In some cases, the call recalibration system 106 utilizes the softmax layer to modify confidence scores of diploid genotypes to simulate (or transform into) probabilities of haploid genotypes for the genomic coordinate. For instance, the call recalibration system 106 utilizes a softmax layer to modify a homozygous reference confidence score of a homozygous reference genotype at the genomic coordinate to generate a haploid reference probability of a reference genotype at the genomic coordinate. Further the call recalibration system 106 utilizes a softmax layer to modify a homozygous alternate confidence score of a homozygous alternate genotype at the genomic coordinate to generate a haploid alternate probability of an alternate genotype at the genomic coordinate.

In one or more embodiments, the call recalibration system 106 prunes or removes one of the three model outputs. For instance, when determining a nucleotide base call for a haploid genomic coordinate, the call recalibration system 106 removes a confidence score that a genotype of the genomic coordinate is heterozygous (or that a het/hom error exists at the coordinate) and does not input such a confidence score into the softmax layer. Based on a first confidence score that the genomic coordinate exhibits a haploid reference genotype and a second confidence score (or a third confidence score) that the genomic coordinate exhibits a haploid alternate genotype, the call recalibration system 106 uses a softmax layer to normalize these remaining two confidence scores (so that they sum to 1) to generate the first genotype probability 408 and the second genotype probability 410. Thus, the call recalibration system 106 generates the first genotype probability 408 and the second genotype probability 410 for haploids based on corresponding diploid probabilities.

As shown in FIG. 4A, the first genotype probability 408 indicates an 80% probability that the genotype of haploid coordinate 3 is 0 or, in other words, constitutes a haploid reference (represented as 0@3→80%). Similarly, the second genotype probability 410 indicates a 20% probability that the genotype of haploid coordinate 3 is 1 or, in other words, constitutes a haploid alternate (represented as 1@3→20%). Additional detail regarding converting the outputs is provided below with reference to FIG. 4B.

As further illustrated in FIG. 4A, the call recalibration system 106 generates or updates a variant call file 412 based on the first genotype probability 408 (e.g., a haploid-reference-genotype probability) and the second genotype probability 410 (e.g., a haploid-alternate-genotype probability). For example, the call recalibration system 106 updates the variant call file 412 to reflect or indicate a final nucleotide base call 414 associated with the haploid genomic coordinate based on the first genotype probability 408 and the second genotype probability 410.

In certain embodiments, the call recalibration system 106 determines the final nucleotide base call 414 to indicate a haploid genotype for the genomic coordinate based on comparing the first genotype probability 408 and the second genotype probability 410 and selecting a highest genotype from among the first genotype probability 408 and the second genotype probability 410. In some cases, the call recalibration system 106 updates additional fields associated with the variant call file 412, such as a base call quality field, a genotype quality field, and/or a genotype field based on comparing the first genotype probability 408 and the second genotype probability 410.

Based on determining that the second genotype probability 410 is highest (i.e., exceeds the first genotype probability 408) or that the nucleotide base call (or the variant call) is most likely a true positive, for instance, the call recalibration system 106 determines a haploid alternate genotype for the genomic coordinate. When the second genotype probability 410 (e.g., a haploid-alternate-genotype probability) exceeds the first genotype probability 408 (e.g., a haploid-reference-type probability), for example, the call recalibration system 106 further determines a modified base call quality metric, a modified genotype metric, and/or a modified genotype quality metric (to include within the variant call file 412). In some cases, the above modifies the genotype quality metric to reflect a likelihood that the nucleotide base call or the variant call is incorrect (in PHRED format) with the existing genotype.

Based on determining that the second genotype probability 410 is not highest (i.e., that the first genotype probability 408 exceeds the second genotype probability 410), the call recalibration system 106 determines a haploid reference genotype for the genomic coordinate. When the first genotype probability 408 (e.g., a haploid-reference-type probability) exceeds the second genotype probability 410 (e.g., a haploid-alternate-genotype probability), for example, the call recalibration system 106 further determines a modified genotype quality metric and/or a modified base call quality metric. For instance, if the call recalibration system 106 predicts a reference genotype call, the call recalibration system 106 keeps the called genotype and sets the score to the value output by the call recalibration machine learning model. If, however, the call recalibration system 106 uses the call recalibration machine learning model to determine a modified base call quality metric for the genotype call at a haploid genomic coordinate, the call recalibration system 106 changes a quality field for the genotype call to include the modified base call quality metric. Alternatively, in some cases, when a base call quality metric falls below a quality threshold, the call recalibration system 106 can drop the nucleotide base call or at least not include the nucleotide base call for the genomic coordinate in a variant call file.

In some embodiments, the call recalibration system 106 generates a final nucleotide base call 414 based on the comparison of the first genotype probability 408 and the second genotype probability 410. As shown, the call recalibration system 106 determines that the first genotype probability 408 is higher than the second genotype probability 410 and therefore generates the final nucleotide base call 414 to indicate that the genotype for the specific haploid coordinate (coordinate 3) is most likely a haploid reference genotype (represented as 3→0).

As illustrated in FIG. 4B, the call recalibration system 106 modifies input and outputs of the call recalibration machine learning model 406 to facilitate generating final nucleotide base calls (e.g., variant calls) for a genomic coordinate of a haploid nucleotide sequence. In some embodiments, the process illustrated in FIG. 4B represents a training and/or tuning of the call recalibration machine learning model 406 to learn parameters for generating nucleotide base calls (e.g., variant calls). In other embodiments, some or all of the process illustrated in FIG. 4B represents application, or inference using, of the call recalibration machine learning model 406.

As shown, the call recalibration system 106 performs a downsampling 418 of (a subset of) diploid nucleotide reads 420 to simulate haploid nucleotide reads. More specifically, the call recalibration system 106 downsamples (or otherwise modifies) diploid data to mimic or simulate haploid data for training or tuning the call recalibration machine learning model 406. Indeed, because ground truth haploid data is sparse, the call recalibration system 106 cannot rely on diploid data alone to learn robust parameters for generating nucleotide base calls for haploid coordinates. Thus, unlike some existing sequencing systems that cannot generate calls for haploid coordinates (due to the lack of training data), in some embodiments, the call recalibration system 106 adapts to haploid scenarios by simulating haploid data from diploid data.

For example, the call recalibration system 106 determines (or receives) diploid nucleotide reads 420 via a call generation model 416. Additionally, the call recalibration system 106 (randomly) selects a subset of the diploid nucleotide reads 420 to use as training or testing data (e.g., a random selection of 50% of the reads). As depicted, the diploid nucleotide reads 420 include reads for four genomic coordinates 1 through 4, as follows: 1) AA 2) AA 3) CC 4) TT. In addition, the call recalibration system 106 determines diploid sequencing metrics 422 from the (subset of the) diploid nucleotide reads 420. In some embodiments, the call recalibration system 106 determines or identifies, based on truth data (e.g., PrecisionFDA truth data, Platinum Genomes, or some other high confidence truth set, such as truth sets from the Genome in a Bottle (GIAB), Global Alliance for Genomic Health (GA4GH), or Telomere to Telomere Consortium) and/or the diploid sequencing metrics 422, one or more genomic coordinates of the diploid nucleotide reads 420 that exhibit homozygous genotypes, such as a homozygous reference genotype or a homozygous alternate genotype.

As further illustrated in FIG. 4B, the call recalibration system 106 generates (or simulates) haploid sequencing metrics 424 from the diploid sequencing metrics 422 via the downsampling 418. For instance, the call recalibration system 106 modifies the homozygous genotypes of the diploid nucleotide reads 420 to simulate haploid genotypes. Specifically, the call recalibration system 106 transforms a homozygous reference genotype to a haploid reference genotype (represented as 0/0→0) and transforms a homozygous alternate genotype to a haploid alternate genotype (represented as 1/1→1). The call recalibration system 106 further selects, as the haploid sequencing metrics 424, the sequencing metrics of the diploid nucleotide reads 420 used to simulate haploid nucleotide reads. Based on these haploid sequencing metrics 424, the call recalibration system 106 can train and/or test the call recalibration machine learning model 406 to accurately generate final nucleotide base calls (e.g., variant calls) for haploid coordinates.

Indeed, in training, testing, and/or inference, the call recalibration system 106 utilizes the call recalibration machine learning model 406 to generate final nucleotide base calls based on sequencing metrics, such as the haploid sequencing metrics 424. As mentioned above, as part of generating a final nucleotide base call 432 via the call recalibration machine learning model 406 (either for training, testing, or inference), the call recalibration system 106 modifies outputs of the call recalibration machine learning model 406. For example, the call recalibration system 106 modifies confidence scores generated by one or more classifier layers(s) 426 of the call recalibration machine learning model 406.

In some embodiments, the call recalibration system 106 does not simulate haploid data from diploid data during an inference process (as opposed to a training or testing process). Indeed, when applying the call recalibration machine learning model 406 to generate predictions, the call recalibration system 106 may only modify the inputs and outputs of the call recalibration machine learning model 406 once the model is trained for haploid scenarios with simulated haploid data. When using the call recalibration machine learning model 406, for instance, the call recalibration system 106 inputs a sequencing metric indicating the data is haploid during the inference process.

Specifically, and as depicted in FIG. 4B, the call recalibration system 106 generates three separate confidence scores, each representing confidence levels of a different genotype belonging to a given genomic coordinate: (i) a first confidence score for a haploid reference genotype (z₀), (ii) a second confidence score for a heterozygous genotype (z₁), and (iii) a third confidence score for a haploid alternate genotype (z₂). The call recalibration system 106 further prunes, ignores, or removes the second confidence score (z₁) because heterozygous genotypes cannot be simulated from diploid data to haploid data and (during implementation) haploid coordinates do not exhibit heterozygous genotypes. In some embodiments, the call recalibration system 106 utilizes a softmax model 428 as another layer in the call recalibration machine learning model 406 to generate final probabilities from the confidence scores.

In particular, and as shown in FIG. 4B, the call recalibration system 106 utilizes the softmax model 428 to generate two genotype probabilities (e.g., the first genotype probability 408 of a haploid reference genotype and the second genotype probability 410 of a haploid alternate genotype) from the modified confidence scores. To elaborate, after ignoring or discarding the second confidence score (z₁), the call recalibration system 106 utilizes the softmax model 428 to normalize across the first confidence score and the third confidence score (so they sum to 1). The call recalibration system 106 further generates the first genotype probability 408 represented by σ₀ = p(0) and the second genotype probability 410 represented by σ₁ = p(1). As described, each probability score represents a probability of a respective haploid genotype at a genomic coordinate.

Based on the probability scores, the call recalibration system 106 further generates the variant call file 430 (e.g., the variant call file 412) including the final nucleotide base call 432 (e.g., the final nucleotide base call 414). For example, the call recalibration system 106 determines the final nucleotide base call 432 from the two genotype probabilities. As shown, for example, the final nucleotide base call 432 is a haploid A for the given genomic coordinate. But the final nucleotide base call 432 could be different predicted nucleotide bases in other embodiments. Additional detail regarding generating a variant call file is provided throughout this disclosure.

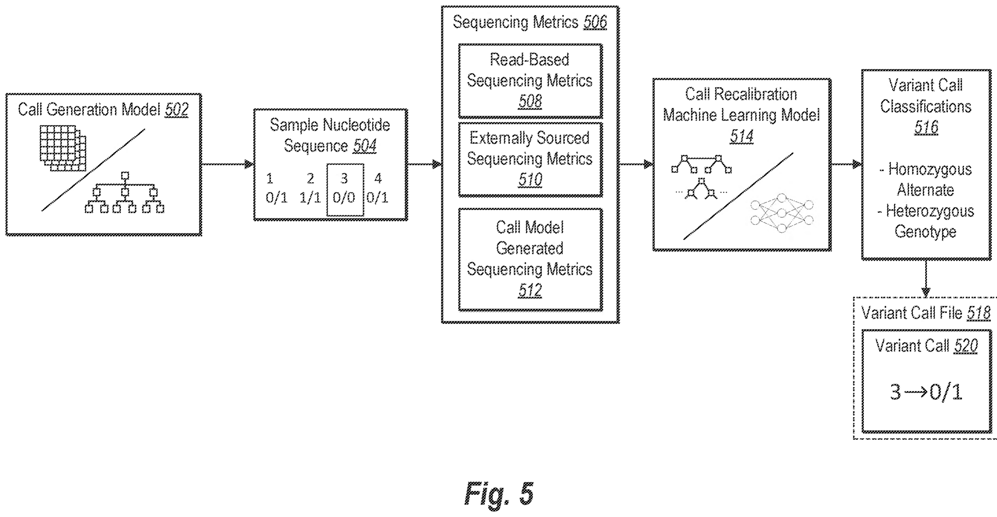

As mentioned above, in certain described embodiments, the call recalibration system 106 generates final nucleotide base calls (e.g., variant calls) for homozygous reference genomic coordinates (as initially predicted by a call generation model). In particular, the call recalibration system 106 generates final nucleotide base calls for genomic coordinates of a sample nucleotide sequence determined (or would be determined) by a call generation model to exhibit homozygous reference genotypes. FIG. 5 illustrates generating a variant call for a genomic coordinate that would or could have been incorrectly identified as a homozygous reference genotype in accordance with one or more embodiments.

As illustrated in FIG. 5, the call recalibration system 106 utilizes a call generation model 502 to generate initial nucleotide base calls for a sample nucleotide sequence 504. In particular, the call recalibration system 106 generates nucleotide base calls that indicate alleles or genotypes associated with particular genomic coordinates. As shown, the call generation model 502 determines genotypes for the sample nucleotide sequence 504 of coordinates 1 through 4 as follows: 1) 0/1 2) 1/1 3) 0/0 4) 0/1. Additionally, the call recalibration system 106 identifies or determines genomic coordinates within the sample nucleotide sequence 504 that exhibit homozygous reference genotypes as determined by the call generation model 502. In the illustrated example, the call recalibration system 106 identifies coordinate 3 as a homozygous reference coordinate. By contrast, in some embodiments, the call recalibration system 106 does not generate an initial nucleotide base call indicating a homozygous reference genotype, but rather determines sequencing metrics 506 for nucleotide reads covering the genomic coordinate consistent with a homozygous reference genotype.

In many cases, existing sequencing systems ignored homozygous reference coordinates, such as coordinate 3, and treated them as true negative variant calls that were not necessary for further processing. However, such treatment relies on the accuracy of the call generation model 502 making the proper nucleotide base call initially, which is not always the case. Indeed, the call generation model 502 can generate large numbers of false negative variant calls in some scenarios. Thus, the call recalibration system 106 recovers some of these false negative variant calls by not ignoring genomic coordinates that were initially (or would have been) identified as homozygous reference genotypes and forcing further analysis at these loci (e.g., to consequently update or modify their determined genotypes).

Specifically, as illustrated in FIG. 5, the call recalibration system 106 extracts or determines sequencing metrics 506 for the homozygous reference coordinate 3. For example, the call recalibration system 106 determines read-based sequencing metric 508, externally sourced sequencing metrics 510, and call model generated sequencing metrics 512 for coordinate 3. Additional detail regarding determining or extracting sequencing metrics is provided below with reference to FIGS. 6A-6C.

As further illustrated in FIG. 5, the call recalibration system 106 utilizes a call recalibration machine learning model 514 (e.g., the call recalibration machine learning model 306 or 406) to generate one or more variant call classifications 516 from the sequencing metrics 506. To elaborate, the call recalibration system 106 generates variant call classifications 516 indicating (or defining a level of) an accuracy of identifying a variant at the genomic coordinate (e.g., coordinate 3).

The following paragraphs describe examples of the variant call classifications 516. As an example variant call classification, the call recalibration system 106 generates a false positive classification utilizing the call recalibration machine learning model 514. For example, the call recalibration system 106 generates a false positive classification that indicates a probability that a nucleotide base call (e.g., genotype call) is a false positive variant, or that the nucleotide base call indicates a variant where no variant actually exists within the sample nucleotide sequence 504. The call recalibration system 106 generates the false positive classification from one or more of the sequencing metrics 506 considered together by the call recalibration machine learning model 514.

In certain implementations, the call recalibration system 106 also (or alternatively) generates a genotype error classification (or a heterozygous genotype classification) as part of the variant call classifications 516. More specifically, the call recalibration system 106 determines, utilizing the call recalibration machine learning model 514, a probability that a genotype associated with a nucleotide base call is incorrect or that a heterozygous genotype exists (e.g., for coordinate 3). For instance, the call recalibration system 106 determines a probability that a het/hom error exists at coordinate 3, where the nucleotide base call may indicate a heterozygous genotype (e.g., 0/1) within the sample nucleotide sequence 504 and the genotype is actually homozygous alternate (e.g., 1/1) with respect to the reference genome. Conversely, the call recalibration system 106 determines a probability of determining that a genotype for coordinate 3 is homozygous alternate (e.g., 1/1) when, in fact, the nucleotide base(s) are heterozygous with respect to the reference genome (e.g., 0/1).

In one or more embodiments, the call recalibration system 106 also (or alternatively) generates, as part of the variant call classifications 516, a true positive classification (or a homozygous alternate classification) for coordinate 3. In particular, the call recalibration system 106 determines, utilizing the call recalibration machine learning model 514, a probability that a nucleotide base call for coordinate 3 is a true positive variant call, or that the nucleotide base call indicates a true variant where a variant does indeed exist in relation to a reference genome, or that a homozygous alternate genotype exists at the genomic coordinate.

As further illustrated in FIG. 5, the call recalibration system 106 generates or updates a variant call file 518 to indicate a variant call 520. More specifically, the call recalibration system 106 generates the variant call 520 based on the variant call classifications 516 to indicate whether there is a variant at coordinate 3. In some cases, the call recalibration system 106 updates one or more of a call quality field, a genotype field, or a genotype quality field corresponding to the variant call file 518 based on the one or more variant call classifications 516. The call quality field, the genotype field, and/or the genotype quality field can indicate an updated variant call as the variant call 520. As shown, the variant call 520 indicates a variant at coordinate 3, changing the initial nucleotide base call for coordinate 3 from indicating a homozygous reference genotype (0/0) to indicate a heterozygous genotype (0/1). In other examples, the call recalibration system 106 does not change the initial nucleotide base call for coordinate 3 or changes the initial nucleotide base call to a different genotype, such as a homozygous alternate genotype (1/1).

In one or more embodiments, the call recalibration system 106 determines a genotype for the indicated genomic coordinate (e.g., coordinate 3) based on comparing the probabilities of the variant call classifications. For example, the call recalibration system 106 determines a homozygous alternate genotype based on determining that a true positive classification (or a homozygous alternate classification) has a highest probability from among the one or more variant call classifications. Specifically, the call recalibration system 106 updates the genotype quality field while also updating the genotype field (e.g. to 1/1) and the PL field.

Alternatively, the call recalibration system 106 determines a heterozygous genotype based on determining that a genotype error classification (e.g., a heterozygous genotype classification) has the highest probability from among the one or more variant call classifications. Specifically, the call recalibration system 106 updates the genotype quality field while also updating the genotype field (e.g., to 0/1) and the PL field.

Alternatively still, the call recalibration system 106 determines a homozygous reference genotype based on determining that neither the true positive classification (e.g., the homozygous alternate classification) nor the genotype error classification (e.g., the heterozygous genotype) has the highest probability from among the one or more variant call classifications. In some cases, the call recalibration system 106 removes or discards a record of comparing probabilities for variant classifications when both the call generation model 502 and the call recalibration machine learning model 514 determine that the genomic coordinate has a homozygous reference genotype.

In one or more embodiments, updating variant calls for homozygous reference coordinates provides or improves forced genotype functionality (e.g., for query of a genotype and genotype probabilities at a specific genomic coordinate). To elaborate, the call recalibration system 106 can determine genotypes of genomic coordinates that initially (e.g., as indicated by the call generation model 502) fail to satisfy a variant quality threshold. Indeed, the call recalibration system 106 can output genotypes to the variant call file 518 even if the variant quality of the genomic coordinate falls below a threshold typically required to identify a structural variant or other difficult-to-determine variants.

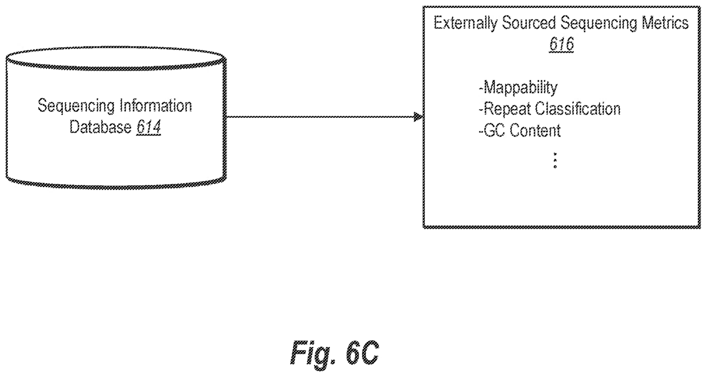

As mentioned above, in certain described embodiments, the call recalibration system 106 determines or extracts sequencing metrics for nucleotide base calls at particular genomic coordinates. In particular, the call recalibration system 106 determines sequencing metrics such as read-based sequencing metrics, externally sourced sequencing metrics, and call model generated sequencing metrics from calls corresponding to nucleotide reads from a sample nucleotide sequence. FIGS. 6A-6C illustrate determining sequencing metrics in accordance with one or more embodiments. Specifically, FIG. 6A illustrates determining read-based sequencing metrics while FIG. 6B illustrates determining call model generated sequencing metrics, and FIG. 6C illustrates determining externally sourced sequencing metrics.

As illustrated in FIG. 6A, the call recalibration system 106 accesses, retrieves, obtains, determines, or generates nucleotide reads 602. In particular, the call recalibration system 106 determines the nucleotide reads 602 utilizing the sequencing device 114 comprising nucleotide base calls for regions from a sample nucleotide sequence (e.g., sample genome). For example, the call recalibration system 106 generates a plurality of nucleotide reads 602 utilizing sequencing-by-synthesis (SBS) techniques and/or Sanger sequencing techniques to determine nucleotide base calls for oligonucleotide clusters from wells in a flow cell and/or via fluorescent tagging. More specifically, the call recalibration system 106 utilizes cluster generation and SBS chemistry to sequence millions or billions of clusters in a flow cell. During SBS chemistry, for each cluster, the call recalibration system 106 stores nucleotide base calls from the nucleotide reads 602 for every cycle of sequencing via real-time analysis (RTA) software.

As further illustrated in FIG. 6A, in some embodiments, the call recalibration system 106 performs read processing and mapping 604. For example, the call recalibration system 106 utilizes RTA software to store base call data in the form of individual base call data files (or BCLs). In some cases, the call recalibration system 106 further converts the BCL files into sequence data 608 (e.g., via BCL to FASTQ conversion), as illustrated in FIG. 6B. As shown in FIG. 6A, the call recalibration system 106 generates multiple-read coverage (e.g., read pileups) that include multiple nucleotide reads 602 or nucleotide base calls corresponding to a single genomic coordinate.

In particular, in certain embodiments, the call recalibration system 106 aligns nucleotide reads with a reference genome or receives information pertaining to the read alignment. Specifically, the call recalibration system 106 determines which nucleotide base(s) of a given read align with which genomic coordinate of a reference sequence (or receives information indicating alignment). Different reads have different lengths and include different nucleotide bases. Accordingly, in some cases, the call recalibration system 106 analyzes each nucleotide of each read to determine (or receives information indicating) where the read "fits" in relation to a reference sequence-e.g., where the bases within the read align with bases in the reference. In some cases, the call recalibration system 106 aligns many reads at a single genomic coordinate, thus resulting a read pileup.

In certain embodiments, the call recalibration system 106 performs additional statistical tests to determine or detect differences between metrics associated with a reference nucleotide sequence and metrics associated with alternative supporting nucleotide reads. Through these statistical tests, the call recalibration system 106 re-engineers raw sequencing metrics to determine read-based sequencing metrics 606. In some cases, the call recalibration system 106 determines or extracts raw sequencing metrics that include one or more of (i) alignment metrics for quantifying alignment of sample nucleotide sequences with genomic coordinates of an example nucleotide sequence (e.g., a reference genome or a nucleotide sequence from an ancestral haplotype), (ii) depth metrics for quantifying depth of nucleotide base calls for sample nucleotide sequences at genomic coordinates of the example nucleotide sequence, or (iii) call-quality metrics for quantifying quality of nucleotide base calls for sample nucleotide sequences at genomic coordinates of the example nucleotide sequence. For instance, the call recalibration system 106 determines mapping-quality metrics (e.g., the MAPQ metrics indicated in FIG. 6A), soft-clipping metrics, or other alignment metrics that measure an alignment of sample sequences with a reference genome. As another example, the call recalibration system 106 determines forward-reverse-depth metrics (or other such depth metrics) or callability metrics for variant nucleotide base calls (or other such call-quality metrics).

As just mentioned, in some embodiments, the call recalibration system 106 re-engineers the raw sequencing metrics to generate the read-based sequencing metrics 606 that are more informative for comparing metrics associated with a reference nucleotide sequence with metrics associated with various supporting alternative nucleotide reads. For example, the call recalibration system 106 determines various metrics for a sample sequence in relation to a reference sequence and further determines various metrics for the sample sequence in relation to alternative supporting sequences. In addition, the call recalibration system 106 performs comparative analyses between metrics associated with the reference sequence and the metrics associated with the alternative supporting reads.

For instance, the call recalibration system 106 compares how nucleotide bases of a sample nucleotide sequence (e.g., sample genome) map to a reference sequence with how the nucleotide bases map to various alternative supporting reads. In some cases, the call recalibration system 106 determines mapping qualities associated with the reference sequence to compare with mapping qualities associated with alternative supporting reads. For example, the call recalibration system 106 determines mapping quality statistics reflecting differences in the distribution of reads supporting a reference sequence versus reads supporting alternative alleles.

In these or other cases, the call recalibration system 106 determines mismatch counts between the sample sequence and the reference sequence and between the reference sequence and alternative supporting reads. The call recalibration system 106 further compares the mismatch counts to determine a comparative-mismatch-count metric. Further, the call recalibration system 106 determines soft-clipping metrics for the sample sequence in relation to the reference sequence and further determines soft-clipping metrics in relation to alternative supporting reads. The call recalibration system 106 also compares the soft clipping metrics between the reference sequence and the alternative supporting reads to generate a comparative-soft-clipping metric. Further still, the call recalibration system 106 compares base call quality metrics in relation to the reference sequence and alternative supporting reads and/or compares query positions of the sample sequence in relation to the reference sequence with those in relation to alternative supporting reads.

As further illustrated in FIG. 6A, the call recalibration system 106 utilizes the comparisons and/or other statistical tests to generate the read-based sequencing metrics 606, including: i) a comparative-mapping-quality-distribution metric indicating a mapping quality distribution comparing mapping qualities in relation to the reference sequence and mapping qualities in relation to alternative supporting reads, ii) a comparative-secondary-mapping-alignment metric indicating a comparison between secondary mapping in relation to bases in the reference sequence and bases in alternative supporting reads, iii) a comparative-mismatch-count metric indicating a comparison between mismatched nucleotide bases in relation to the reference sequence and mismatched bases in relation to alternative supporting reads, iv) a comparative-soft-clipping metric indicating a comparison between soft-clipping metrics in relation to the reference sequence and soft-clipping metrics in relation to alternative supporting reads, v) one or more comparative-read-depth metrics indicating comparisons between read depths of the nucleotide reads 602 and one or more average read depths (e.g., local average read depths at a particular genomic coordinate and global average read depths across a number genomic coordinates in a region), vi) one or more comparative-base-quality metric indicating comparisons between base qualities in relation to the reference sequence and base qualities in relation to alternative supporting reads (e.g., for overall base quality, early base quality, and late base quality in the nucleotide reads 602), vii) a comparative-query-position metric indicating a comparison between query positions in relation to the reference sequence and query positions in relation to alternative supporting reads, viii) one or more contextual-information metrics indicating homopolymers and periodicity of nucleotide base calls, ix) a strand-bias metric indicating a strand bias associated with one or more of the nucleotide reads 602, and x) a read-direction-bias metric indicating a read direction bias associated with the nucleotide reads 602. In some cases, the call recalibration system 106 generates or re-engineers additional or alternative read-based sequencing metrics as part of the read-based sequencing metrics 606.

In addition to the read-based sequencing metrics 606, as illustrated in FIG. 6B, the call recalibration system 106 generates call model generated sequencing metrics 612. In particular, the call recalibration system 106 generates the call model generated sequencing metrics from sequence data 608 utilizing a call generation model 610. For example, the call recalibration system 106 extracts or determines sequence data 608 based on the read processing and mapping 604 described in relation to FIG. 6A. In some cases, the call recalibration system 106 generates the sequence data 608 as part of one or more digital files, such as BCL and FASTQ files.

To generate such files, in some embodiments, the sequencing device 114 (or the call recalibration system 106) utilizes cluster generation and SBS chemistry to sequence millions or billions of clusters in a flow cell. During SBS chemistry, for each cluster, the sequencing device 114 (or the call recalibration system 106) stores nucleotide base calls from the nucleotide reads 602 for every cycle of sequencing via real-time analysis (RTA) software. The sequencing device 114 (or the call recalibration system 106) utilizes RTA software to further store base call data in the form of individual base call data files (or BCLs). In some cases, the sequencing device 114 (or the call recalibration system 106) further converts the BCL files into sequence data 608 (e.g., via BCL to FASTQ conversion). For instance, the sequencing device 114 (or the call recalibration system 106) generates a FASTQ file from the nucleotide reads 602, where the FASTQ file includes sequence data 608.

In some cases, the call recalibration system 106 generates the sequence data 608 for each cluster that passes an initial quality filter from a sample sequence. For example, the call recalibration system 106 generates entries for each cluster, where each entry includes four lines (or four items of sequence data): i) a sequence identifier with information about the sequencing run and the cluster, ii) nucleotide base calls that make up the sequence (e.g., a sequence of A, C, T, G, and/or N calls), iii) a separator (e.g., a "+" sign), and iv) base call quality metrics indicating probabilities of correctness for the nucleotide base calls (Phred +33 encoded).

As further illustrated in FIG. 6B, the call recalibration system 106 implements, utilizes, or applies the call generation model 610 to process or analyze the sequence data 608. Indeed, in some embodiments, the call recalibration system 106 generates the call model generated sequencing metrics 612 by utilizing the call generation model 610 to re-engineer raw sequencing metrics (e.g., raw sequencing metrics within the sequence data 608). In particular, the call generation model 610 includes mapping-and-alignment components to map and align nucleotide base calls from the sequence data 608. In addition, the call generation model 610 includes variant calling components to generate nucleotide base calls (e.g., reference-base calls such as variant calls or non-variant calls) from the sequence data 608. In some cases, the call recalibration system 106 extracts the call model generated sequencing metrics 612 that have been generated utilizing the mapping-and-alignment components and the variant calling components of the call generation model 610.

To illustrate examples of the call model generated sequencing metrics 612, in some cases, the call recalibration system 106 generates (variant calling metrics including one or more of: i) a base call quality metric (e.g., DRAGEN QUAL score) indicating a quality score for nucleotide base calls generated via the call generation model 610, ii) a call model generated-foreign-read-detection metric (e.g., foreign read detection (FRD) score) indicating a probability that one or more of the nucleotide reads 602 in a pileup might be foreign reads (e.g., their true location is elsewhere in the reference sequence), iii) a call model generated-base-quality-dropoff metric (e.g., base quality dropoff (BQD) score) indicating a probability of base quality dropoff based on one or more of strand bias, error position in a thread, or low mean base quality over a subset of the nucleotide reads 602, iv) average read depths, v) indel statistics (e.g., a polymerase chain reaction or "PCR" curve) and/or vi) hidden Markov model (HMM) statistics, vii) a secondary-alignment metric indicating a probability that a secondary nucleotide base call is correct, viii) a base-context metric indicating contextual information for nucleotide around a nucleotide base call, iv) a nearby-call metric indicating nearby (e.g., adjacent or within a threshold degree of separation from) a nucleotide base call, x) a joint-detection metric indicating a probability of detecting a joint corresponding to two or more overlapping nucleotide base calls, xii) read-filtering metrics indicating threshold quality metrics or other metrics for filtering out nucleotide base calls with low mapping quality, base quality, or other quality metrics, or others. The call recalibration system 106 generates the call model generated sequencing metrics 612 from internal (e.g., proprietary, and model-specific) variables that reflect interacting processing paths, corner cases, and difficult predictions/decisions.

As indicated above, in some cases, the call recalibration system 106 determines FRD scores according to the methods described in U.S. Patent Application No. 16/280,022 to Eric Jon Ojard, entitled *System and Method for Correlated Error Event Mitigation for Variant Calling,* which is incorporated by reference herein in its entirety. In certain implementations, the call recalibration system 106 also (or alternatively) determines BQD scores, FRD scores, HMM statistics, and/or other variant calling metrics according to the methods described in U.S. Patent Application Nos. 17/165,828, 15/643,381, and 14/811,836, which are incorporated by reference herein in their entireties.

As illustrated in FIG. 6B, the call model generated sequencing metrics 612 include, but are not limited to, variant calling metrics extracted via the variant calling components of the call generation model 610. In addition or in the alternative to the examples of the call model generated sequencing metrics 612 described above, in some cases, the call recalibration system 106 generates (e.g., via metric re-engineering) variant calling metrics including one or more of: i) a number of samples in a population, ii) a number of reads processed for generating nucleotide base calls, a number of variants (e.g., SNPs, indels, and MNPs), iii) a number of biallelic sites (e.g., genomic coordinates that contain two observed alleles), iv) a number of multiallelic sites (e.g., a number of sites in a variant call file that contain three or more observed alleles), v) a number of SNPs, vi) numbers of different types of indels (e.g., homozygous insertions, heterozygous insertions, and heterozygous deletions), vii) a total number of heterozygous indels (e.g., insertion + deletion, insertion + SNP, or deletion + SNP), viii) a number of de novo SNPs (e.g., SNPs with de novo quality metrics that satisfy a threshold level), ix) a number of de novo indels (e.g., indels with de novo quality metrics that satisfy a threshold level), x) a number of de novo MNPs (e.g., MNPs with de novo quality metrics that satisfy a threshold level, xi) a number of SNPs in a first chromosome divided by a number of SNPs in a second chromosome, xii) a number of SNP transitions, xiii) a number of SNP transversions, xiv) a number of heterozygous variants, xv) a number of homozygous variants, xvi) a ratio between the number of heterozygous variants and the number of homozygous variants, xvii) a number of variants detected within a dbSNP reference file, and/or xviii) a total number of variants minus the number detected within the dbSNP file.

Additionally, the call model generated sequencing metrics 612 can include mapping-and-alignment sequencing metrics extracted via the mapping-and-alignment components of the call generation model 610. For instance, the call recalibration system 106 generates or extracts (e.g., via metric re-engineering) mapping-and-alignment metrics including one or more of: i) a number of total input reads, ii) a number of duplicate marked reads, iii) a number of duplicate marked and mate reads removed, iv) a number of unique reads, v) a number of reads with mate sequenced, vi) a number of reads without mate sequenced, vii) indications of reads that fail quality checks, viii) indications of mapped reads, ix) a number of unique and mapped reads, x) a number of unmapped reads, xi) a number of singleton reads (e.g., where the read is mapped but the paired mate could not be read), xii) a number of paired reads, xiii) a number of properly paired reads (e.g., where both reads in a pair are mapped and fall within an acceptable range from each other based on an estimated insert length distribution), xiv) a number of discordant reads (e.g., not properly paired reads), xv) a number of paired reads mapped to different chromosomes, xvi) a number of paired reads mapped to different chromosomes that also have a mapping-quality metric of 10 or greater, xvii) percentages of reads within indels R1 and R2, xviii) percentages of bases in R1 and R2 that are soft clipped, xix) a number of mismatched bases in R1 and R2, xx) a number of bases with a base quality of at least 30 (e.g., total and/or in R1 or R2), xxi) a number of alignments (e.g., total alignments, secondary alignments, and/or supplementary alignments), xxii) an estimated read length, and xxiii) an estimated sample contamination.

Turning now to FIG. 6C, the call recalibration system 106 generates, extracts, or determines externally sourced sequencing metrics 616. In particular, the call recalibration system 106 determines externally sourced sequencing metrics 616 from one or more databases external to the call recalibration system 106, such as a sequencing information database 614 (e.g., the database 116). For example, the call recalibration system 106 accesses sequencing metrics that are generic or applicable to sequencing nucleotides generally. In addition, the call recalibration system 106 accesses or determines sequencing information about a particular reference sequence (e.g., stored within the sequencing information database 614). In some cases, the call recalibration system 106 determines externally sourced sequencing metrics 616 including: i) a mappability metric indicating an ease or difficult of mapping a particular nucleotide sequence (or a particular nucleotide read or nucleotide base call), ii) a guanine-cytosine-content metric indicating a count (or a dropout or a mean) of guanine-cytosine content in a reference nucleotide sequence (e.g., reference genome), iii) a replication-timing metric indicating a time required to replicate a particular number of nucleotides from a reference sequence, iv) one or more DNA-structure-metrics indicating DNA structures of a reference sequence (e.g., reference genome), v) a conservation metric indicating a measure of sequence conservation across multiple species (e.g., a measure of change relative to an average), and/or others.

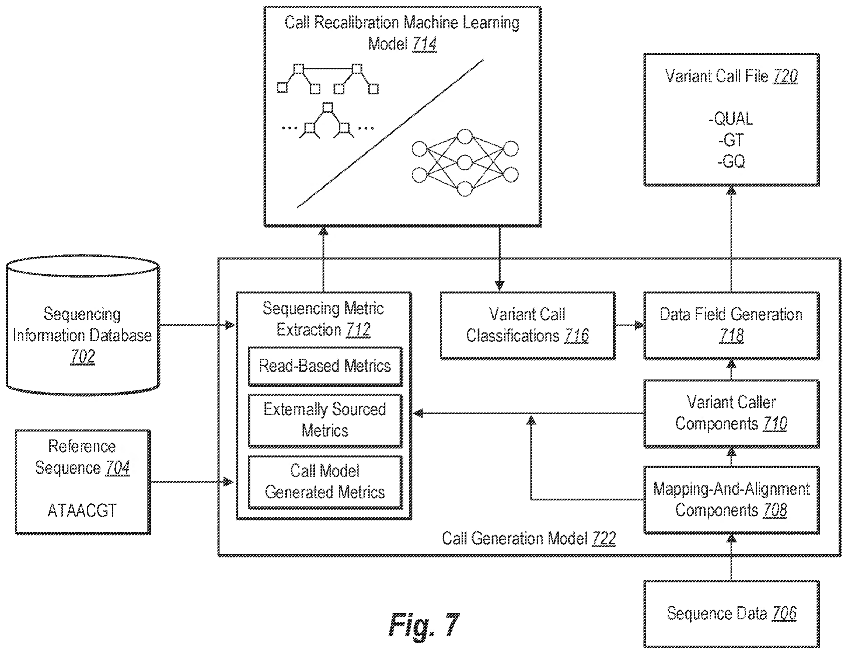

As mentioned, in certain described embodiments, the call recalibration system 106 utilizes a call recalibration machine learning model together with a call generation model to generate a nucleotide base call. In particular, the call recalibration system 106 utilizes the call recalibration machine learning model to modify data fields corresponding to a variant call file representing a nucleotide base call. FIG. 7 illustrates generating a nucleotide base call by modifying a variant call file utilizing a call recalibration machine learning model and call generation model in accordance with one or more embodiments.

As illustrated in FIG. 7, the call recalibration system 106 accesses a sequencing information database 702 (e.g., the sequencing information database 614), a reference sequence 704, and sequence data 706 (e.g., the sequence data 608) extrapolated from one or more nucleotide reads. Indeed, the call recalibration system 106 performs sequencing-metric extraction 712 to extract or re-engineer sequencing metrics as described above in relation to FIGS. 6A-6C. For example, the call recalibration system 106 generates read-based sequencing metrics, externally sourced sequencing metrics, and call model generated sequencing metrics. In some cases, the call recalibration system 106 utilizes mapping-and-alignment components 708 of a call generation model 722 (e.g., the call generation model 610) to determine mapping-and-alignment sequencing metrics as described above. In addition, the call recalibration system 106 utilizes variant caller components 710 of the call generation model 722 to generate variant calling metrics as described above. Further, the call recalibration system 106 determines read-based sequencing metrics and externally source sequencing metrics as well (e.g., from sequencing information database 702 and/or the reference sequence 704).

As further illustrated in FIG. 7, the call recalibration system 106 generates variant call classifications 716. More specifically, the call recalibration system 106 utilizes a call recalibration machine learning model 714 to generate the variant call classifications 716 from the sequencing metrics. For example, the call recalibration machine learning model 714 generates variant call classifications 716 including a false positive classification, a genotype error classification, and a true-positive classification. Specifically, the false positive classification indicates a probability that a nucleotide base call (e.g., a variant call) is a false positive. Conversely, a true-positive classification indicates a probability that a nucleotide base call (e.g., a variant call) is a true positive. Additionally, a genotype error classification indicates a probability of error associated with a genotype for a nucleotide base call (e.g., a variant call).

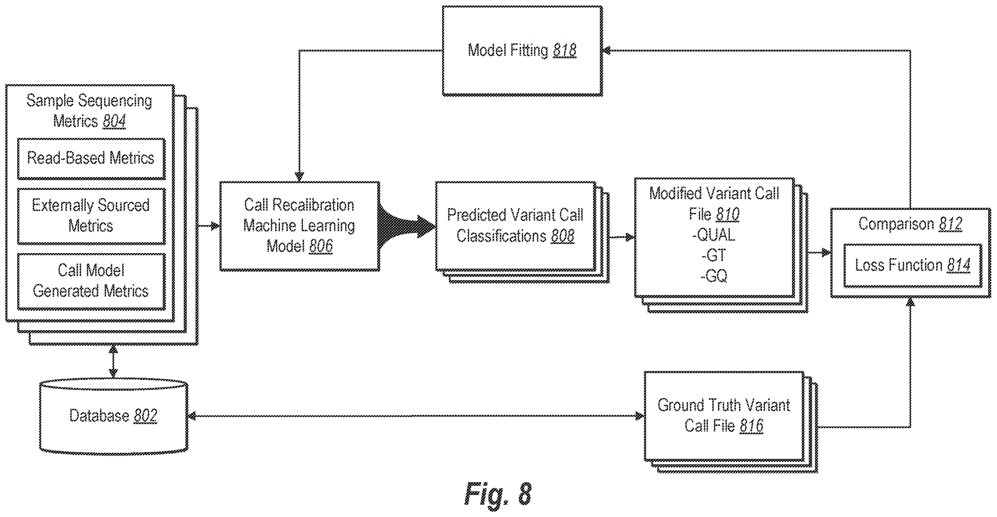

In some cases, the call recalibration machine learning model 714 is an ensemble of gradient boosted trees that processes the sequencing metrics to generate the variant call classifications 716. For instance, the call recalibration machine learning model 714 includes a series of weak learners such as non-linear decision trees that are trained in a logistic regression to generate the variant call classifications 716. In some cases, the call recalibration machine learning model 714 includes metrics within various trees that define how the call recalibration machine learning model 714 processes the sequencing metrics to generate the variant call classifications 716. Additional detail regarding the training of the call recalibration machine learning model 714 is provided below with reference to FIG. 8.

In certain embodiments, the call recalibration machine learning model 714 is a different type of machine learning model such as a neural network, a support vector machine, or a random forest. For example, in cases where the call recalibration machine learning model 714 is a neural network, the call recalibration machine learning model 714 includes one or more layers each with neurons that make up the layer for processing the sequencing metrics. In some cases, the call recalibration machine learning model 714 generates the variant call classifications 716 by extracting latent vectors from the sequencing metrics, passing the latent vectors from layer to layer (or neuron to neuron) to manipulate the vectors until utilizing an output layer (e.g., one or more fully connected layers) to generate the variant call classifications 716 (e.g., as a set of three separate classifications).

As suggested above, in some embodiments, the call recalibration system 106 can utilize multiple call recalibration machine learning models together. For example, the call recalibration system 106 utilizes the call recalibration machine learning model 714 to generate a first set of variant call classifications and further utilizes a second call recalibration machine learning model (e.g., with the same or a different architecture) to generate a second set of variant call classifications. For example, the call recalibration system 106 utilizes two (or more) different call recalibration machine learning models in parallel, each trained with different random seeds (e.g., for different biases to process data differently), resulting in different variant call classifications from the same sequencing metrics.

In some embodiments, the call recalibration system 106 further generates a combined set of variant call classifications from the different variant call classifications generated via the different call recalibration machine learning models. In some cases, the call recalibration system 106 generates variant call classifications (e.g., the variant call classifications 716) from a first set and a second set of variant call classifications generated from a first call recalibration machine learning model and a second call recalibration machine learning model, respectively. For instance, the call recalibration system 106 determines an average or a weighted combination of the first and second set of variant call classifications to generate the combined variant call classifications for recalibrating a nucleotide base call. In some embodiments, the call recalibration system 106 determines a mean for each variant call classification across each call recalibration machine learning model and renormalizes the mean variant call classification. In other embodiments, the call recalibration system 106 learns linear weights and adapts the weights to minimize overall error or loss for the variant call classifications. In still other embodiments, the call recalibration system 106 weights the variant call classifications for each call recalibration machine learning model based on the inverse of average error across the models.

In one or more implementations, the call recalibration system 106 further utilizes a metamodel subsequent to the call recalibration machine learning models. For example, the call recalibration system 106 utilizes a classification-combiner-machine learning model to combine variant call classifications generated from each call recalibration machine learning model-such as by selecting weights to apply to the variant call classifications generated by each call recalibration machine learning model. Indeed, in some cases, the call recalibration system 106 trains the classification-combiner-machine learning model to determine, select, or predict respective weights for call recalibration machine learning models to result in a highest accuracy or a minimized loss.

When generating the variant call classifications 716, in some embodiments, the call recalibration system 106 generates variant call classifications by utilizing statistics to summarize a mapping quality distribution (e.g., a comparative-mapping-quality-distribution metric) of reference supporting reads and alternative supporting reads. For example, the call recalibration system 106 can determine and utilize the mean of the MAPQ for reads supporting an alternative allele as a variant call classification. In these or other embodiments, the call recalibration machine learning model 714 learns from the data that, when the MAPQ of an alternative allele is low and a depth metric is high relative to other MAPQ and depth metrics in distributions, a resultant nucleotide base call is more likely to be a false positive variant. Indeed, as the probability of a false positive variant increases, the MAPQ metrics would likely decrease.

As a further example of generating the variant call classifications 716 utilizing the call recalibration machine learning model 714, in some cases, the call recalibration system 106 compares a mapping quality (e.g., MAPQ) associated with a nucleotide read (e.g., from the sequencing metrics) with a mapping-quality threshold. For instance, the call recalibration system 106 utilizes a mapping-quality threshold such as a threshold difference between best and secondbest alignment scores. Upon determining that the mapping quality does not satisfy the threshold, the call recalibration system 106 adjusts one or more of the variant call classifications 716 accordingly. For instance, the call recalibration system 106 increases a probability of genotype error and/or false positive error based on whether the mapping quality satisfies the corresponding threshold.

In addition (or in the alternative) to the method of generating the variant call classifications 716 just described, the call recalibration system 106 can (i) utilize an accumulation of statistical analyses over complex functions (depending on the architecture of the call recalibration machine learning model 714) to determine how to best fit the data (e.g., based on relationship between the various metrics) or (ii) compare other metrics, such as read depth, base quality, or others associated with a nucleotide base call (e.g., from the sequencing metrics) with corresponding thresholds. The call recalibration system 106 further generates variant call classifications 716 accordingly. For example, in some embodiments, the call recalibration system 106 trains the call recalibration machine learning model 714 to minimize a loss generated from a number of (different types of) sequencing metrics to determine weights and biases that best fit the data (e.g., that result in a reduced or minimized loss) for generating the variant call classifications 716. As another example, upon determining that a read depth fails to satisfy a read-depth threshold (e.g., a maximum read depth corresponding to a particular genomic coordinate or generally across all genomic coordinates), the call recalibration system 106 increases a genotype error probability and/or increases or decreases a false positive probability and a true-positive probability for a corresponding nucleotide base call.

In addition to generating the variant call classifications 716, as further illustrated in FIG. 7, the call recalibration system 106 performs data field generation 718. More specifically, the call recalibration system 106 generates data fields for a nucleotide base call corresponding to a variant call file utilizing the variant caller components 710 of the call generation model 722 and modifies or maintains values for such data fields based the variant call classifications 716. For instance, the call recalibration system 106 modifies various metrics such as quality metrics, mapping metrics, or other metrics associated with the nucleotide base call. In certain embodiments, the nucleotide base call is represented or defined by the variant call file 720 which includes metrics corresponding to the data fields, such as a call-quality metric corresponding to a call-quality field, a genotype metric corresponding to a genotype field, and a genotype-quality metric corresponding to a genotype-quality field.

In certain embodiments, the call recalibration system 106 generates (data fields for) a nucleotide base call utilizing the variant caller components 710 together with the variant call classifications 716. For instance, the call recalibration system 106 generates, utilizing the variant caller components 710, data fields for various metrics of a nucleotide base call such as nucleotide(s) included in the call, a call quality (QUAL), a genotype (GT), and a genotype quality (GQ).

In addition to generating a nucleotide base call via the call generation model 722, the call recalibration system 106 also recalibrates or modifies the nucleotide base call via the variant call classifications 716 from the call recalibration machine learning model 714. In one or more implementations, the call recalibration system 106 modifies the nucleotide base call by modifying or recalibrating data fields for one or more of the metrics associated with the nucleotide base call (e.g., as included within the variant call file 720). For example, the call recalibration system 106 determines updated values for metrics such as the call quality, the genotype, and the genotype quality from the variant call classifications 716. Indeed, the call recalibration system 106 combines or compares the variant call classifications 716 to recalibrate the corresponding metrics of the nucleotide base call included in the variant call file 720.

To update or recalibrate the call-quality metric associated with a nucleotide base call, the call recalibration system 106 determines how each of the variant call classifications 716 impact or affect the base call quality metric and adjusts the base call quality metric accordingly. For example, the call recalibration system 106 determines that a high probability for a genotype error results in a lower overall genotype quality and possibly a different overall call quality. As another example, the call recalibration system 106 determines that a high probability for a false positive variant results in a lower overall call quality. As yet another example, the call recalibration system 106 determines that a high probability for a true positive variant results in a higher overall (variant) call quality. As a further example, if the call recalibration system 106 determines a high probability for a genotype error (e.g., higher than for the other two variant call classifications 716), then the call recalibration system 106 determines that nucleotide base call is most likely a true variant with the wrong genotype. The call recalibration system 106 accordingly updates the genotype along with the genotype quality and the call quality associated with the nucleotide base call.

In one or more implementations, the call recalibration system 106 generates a combination (e.g., a weighted combination or an average) of the variant call classifications 716 to recalibrate the call-quality metric. In particular, the call recalibration system 106 weights the false positive classification, the genotype error classification, and the true-positive classification according to their respective impact on (variant) call quality. In some cases, the call recalibration system 106 weights each variant call classification evenly, while in other cases the call recalibration system 106 determines different weights for each variant call classification. In any event, the call recalibration system 106 determines a weighted combination or a weighted average of the variant call classifications 716 to recalibrate (increase or decrease) a call-quality metric for a nucleotide base call (e.g., an initial variant call).

To update or recalibrate the genotype metric (e.g., within the GT field of the variant call file 720) associated with a nucleotide base call, the call recalibration system 106 utilizes one or more of the variant call classifications 716. For example, the call recalibration system 106 compares the three variant call classifications as the variant call classifications 716 (e.g., the false positive classification, the genotype error classification, and the true-positive classification) to determine which of the variant call classifications 716 has a highest probability. In some cases, the call recalibration system 106 utilizes the variant call classification with the highest probability to recalibrate the genotype metric (e.g., from 0 as corresponding to the reference base to 1 as corresponding to a first alternative supporting read). For instance, if the call recalibration system 106 determines a highest probability for the false positive classification, then the call recalibration system 106 recalibrates the genotype metric accordingly. As another example, if the call recalibration system 106 determines a highest probability for the true-positive classification, then the call recalibration system 106 recalibrates (or refrains from recalibrating) the genotype metric.

In other embodiments, the call recalibration system 106 utilizes only the genotype error probability to modify the genotype metric. For example, if the call recalibration system 106 determines a high genotype error probability, then the call recalibration system 106 recalibrates the genotype metric to indicate a different genotype of a nucleotide base call.

To update or recalibrate the genotype-quality metric (e.g., within the GQ field of the variant call file 720) associated with a nucleotide base call, the call recalibration system 106 utilizes one or more of the variant call classifications 716. More specifically, the call recalibration system 106 determines how each of the variant call classifications 716 affect the genotype-quality metric and recalibrates the genotype-quality metric accordingly (e.g., by increasing or decreasing the quality score between 0 to 10 or 0 to 100 or on some other scale). For example, the call recalibration system 106 determines that a higher genotype error probability (generally) indicates a lower genotype-quality metric, and the call recalibration system 106 reduces the metric accordingly.

In some cases, the call recalibration system 106 determines a combination (e.g., a weighted combination or a weighted average) of the variant call classifications 716 to modify the genotype-quality metric. For example, the call recalibration system 106 determines a combined effect that the variant call classifications 716 have on the genotype-quality metric. As another example, the call recalibration system 106 determines individual impacts that each variant call classification has on the genotype-quality metric and weights each variant call classification accordingly. The call recalibration system 106 further recalibrates the genotype-quality metric by increasing or decreasing its value based on the indicated probabilities associated with each of the variant call classifications 716.

As described, the call recalibration system 106 generates variant call classifications 716 and a nucleotide base call from the same set of sequencing metrics (or a subset of the sequencing metrics that are shared between the call recalibration machine learning model 714 and the call generation model 722). Indeed, the call recalibration system 106 utilizes the call recalibration machine learning model 714 to generate the variant call classifications 716 from sequencing metrics while also generating a nucleotide base call for a sample sequence. Indeed, the call recalibration system 106 can operate the call recalibration machine learning model 714 in parallel with the call generation model 722 to generate metrics for a nucleotide base call and variant call classifications 716 for recalibrating the generated metrics.

As further illustrated in FIG. 7, the call recalibration system 106 generates a variant call file 720. In particular, the call recalibration system 106 generates a variant call file 720 that represents or defines a nucleotide base call from the sequencing metrics corresponding to a genomic coordinate. As shown, the variant call file 720 includes various call metrics such as a call-quality metric (QUAL), a genotype metric (GT), and a genotype-quality metric (GQ). To generate the variant call file 720, as described, the call recalibration system 106 generates metrics for a nucleotide base call utilizing the call generation model 722 and recalibrates the nucleotide base call utilizing the variant call classifications 716 from the call recalibration machine learning model 714.

In one or more implementations, the call recalibration system 106 updates or otherwise modifies the data fields for the variant call file 720 according to particular algorithms. After modifying such data fields, the call recalibration system 106 can generate the variant call file 720 (e.g., a post-filter variant call file) to include metrics reflecting the updated data fields for QUAL, GT, and GQ. For instance, in some cases, the call recalibration system 106 updates the QUAL field for every variant based on the probability of a false positive variant (e.g., the false positive classification). As indicated above, in some cases, QUAL indicates the probability that there is some kind of variant (or other nucleotide base call) at a given location, measured in PHRED scale.

In addition, if the call recalibration system 106 determines that the highest probability from among the three variant call classifications (as the variant call classifications 716) is the genotype error classification (e.g., the probability of a het/hom error), then the call recalibration system 106 updates the GQ field while preserving or maintaining the GT field. Specifically, in some embodiments, the call recalibration system 106 updates the GQ field based on the true-positive classification (e.g., the probability of a true genotype).

Further, if the call recalibration system 106 determines that the highest probability from among the variant call classifications 716 is the true-positive classification, in some cases, the call recalibration system 106 updates both the GQ field and the GT field. Specifically, the call recalibration system 106 updates the GQ field based on the genotype error classification and further updates the GT field to switch the genotype depending on whether the existing GT is 0/X or X/X (where X is a non-zero value).

If the call recalibration system 106 determines that neither the true-positive classification nor the genotype error classification has the highest probability among the variant call classifications 716, in some embodiments, the call recalibration system 106 updates the GQ field. In other words, if the call recalibration system 106 determines that the false positive classification has the highest probability, the call recalibration system 106 updates the GQ field. In particular, the call recalibration system 106 updates the GQ field based on the probability indicated by the true-positive classification.

As suggested above, in some embodiments, the call recalibration system 106 increases or decreases a base call quality metric (e.g., Q score) for a nucleotide base call. Based on the variant call classifications 716, for example, the call recalibration system 106 increases base call quality metrics for nucleotide base calls that would not have previously passed a quality filter and determines that the increased base call quality metrics now passes the quality filter. In some such cases, the call recalibration system 106 includes nucleotide base calls with such increased base call quality metrics (passing the quality filter) in a post-filter variant call file. By contrast, in other cases, the call recalibration system 106 decreases base call quality metrics for nucleotide base calls that previously would have passed a quality filter and determines that the decreased base call quality metrics now fail the quality filter. In some such cases, the call recalibration system 106 excludes nucleotide base calls with decreased base call quality metrics (failing the quality filter) from a post-filter variant call file, but includes the nucleotide base calls with such decreased base call quality metrics in a pre-filter variant call file.

For example, the call recalibration system 106 can remove false positive variant calls and recover false negative variant calls by changing corresponding base call quality metrics. To remove a false positive, in some cases, the call recalibration system 106 decreases the base call quality metric of a nucleotide base call that initially passed a quality filter-based on the variant call classifications 716 from the call recalibration machine learning model 714. Based on determining the decreased base call quality metric falls below a threshold metric (e.g., a Q score of 3.0 or 10.0), the call recalibration system 106 determines that the nucleotide base call no longer passes the quality filter. The call recalibration system 106 thus filters out, or removes, the false positive-nucleotide base call that initially passed the filter by changing its base call quality metric.

In addition to removing false positive variant calls based on changes to base call quality metrics, the call recalibration system 106 can remove false positive variant calls based on changes to genotype. To remove a false positive, in some cases, the call recalibration system 106 changes a genotype of an initial nucleotide base call indicating a different nucleotide base than a reference base (e.g., GT = 1 or 2) to a genotype of an updated nucleotide base call indicating a same nucleotide base as the reference base (e.g., GT = 0)-based on the variant call classifications 716 from the call recalibration machine learning model 714. Based on the genotype being the same as the reference base, the call recalibration system 106 does not identify the nucleotide base call as a variant and, in some cases, excludes data for the nucleotide base call from a variant call file.

To recover a false negative, the call recalibration system 106 increases the base call quality metric of a nucleotide base call that initially failed a quality filter-based on the variant call classifications 716 from the call recalibration machine learning model 714. Based on determining the increased base call quality metric exceeds a threshold metric, the call recalibration system 106 determines that the nucleotide base call passes the quality filter. The call recalibration system 106 thus recovers a false-negative-nucleotide base call that was initially filtered out by changing its base call quality metric.

In addition to recovering false negative variant calls based on changes to base call quality metrics, the call recalibration system 106 can recover false negative variant calls based on changes to genotype. To recover a false negative, in some cases, the call recalibration system 106 changes a genotype of an initial nucleotide base call indicating the same nucleotide base as a reference base (e.g., GT = 0) to a different genotype of an updated nucleotide base call indicating a different nucleotide base than the reference base (e.g., GT = 1 or 2)-based on the variant call classifications 716 from the call recalibration machine learning model 714. Based on the differing genotype of the updated nucleotide base call and a passing base call quality metric, the call recalibration system 106 identifies the nucleotide base call as a variant and includes the nucleotide base call within a variant call file.

Indeed, in some implementations, the call recalibration system 106 operates in a specific sequential order utilizing the call generation model 722 and the call recalibration machine learning model 714. For example, the call recalibration system 106 generates a FASTQ file by converting a BCL file to FASTQ. In addition, the call recalibration system 106 (subsequently) utilizes the mapping-and-alignment components 708 of the call generation model 722 to map and align nucleotide bases from a sample nucleotide sequence. In some cases, the call recalibration system 106 maps and aligns the nucleotide bases of the sample sequence in relation to a reference sequence (e.g., reference genome) and/or various alternative supporting reads.

After mapping and aligning, as described herein, the call recalibration system 106 then utilizes the variant caller components 710 of the call generation model 722 to generate an initial nucleotide base call for the sample sequence corresponding to a particular genomic coordinatebased on various sequencing metrics. After or at the same time, the call recalibration system 106 also applies the call recalibration machine learning model 714 to generate the variant call classifications 716 from sequencing metrics extracted via the mapping and aligning, the variant calling, and/or from other sources as described above. Based on the variant call classifications 716, the call recalibration system 106 recalibrates the nucleotide base call (e.g., by modifying various data fields corresponding to specific metrics of the nucleotide base call such as QUAL, GT, and GQ).

In some cases, the call recalibration system 106 further applies a quality filter to the nucleotide base call to determine whether the nucleotide base call passes the quality filter (e.g., a hard pass filter of Q20 or other Q score). The call recalibration system 106 subsequently identifies a subset of nucleotide base calls that represent variants from reference bases and pass the quality filter. The call recalibration system 106 further generates a modified or updated variant call file (e.g., the variant call file 720) that includes the subset of nucleotide base calls and recalibrated metrics for the subset of nucleotide base calls, such as updated QUAL metrics, updated GT metrics, and/or updated GQ metrics.

As mentioned above, in certain embodiments, the call recalibration system 106 trains or tunes a call recalibration machine learning model (e.g., the call recalibration machine learning model 714). In particular, the call recalibration system 106 utilizes an iterative training process to fit a call recalibration machine learning model by adjusting or adding decision trees or learning parameters that result in accurate variant call classifications (e.g., variant call classifications 716). FIG. 8 illustrates training a call recalibration machine learning model in accordance with one or more embodiments.

As illustrated in FIG. 8, the call recalibration system 106 accesses sample sequencing metrics 804 from a database 802 (e.g., the database 116). For example, the call recalibration system 106 accesses sample sequencing metrics including sample read-based metrics, sample externally sourced sequencing metrics, and sample call model generated sequencing metrics. In some cases, the sample sequencing metrics 804 have a corresponding ground truth variant call file 816 associated with them, where the ground truth variant call file 816 indicates an actual nucleotide base call and its various metrics that result from the sample sequencing metrics 804. For instance, the call recalibration system 106 utilizes sample sequencing metrics 804 and ground truth variant call files from a training dataset from the food and drug administration, called the PrecisionFDA dataset. In some cases, the sample sequencing metrics 804 include a subset of sample sequencing metrics for each nucleotide base call in a ground truth variant call file. The ground truth variant call file can have a ground truth variant call (e.g., genotype metric in a genotype field) and/or a ground truth base call corresponding to each subset of sample sequencing metrics.

As further illustrated in FIG. 8, the call recalibration system 106 generates predicted variant call classifications 808 based on the sample sequencing metrics 804. Specifically, the call recalibration system 106 utilizes a call recalibration machine learning model 806 (e.g., the call recalibration machine learning model 714) to generate the predicted variant call classifications 808. Indeed, in some embodiments, the call recalibration machine learning model 806 generates a set of three predicted variant call classifications 808 including a predicted false positive classification, a predicted genotype error classification, and a predicted true-positive classification. The predicted variant call classifications 808 can accordingly take the form of any of the variant call classifications described above.

Based on the predicted variant call classifications 808, the call recalibration system 106 determines nucleotide base calls and generates a modified variant call file 810 comprising the nucleotide base calls and corresponding fields. As indicated above, the call recalibration system 106 can utilize (i) a call generation model to generate an initial nucleotide base call and (ii) the call recalibration machine learning model 806 to modify data fields corresponding to a variant call file for the nucleotide base call. Such modified or recalibrated values are output in the modified variant call file 810 by, for example the call generation model. For example, the call recalibration system 106 determines recalibrated values for particular metrics within the modified variant call file 810, including a call-quality metric (QUAL), a genotype metric (GT), and a genotype-quality metric (GQ).

As further illustrated in FIG. 8, the call recalibration system 106 performs a comparison 812. Specifically, the call recalibration system 106 performs the comparison 812 between (i) variant nucleotide base calls and/or data fields in the modified variant call file 810 and (ii) variant nucleotide base calls and/or data fields in the ground truth variant call file 816. In some embodiments, the call recalibration system 106 utilizes a loss function 814 to compare variant nucleotide base calls and/or data fields from the two variant call files (e.g., to determine an error or a measure of loss between them). For instance, in cases where the call recalibration machine learning model 806 is an ensemble of gradient boosted trees, the call recalibration system 106 utilizes a mean squared error loss function (e.g., for regression) and/or a logarithmic loss function (e.g., for classification) as the loss function 814.

By contrast, in embodiments where the call recalibration machine learning model 806 is a neural network, the call recalibration system 106 can utilize a cross entropy loss function, an L1 loss function, or a mean squared error loss function as the loss function 814. For example, the call recalibration system 106 utilizes the loss function 814 to determine a difference between variant nucleotide base calls and/or data fields from the modified variant call file 810 and the ground truth variant call file 816.

As further illustrated in FIG. 8, the call recalibration system 106 performs model fitting 818. In particular, the call recalibration system 106 fits the call recalibration machine learning model 806 based on the comparison 812. For instance, the call recalibration system 106 performs modifications or adjustments to the call recalibration machine learning model 806 to reduce the measure of loss from the loss function 814 for a subsequent training iteration.

For gradient boosted trees, for example, the call recalibration system 106 trains the call recalibration machine learning model 806 on the gradients of the errors determined by the loss function 814. For instance, the call recalibration system 106 solves a convex optimization problem (e.g., of infinite dimensions) while regularizing the objective to avoid overfitting. In certain implementations, the call recalibration system 106 scales the gradients to emphasize corrections to under-represented classes (e.g., where there are significantly more true positives than false positive variant calls).

In some embodiments, the call recalibration system 106 adds a new weak learner (e.g., a new boosted tree) to the call recalibration machine learning model 806 for each successive training iteration as part of solving the optimization problem. For example, the call recalibration system 106 finds a feature (e.g., a sequencing metric) that minimizes a loss from the loss function 814 and either adds the feature to the current iteration's tree or starts to build a new tree with the feature.

In addition or in the alternative to gradient boosted decision trees, the call recalibration system 106 trains a logistic regression to learn parameters for generating one or more variant call classifications such as a true-positive classification. To avoid overfitting, the call recalibration system 106 further regularizes based on hyperparameters such as the learning rate, stochastic gradient boosting, the number of trees, the tree-depth(s), complexity penalization, and L1/L2 regularization.

In embodiments where the call recalibration machine learning model 806 is a neural network, the call recalibration system 106 performs the model fitting 818 by modifying internal parameters (e.g., weights) of the call recalibration machine learning model 806 to reduce the measure of loss for the loss function 814. Indeed, the call recalibration system 106 modifies how the call recalibration machine learning model 806 analyzes and passes data between layers and neurons by modifying the internal network parameters. Thus, over multiple iterations, the call recalibration system 106 improves the accuracy of the call recalibration machine learning model 806.

Indeed, in some cases, the call recalibration system 106 repeats the training process illustrated in FIG. 8 for multiple iterations. For example, the call recalibration system 106 repeats the iterative training by selecting a new set of sequencing metrics for each nucleotide base call along with a corresponding ground truth nucleotide base call in a corresponding ground truth variant call file. The call recalibration system 106 further generates a new set of predicted variant call classifications for each iteration along with a new modified variant call file. As described above, the call recalibration system 106 also compares a variant nucleotide base calls and/or data fields from the modified variant call file at each iteration with the corresponding variant nucleotide base calls and/or data fields from the corresponding ground truth variant call file and further performs model fitting 818. The call recalibration system 106 repeats this process until the call recalibration machine learning model 806 generates predicted variant call classifications that result in variant calls that satisfies a threshold measure of loss. In some embodiments, the call recalibration system 106 performs the training process of FIG. 8 for homozygous reference coordinates to update or modify variant calls of these coordinates and to thereby recover false negative variant calls (based on simulating haploid data from diploid data and modifying inputs and outputs of the call recalibration machine learning model 806 as described).

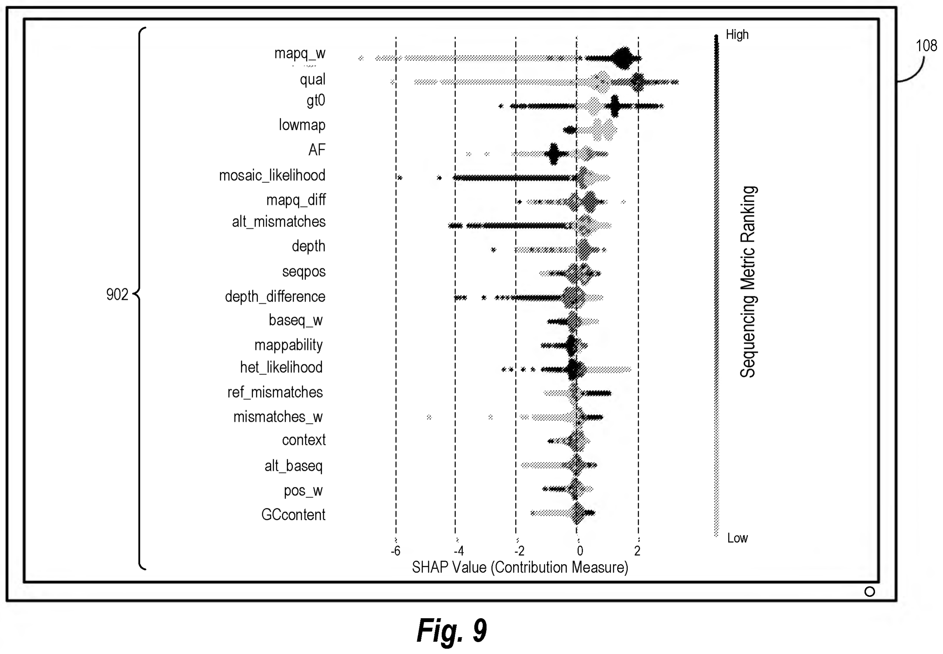

As mentioned above, in certain described embodiments, the call recalibration system 106 generates and provides contribution measures associated with sequencing metrics. In particular, the call recalibration system 106 determines respective contribution measures indicating how impactful individual sequencing metrics are in determining a particular nucleotide base call. FIG. 9 illustrates an example visualization of contribution measures for sequencing metrics associated with a nucleotide base call in accordance with one or more embodiments.

As illustrated in FIG. 9, the client device 108 displays a contribution-measure interface 902 that includes individual depictions of contribution measures associated with corresponding sequencing metrics. Indeed, the call recalibration system 106 determines a contribution measure for a sequencing metric based on how impactful or influential the sequencing metric is on a final nucleotide base call. Unlike many existing sequencing systems that utilize deep learning architectures, the structure of the call generation model used by the call recalibration system 106 facilitates the determination of such contribution measures on a metric-by-metric basis.

For example, the call recalibration system 106 determines contribution measures by determining Shapley Additive Explanation (SHAP) values for each of the sequencing metrics for a nucleotide base call. Specifically, the call recalibration system 106 determines a SHAP value by determining an impact of a sequencing metric as compared to the results of a baseline value (e.g., a baseline value for the sequencing metric). As shown in FIG. 9, the call recalibration system 106 determines contribution measures for a number of listed sequencing metrics, where the thicker (e.g., more bulbous) portions of the graphs for each sequencing metric (roughly) indicate its contribution measure.

As further shown in FIG. 9, the call recalibration system 106 can rank the sequencing metrics according to contribution measures as well. For instance, the call recalibration system 106 determines that the contribution for the mapq_p metric is highest among those displayed within the contribution-measure interface 902, followed by the qual metric, the gt0 metric, and so forth down the list.

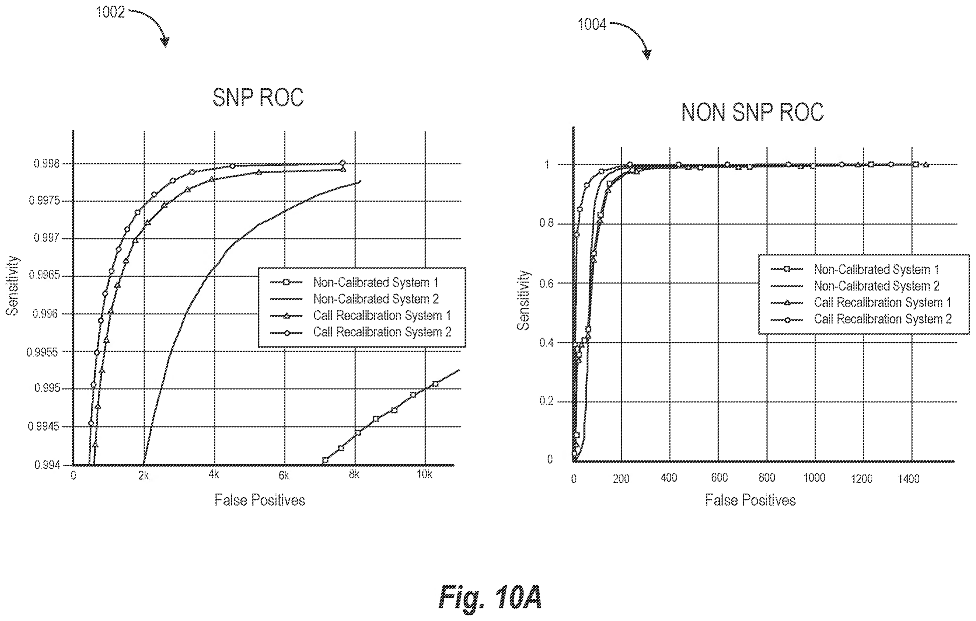

As mentioned above, in certain described embodiments, the call recalibration system 106 improves in accuracy over existing sequencing systems. In particular, the call recalibration system 106 reduces false positive variant nucleotide base calls and false negative variant nucleotide base calls compared to existing sequencing systems. Indeed, by utilizing a call recalibration machine learning model to recalibrate nucleotide base calls, the call recalibration system 106 even improves over previous versions of the call generation model that did not utilize a call recalibration machine learning model (but which still outperform other systems). FIGS. 10A-10B illustrate graphs and tables of experiments demonstrating the accuracy improvements of the call recalibration system 106 as compared to some existing systems.

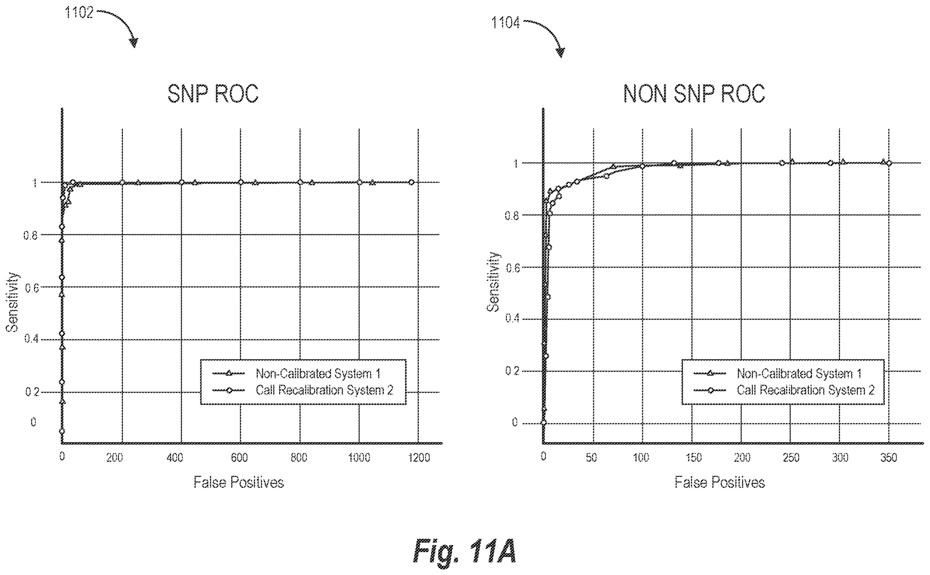

For reference and as depicted in FIGS. 10A-10B and 11A-11B, the name "Non-Recalibrated System 1" refers to an existing sequencing system that uses a linear reference genome for variant calling. By contrast, the name "Non-Recalibrated System 2" refers to an existing sequencing system that uses a graph reference genome for variant calling. Further, the name "Call Recalibration System 1" refers to an embodiment of the call recalibration system 106 that is not configured for nucleotide base calls at multiallelic genomic coordinates, haploid genomic coordinates, and would-be homozygous reference genomic coordinates. By contrast, the name "Call Recalibration System 2" refers to an embodiment of the call recalibration system 106 that is not configured for nucleotide base calls at multiallelic genomic coordinates, haploid genomic coordinates, and would-be homozygous reference genomic coordinates.

As illustrated in FIG. 10A, a graph 1002 depicts a number of receiver operating characteristic (ROC) curves that compare SNP false positives for two variations of the call recalibration system 106 with those of two non-recalibrated systems. The graph 1002 depicts portions of ROC curves representing sensitivity over false positive variants detected, where sensitivity represents a number of correctly determined true positive variant calls divided by the sum of true positive variant calls and false positive variant calls. In particular, the graph 1002 depicts ROC curves for different embodiments of the call recalibration system 106 utilizing the call recalibration machine learning model-that is, "Call Recalibration System 1" and "Call Recalibration System 2," as described above. The experiment was performed using the PrecisionFDA truth set (e.g., the Precision FDA HG002 high confidence truthset). Generally, curves that trend upward and to the left in the graph 1002 are more accurate. As shown, embodiments of the call recalibration system 106 exhibits improved accuracy over each of the three non-recalibrated systems, with higher sensitivity and fewer false positive variant calls comparatively. As shown by the improvements between the ROC curves for the Call Recalibration System 1 to the Call Recalibration System 2, the gain in sensitivity is due in part to recovering false negative variant calls at genomic coordinates that would have been identified as homozygous reference genotypes by another sequencing system.

Additionally, the graph 1004 depicts a number of ROC curves that compare non-SNP (e.g., indel) false positive variant calls for different embodiments of the call recalibration system 106 with those of a couple non-recalibrated systems, Non-Recalibrated System 1 and Non-Recalibrated System 2. The graph 1004 depicts ROC curves representing sensitivity over false positive variants detected. In particular, the graph 1004 depicts an ROC curve for an embodiment of the call recalibration system 106-configured for nucleotide base calls at multiallelic genomic coordinates, haploid genomic coordinates, and would-be homozygous reference genomic coordinates-that removes or reduces the bump or jog prevalent in the non-recalibrated systems at a sensitivity of ~0.4 (instead continuing smoothly upward on a nearly vertical trajectory). Indeed, due at least in part to the improvements at multiallelic genomic coordinates, an embodiment of the call recalibration system 106 (here, Call Recalibration System 2) exhibits fewer false positive variant calls at similar sensitivities, as compared to one or more non-recalibrated systems that do not recalibrate multiallelic variants (e.g., the Non-Recalibrated System 2). The experiment was performed using the PrecisionFDA truth set (e.g., the Precision FDA HG002 high confidence truth set).

As illustrated in FIG. 10B, table 1006 corresponds to the graph 1002, while the table 1008 corresponds to the graph 1004. The numbers of the table 1006 and the table 1008 are taken at a best F-measure point for the curves in each of the graphs 1002 and 1004, respectively. As shown in table 1006, both embodiments of the call recalibration system 106 have fewer false negative variant calls (FN), fewer false positive variant calls (FP), and more true positives (TP) than any of the non-recalibrated systems. For example, at the best F-measure point, an embodiment of the call recalibration system 106-shown as Call Recalibration System 2 and is configured for nucleotide base calls at multiallelic genomic coordinates, haploid genomic coordinates, and would-be homozygous reference genomic coordinates-produces 7309 false negative variant calls and 2801 false positive variant calls, as shown in the table 1006. The other embodiment of the call recalibration system 106-shown as Call Recalibration System 1 but is not configured for nucleotide base calls at multiallelic genomic coordinates and would-be homozygous reference genomic coordinates-generates 7717 false negative variant calls and 3216 false positive variant calls. Similarly, the call recalibration system 106 has fewer het/hom errors, better recall, and higher precision as well.

As illustrated in table 1008, the embodiments of the call recalibration system 106 outperforms the non-recalibrated systems for non-SNP scenarios as well. For example, at the best F-measure point of the table 1008, an embodiment of the call recalibration system 106-shown as Call Recalibration System 2 and is configured for nucleotide base calls at multiallelic genomic coordinates, haploid genomic coordinates, and would-be homozygous reference genomic coordinates-produces 513 false positive variant calls while the other embodiment of the call recalibration system 106 produces 618 false positive variant calls. Both non-recalibrated systems produce far more false positive variant calls. The embodiments of the call recalibration system 106 also have higher precision than any of the non-recalibrated systems.

In addition to the diploid accuracy improvements shown in FIGS. 10A-10B, FIGS. 11A-11B illustrate haploid accuracy improvements. Specifically, the graphs 1102 and 1104 each depict two ROC curves, one for the call recalibration system 106 and one for a non-recalibrated system. For instance, the graph 1102 depicts ROC curves for SNPs, while the graph 1104 depicts ROC curves for non-SNPs (e.g., indels). In each case, as a result of the accuracy improvements at haploid coordinates, the call recalibration system 106 has higher sensitivity and fewer false positive variant calls as compared to the non-recalibrated system. Indeed, in each of the graphs 1102 and 1104, the ROC curve for the call recalibration system 106 is improved, with a best F-measure point that is located at a cross-section indicating fewer false positive variant calls at (approximately) the same sensitivity as compared to the non-recalibrated system. The experiments for the graphs 1102 and 1104 were performed using the PrecisionFDA truth set.

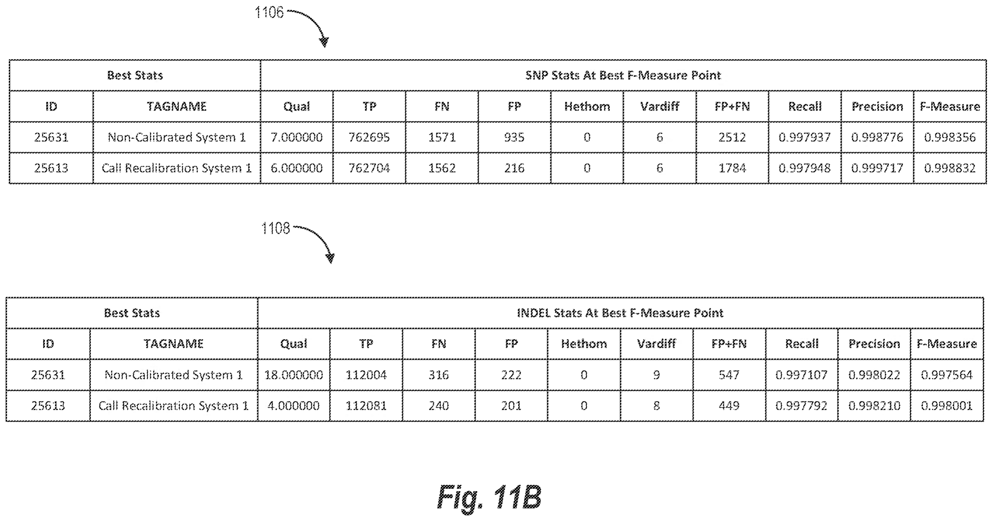

As illustrated in FIG. 11B, the table 1106 corresponds to the graph 1102, and the table 1108 corresponds to the graph 1104. Indeed, the table 1106 indicates SNP results for the call recalibration system 106 compared to the non-recalibrated system at a best F-measure point. As shown, the call recalibration system 106 has more true positives, fewer false negative variant calls, fewer false positive variant calls, higher recall, and higher precision for SNPs. Looking to the table 1108, the call recalibration system 106 produces (at the best F-measure point) more true positives, fewer false negative variant calls, fewer false positive variant calls, higher recall, and higher precision than the non-recalibrated system for non-SNPs as well.

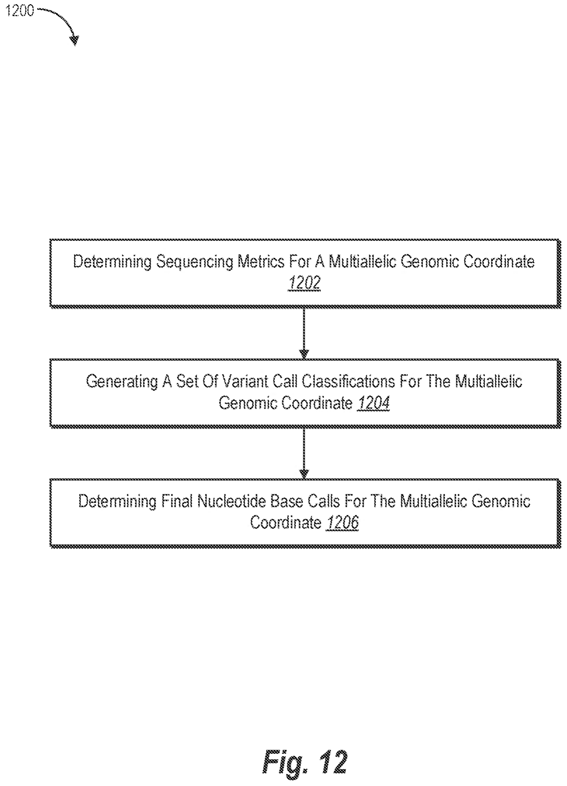

Turning now to FIGS. 12-14, these figures illustrate example flowcharts, each of a series of acts of generating a final nucleotide base call or variant call based on variant call classifications from a call recalibration machine learning model in accordance with one or more embodiments. While FIGS. 12-14 illustrate acts according to one embodiment, alternative embodiments may omit, add to, reorder, and/or modify any of the acts shown in FIGS. 12-14. The acts of FIGS. 12-14 can be performed as part of a method. Alternatively, a non-transitory computer readable storage medium can comprise instructions that, when executed by one or more processors, cause a computing device to perform the acts depicted in FIGS. 12-14. In still further embodiments, a system comprising at least one processor and a non-transitory computer readable medium comprising instructions that, when executed by one or more processors, cause the system to perform the acts of FIGS. 12-14.

As shown in FIG. 12, the series of acts 1200 includes an act 1202 of determining sequencing metrics for a multiallelic genomic coordinate. In particular, the act 1202 can include determining sequencing metrics for nucleotide base calls of nucleotide reads corresponding to a multiallelic genomic coordinate of a sample nucleotide sequence.

In addition, the series of acts 1200 includes an act 1204 of generating a set of variant call classifications for the multiallelic genomic coordinate. In particular, the act 1204 can involve generating, utilizing a call recalibration machine learning model and based on the sequencing metrics, a set of variant call classifications comprising a reference probability of a homozygous reference genotype at the multiallelic genomic coordinate, a differing genotype probability of a genotype error at the multiallelic genomic coordinate, and a correct variant probability of a correct variant call genotype at the multiallelic genomic coordinate.

For example, generating the reference probability can include determining a probability that a genotype at the multiallelic genomic coordinate is a homozygous genotype with respect to a reference genome. Generate the differing genotype probability can include determining a probability that a predicted genotype for the multiallelic genomic coordinate is an incorrect genotype or an incorrect allele in the predicted genotype. Generating the correct variant probability can include determining a probability that a predicted genotype for the multiallelic genomic coordinate is correct as initially determined by a call generation model.

As further illustrated in FIG. 12, the series of acts 1200 includes an act 1206 of determining final nucleotide base calls for the multiallelic genomic coordinate. In particular, the act 1206 can involve determining final nucleotide base calls for the multiallelic genomic coordinate based on the set of variant call classifications. For example, the act 1206 can involve predicting two nucleotide bases from three or more candidate alleles at the multiallelic genomic coordinate.

The series of acts 1200 can also include an act of modifying a base call quality metric or a genotype quality metric based on the set of variant call classifications. Further, the series of acts 1200 can include an act of generating a variant call file that includes the modified base call quality metric or the modified genotype quality metric. In addition, the series of acts 1200 can include an act of generating updated genotype likelihoods for candidate nucleotide base calls of alleles at the multiallelic genomic coordinate. In some embodiments, the series of acts 1200 includes an act of generating a variant call file that includes the updated genotype likelihoods.

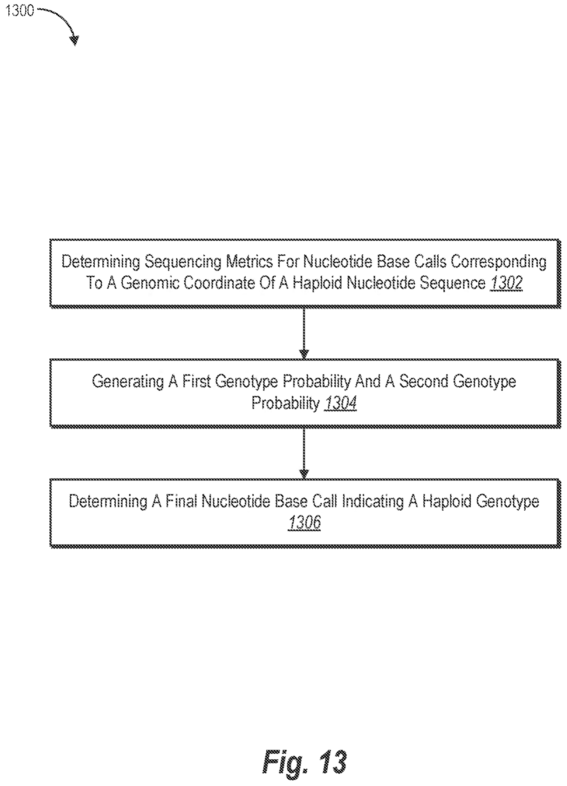

As shown in FIG. 13, the series of acts 1300 includes an act 1302 of determining sequencing metrics for nucleotide base calls corresponding to a genomic coordinate of a haploid nucleotide sequence. In particular, the act 1302 can involve determining sequencing metrics for nucleotide base calls of nucleotide reads corresponding to a genomic coordinate of a haploid nucleotide sequence from a sample.

The series of acts 1300 can also include an act 1304 of generating a first genotype probability and a second genotype probability. In particular, the act 1304 can involve generating, utilizing a call recalibration machine learning model and based on the sequencing metrics, a first genotype probability of a first genotype at the genomic coordinate and a second genotype probability of a second genotype at the genomic coordinate. In some cases, the act 1304 includes acts of generating the first genotype probability comprises generating a probability that the first genotype at the genomic coordinate is a haploid reference genotype and generating the second genotype probability comprises generating a probability that the second genotype at the genomic coordinate is a haploid alternate genotype.

Generating the first genotype probability can include utilizing a layer of the call recalibration machine learning model to modify a homozygous reference probability of a homozygous reference genotype at the genomic coordinate to generate a haploid reference probability of a reference genotype at the genomic coordinate. Generating the second genotype probability can include utilizing the layer of the call recalibration machine learning model to modify a homozygous alternate probability of a homozygous alternate genotype at the genomic coordinate to generate a haploid alternate probability of an alternate genotype at the genomic coordinate.

In some cases, the act 1304 involves generating, for the genomic coordinate utilizing one or more layers of the call recalibration machine learning model, a first confidence score corresponding to a first genotype, a second confidence score corresponding to a second genotype, and a third confidence score corresponding to a third genotype. The act 1304 can also involve excluding the second confidence score corresponding to the second genotype and normalizing the first confidence score and the third confidence score utilizing a softmax model to generate the first genotype probability and the second genotype probability.

As further shown, the series of acts 1300 can include an act 1306 of determining a final nucleotide base call indicating a haploid genotype. In particular, the act 1306 can involve determining a final nucleotide base call indicating a haploid genotype for the genomic coordinate based on the first genotype probability and the second genotype probability. For example, the act 1306 can involve determining one of: a haploid alternate genotype for the genomic coordinate, a modified base call quality metric, a modified genotype metric, and a modified genotype quality metric based on determining that the second genotype probability exceeds the first genotype probability or a haploid reference genotype for the genomic coordinate, a modified base call quality metric, and a modified genotype quality metric based on determining that the first genotype probability exceeds the second genotype probability.

In some embodiments, the series of acts 1300 includes an act of converting a haploid reference genotype call generated by a call generation model to a diploid homozygous reference genotype call as an input for the call recalibration machine learning model. The series of acts 1300 can include an act of converting a haploid alternate genotype call generated by the call generation model to a diploid homozygous alternate genotype call as an input for the call recalibration machine learning model. Additionally, the series of acts 1300 can include an act of generating, utilizing the call recalibration machine learning model, the first genotype probability and the second genotype probability based further on the diploid homozygous reference genotype call or the diploid homozygous alternate genotype call.

In certain embodiments, the series of acts 1300 includes an act of downsampling diploid sequencing metrics to simulate haploid sequencing metrics corresponding to the haploid nucleotide sequence. Downsampling diploid sequencing metrics to simulate haploid sequencing metrics can include acts of selecting a subset of diploid nucleotide reads from the sample to simulate haploid nucleotide reads and selecting, based on nucleotide base calls of the subset of diploid nucleotide reads, a subset of genomic coordinates exhibiting homozygous reference genotypes or homozygous alternate genotypes as indicated by a call generation model or as indicated by a ground-truth base-call dataset (e.g., a well-curated truth set such as PrecisionFDA v4.2.1).

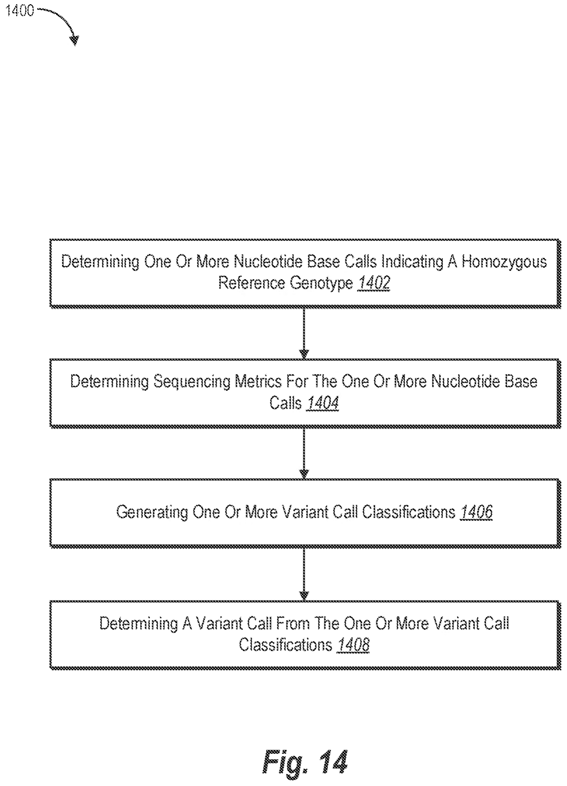

As shown in FIG. 14, the series of acts 1400 includes an act 1402 of determining one or more nucleotide base calls indicating a homozygous reference genotype. In particular, the act 1402 can involve determining, for one or more nucleotide reads, one or more nucleotide base calls indicating a homozygous reference genotype at a genomic coordinate of a sample nucleotide sequence.

The series of acts 1400 can include an act 1404 of determining sequencing metrics for the one or more nucleotide base calls. In particular, the act 1404 can involve determining sequencing metrics for the one or more nucleotide base calls corresponding to the genomic coordinate. For example, the act 1404 can involve determining one or more of read-based sequencing metrics, externally sourced sequencing metrics, or call model generated sequencing metrics for the genomic coordinate indicated as having a homozygous reference genotype.

As shown, the series of acts 1400 can include an act 1406 of generating one or more variant call classifications. In particular, the act 1406 can involve generating, utilizing a call recalibration machine learning model and based on the sequencing metrics from the one or more nucleotide base calls, one or more variant call classifications indicating an accuracy of identifying a variant at the genomic coordinate.

As further illustrated in FIG. 14, the series of acts 1400 can include an act 1408 of determining a variant call from the one or more variant call classifications. In particular, the act 1408 can involve determining a variant call for the genomic coordinate based on the one or more variant call classifications. For example, the act 1408 can involve receiving, from a call generation model, an indication of the homozygous reference genotype at the genomic coordinate and determining the variant call for the genomic coordinate by modifying the homozygous reference genotype to a different genotype based on the one or more variant call classifications.

In some embodiments, the series of acts 1400 includes an act of identifying a previous homozygous reference genotype call from a call generation model for the sample at the genomic coordinate. Further, the series of acts 1400 includes an act of identifying a ground truth base call for the sample at the genomic coordinate and an act of modifying the call recalibration machine learning model based on a comparison of the variant call for the genomic coordinate and the ground truth base call for the genomic coordinate. The series of acts 1400 can include an act of updating one or more of a call quality field, a genotype field, or a genotype quality field corresponding to a variant call file based on the one or more variant call classifications.

In certain implementations, the series of acts 1400 includes an act of determining, for the genomic coordinate, one of: a homozygous alternate genotype based on determining that a true positive classification (e.g., a homozygous alternate classification) has a highest probability from among the one or more variant call classifications, a heterozygous genotype based on determining that a genotype error classification (e.g., a heterozygous genotype classification) has the highest probability from among the one or more variant call classifications, or a homozygous reference genotype based on determining that neither the true positive classification nor the genotype error classification has the highest probability from among the one or more variant call classifications.

The methods described herein can be used in conjunction with a variety of nucleic acid sequencing techniques. Particularly applicable techniques are those wherein nucleic acids are attached at fixed locations in an array such that their relative positions do not change and wherein the array is repeatedly imaged. Embodiments in which images are obtained in different color channels, for example, coinciding with different labels used to distinguish one nucleotide base type from another are particularly applicable. In some embodiments, the process to determine the nucleotide sequence of a target nucleic acid (i.e., a nucleic acid polymer) can be an automated process. Preferred embodiments include sequencing-by-synthesis (SBS) techniques.

SBS techniques generally involve the enzymatic extension of a nascent nucleic acid strand through the iterative addition of nucleotides against a template strand. In traditional methods of SBS, a single nucleotide monomer may be provided to a target nucleotide in the presence of a polymerase in each delivery. However, in the methods described herein, more than one type of nucleotide monomer can be provided to a target nucleic acid in the presence of a polymerase in a delivery.

SBS can utilize nucleotide monomers that have a terminator moiety or those that lack any terminator moieties. Methods utilizing nucleotide monomers lacking terminators include, for example, pyrosequencing and sequencing using γ-phosphate-labeled nucleotides, as set forth in further detail below. In methods using nucleotide monomers lacking terminators, the number of nucleotides added in each cycle is generally variable and dependent upon the template sequence and the mode of nucleotide delivery. For SBS techniques that utilize nucleotide monomers having a terminator moiety, the terminator can be effectively irreversible under the sequencing conditions used as is the case for traditional Sanger sequencing which utilizes dideoxynucleotides, or the terminator can be reversible as is the case for sequencing methods developed by Solexa (now Illumina, Inc.).

SBS techniques can utilize nucleotide monomers that have a label moiety or those that lack a label moiety. Accordingly, incorporation events can be detected based on a characteristic of the label, such as fluorescence of the label; a characteristic of the nucleotide monomer such as molecular weight or charge; a byproduct of incorporation of the nucleotide, such as release of pyrophosphate; or the like. In embodiments, where two or more different nucleotides are present in a sequencing reagent, the different nucleotides can be distinguishable from each other, or alternatively, the two or more different labels can be the indistinguishable under the detection techniques being used. For example, the different nucleotides present in a sequencing reagent can have different labels and they can be distinguished using appropriate optics as exemplified by the sequencing methods developed by Solexa (now Illumina, Inc.).

Preferred embodiments include pyrosequencing techniques. Pyrosequencing detects the release of inorganic pyrophosphate (PPi) as particular nucleotides are incorporated into the nascent strand (Ronaghi, M., Karamohamed, S., Pettersson, B., Uhlen, M. and Nyren, P. (1996) "Real-time DNA sequencing using detection of pyrophosphate release." Analytical Biochemistry 242(1), 84-9; Ronaghi, M. (2001) "Pyrosequencing sheds light on DNA sequencing." Genome Res. 11(1), 3-11; Ronaghi, M., Uhlen, M. and Nyren, P. (1998) "A sequencing method based on real-time pyrophosphate." Science 281(5375), 363; U.S. Pat. No. 6,210,891; U.S. Pat. No. 6,258,568 and U.S. Pat. No. 6,274,320, the disclosures of which are incorporated herein by reference in their entireties). In pyrosequencing, released PPi can be detected by being immediately converted to adenosine triphosphate (ATP) by ATP sulfurylase, and the level of ATP generated is detected via luciferase-produced photons. The nucleic acids to be sequenced can be attached to features in an array and the array can be imaged to capture the chemiluminescent signals that are produced due to incorporation of a nucleotides at the features of the array. An image can be obtained after the array is treated with a particular nucleotide type (e.g., A, T, C or G). Images obtained after addition of each nucleotide type will differ with regard to which features in the array are detected. These differences in the image reflect the different sequence content of the features on the array. However, the relative locations of each feature will remain unchanged in the images. The images can be stored, processed and analyzed using the methods set forth herein. For example, images obtained after treatment of the array with each different nucleotide type can be handled in the same way as exemplified herein for images obtained from different detection channels for reversible terminator-based sequencing methods.

In another exemplary type of SBS, cycle sequencing is accomplished by stepwise addition of reversible terminator nucleotides containing, for example, a cleavable or photobleachable dye label as described, for example, in WO 04/018497 and U.S. Pat. No. 7,057,026, the disclosures of which are incorporated herein by reference. This approach is being commercialized by Solexa (now Illumina Inc.), and is also described in WO 91/06678 and WO 07/123,744, each of which is incorporated herein by reference. The availability of fluorescentlylabeled terminators in which both the termination can be reversed and the fluorescent label cleaved facilitates efficient cyclic reversible termination (CRT) sequencing. Polymerases can also be coengineered to efficiently incorporate and extend from these modified nucleotides.

Preferably in reversible terminator-based sequencing embodiments, the labels do not substantially inhibit extension under SBS reaction conditions. However, the detection labels can be removable, for example, by cleavage or degradation. Images can be captured following incorporation of labels into arrayed nucleic acid features. In particular embodiments, each cycle involves simultaneous delivery of four different nucleotide types to the array and each nucleotide type has a spectrally distinct label. Four images can then be obtained, each using a detection channel that is selective for one of the four different labels. Alternatively, different nucleotide types can be added sequentially and an image of the array can be obtained between each addition step. In such embodiments, each image will show nucleic acid features that have incorporated nucleotides of a particular type. Different features are present or absent in the different images due the different sequence content of each feature. However, the relative position of the features will remain unchanged in the images. Images obtained from such reversible terminator-SBS methods can be stored, processed and analyzed as set forth herein. Following the image capture step, labels can be removed and reversible terminator moieties can be removed for subsequent cycles of nucleotide addition and detection. Removal of the labels after they have been detected in a particular cycle and prior to a subsequent cycle can provide the advantage of reducing background signal and crosstalk between cycles. Examples of useful labels and removal methods are set forth below.

In particular embodiments some or all of the nucleotide monomers can include reversible terminators. In such embodiments, reversible terminators/cleavable fluors can include fluor linked to the ribose moiety via a 3' ester linkage (Metzker, Genome Res. 15:1767-1776 (2005), which is incorporated herein by reference). Other approaches have separated the terminator chemistry from the cleavage of the fluorescence label (Ruparel et al., Proc Natl Acad Sci USA 102: 5932-7 (2005), which is incorporated herein by reference in its entirety). Ruparel et al described the development of reversible terminators that used a small 3' allyl group to block extension, but could easily be deblocked by a short treatment with a palladium catalyst. The fluorophore was attached to the base via a photocleavable linker that could easily be cleaved by a 30 second exposure to long wavelength UV light. Thus, either disulfide reduction or photocleavage can be used as a cleavable linker. Another approach to reversible termination is the use of natural termination that ensues after placement of a bulky dye on a dNTP. The presence of a charged bulky dye on the dNTP can act as an effective terminator through steric and/or electrostatic hindrance. The presence of one incorporation event prevents further incorporations unless the dye is removed. Cleavage of the dye removes the fluor and effectively reverses the termination. Examples of modified nucleotides are also described in U.S. Pat. No. 7,427,673, and U.S. Pat. No. 7,057,026, the disclosures of which are incorporated herein by reference in their entireties.

Additional exemplary SBS systems and methods which can be utilized with the methods and systems described herein are described in U.S. Patent Application Publication No. 2007/0166705, U.S. Patent Application Publication No. 2006/0188901, U.S. Pat. No. 7,057,026, U.S. Patent Application Publication No. 2006/0240439, U.S. Patent Application Publication No. 2006/0281109, PCT Publication No. WO 05/065814, U.S. Patent Application Publication No. 2005/0100900, PCT Publication No. WO 06/064199, PCT Publication No. WO 07/010,251, U.S. Patent Application Publication No. 2012/0270305 and U.S. Patent Application Publication No. 2013/0260372, the disclosures of which are incorporated herein by reference in their entireties.

Some embodiments can utilize detection of four different nucleotides using fewer than four different labels. For example, SBS can be performed utilizing methods and systems described in the incorporated materials of U.S. Patent Application Publication No. 2013/0079232. As a first example, a pair of nucleotide types can be detected at the same wavelength, but distinguished based on a difference in intensity for one member of the pair compared to the other, or based on a change to one member of the pair (e.g. via chemical modification, photochemical modification or physical modification) that causes apparent signal to appear or disappear compared to the signal detected for the other member of the pair. As a second example, three of four different nucleotide types can be detected under particular conditions while a fourth nucleotide type lacks a label that is detectable under those conditions, or is minimally detected under those conditions (e.g., minimal detection due to background fluorescence, etc.). Incorporation of the first three nucleotide types into a nucleic acid can be determined based on presence of their respective signals and incorporation of the fourth nucleotide type into the nucleic acid can be determined based on absence or minimal detection of any signal. As a third example, one nucleotide type can include label(s) that are detected in two different channels, whereas other nucleotide types are detected in no more than one of the channels. The aforementioned three exemplary configurations are not considered mutually exclusive and can be used in various combinations. An exemplary embodiment that combines all three examples, is a fluorescent-based SBS method that uses a first nucleotide type that is detected in a first channel (e.g. dATP having a label that is detected in the first channel when excited by a first excitation wavelength), a second nucleotide type that is detected in a second channel (e.g. dCTP having a label that is detected in the second channel when excited by a second excitation wavelength), a third nucleotide type that is detected in both the first and the second channel (e.g. dTTP having at least one label that is detected in both channels when excited by the first and/or second excitation wavelength) and a fourth nucleotide type that lacks a label that is not, or minimally, detected in either channel (e.g. dGTP having no label).

Further, as described in the incorporated materials of U.S. Patent Application Publication No. 2013/0079232, sequencing data can be obtained using a single channel. In such so-called one-dye sequencing approaches, the first nucleotide type is labeled but the label is removed after the first image is generated, and the second nucleotide type is labeled only after a first image is generated. The third nucleotide type retains its label in both the first and second images, and the fourth nucleotide type remains unlabeled in both images.

Some embodiments can utilize sequencing by ligation techniques. Such techniques utilize DNA ligase to incorporate oligonucleotides and identify the incorporation of such oligonucleotides. The oligonucleotides typically have different labels that are correlated with the identity of a particular nucleotide in a sequence to which the oligonucleotides hybridize. As with other SBS methods, images can be obtained following treatment of an array of nucleic acid features with the labeled sequencing reagents. Each image will show nucleic acid features that have incorporated labels of a particular type. Different features are present or absent in the different images due the different sequence content of each feature, but the relative position of the features will remain unchanged in the images. Images obtained from ligation-based sequencing methods can be stored, processed and analyzed as set forth herein. Exemplary SBS systems and methods which can be utilized with the methods and systems described herein are described in U.S. Pat. No. 6,969,488, U.S. Pat. No. 6,172,218, and U.S. Pat. No. 6,306,597, the disclosures of which are incorporated herein by reference in their entireties.

Some embodiments can utilize nanopore sequencing (Deamer, D. W. & Akeson, M. "Nanopores and nucleic acids: prospects for ultrarapid sequencing." Trends Biotechnol. 18, 147-151 (2000); Deamer, D. and D. Branton, "Characterization of nucleic acids by nanopore analysis". Acc. Chem. Res. 35:817-825 (2002); Li, J., M. Gershow, D. Stein, E. Brandin, and J. A. Golovchenko, "DNA molecules and configurations in a solid-state nanopore microscope" Nat. Mater. 2:611-615 (2003), the disclosures of which are incorporated herein by reference in their entireties). In such embodiments, the target nucleic acid passes through a nanopore. The nanopore can be a synthetic pore or biological membrane protein, such as α-hemolysin. As the target nucleic acid passes through the nanopore, each base-pair can be identified by measuring fluctuations in the electrical conductance of the pore. (U.S. Pat. No. 7,001,792; Soni, G. V. & Meller, "A. Progress toward ultrafast DNA sequencing using solid-state nanopores." Clin. Chem. 53, 1996-2001 (2007); Healy, K. "Nanopore-based single-molecule DNA analysis." Nanomed. 2, 459-481 (2007); Cockroft, S. L., Chu, J., Amorin, M. & Ghadiri, M. R. "A single-molecule nanopore device detects DNA polymerase activity with single-nucleotide resolution." J. Am. Chem. Soc. 130, 818-820 (2008), the disclosures of which are incorporated herein by reference in their entireties). Data obtained from nanopore sequencing can be stored, processed and analyzed as set forth herein. In particular, the data can be treated as an image in accordance with the exemplary treatment of optical images and other images that is set forth herein.

Some embodiments can utilize methods involving the real-time monitoring of DNA polymerase activity. Nucleotide incorporations can be detected through fluorescence resonance energy transfer (FRET) interactions between a fluorophore-bearing polymerase and γ-phosphate-labeled nucleotides as described, for example, in U.S. Pat. No. 7,329,492 and U.S. Pat. No. 7,211,414 (each of which is incorporated herein by reference) or nucleotide incorporations can be detected with zero-mode waveguides as described, for example, in U.S. Pat. No. 7,315,019 (which is incorporated herein by reference) and using fluorescent nucleotide analogs and engineered polymerases as described, for example, in U.S. Pat. No. 7,405,281 and U.S. Patent Application Publication No. 2008/0108082 (each of which is incorporated herein by reference). The illumination can be restricted to a zeptoliter-scale volume around a surface-tethered polymerase such that incorporation of fluorescently labeled nucleotides can be observed with low background (Levene, M. J. et al. "Zero-mode waveguides for single-molecule analysis at high concentrations." Science 299, 682-686 (2003); Lundquist, P. M. et al. "Parallel confocal detection of single molecules in real time." Opt. Lett. 33, 1026-1028 (2008); Korlach, J. et al. "Selective aluminum passivation for targeted immobilization of single DNA polymerase molecules in zero-mode waveguide nano structures." Proc. Natl. Acad. Sci. USA 105, 1176-1181 (2008), the disclosures of which are incorporated herein by reference in their entireties). Images obtained from such methods can be stored, processed and analyzed as set forth herein.

Some SBS embodiments include detection of a proton released upon incorporation of a nucleotide into an extension product. For example, sequencing based on detection of released protons can use an electrical detector and associated techniques that are commercially available from Ion Torrent (Guilford, CT, a Life Technologies subsidiary) or sequencing methods and systems described in US 2009/0026082 A1; US 2009/0127589 A1; US 2010/0137143 A1; or US 2010/0282617 A1, each of which is incorporated herein by reference. Methods set forth herein for amplifying target nucleic acids using kinetic exclusion can be readily applied to substrates used for detecting protons. More specifically, methods set forth herein can be used to produce clonal populations of amplicons that are used to detect protons.

The above SBS methods can be advantageously carried out in multiplex formats such that multiple different target nucleic acids are manipulated simultaneously. In particular embodiments, different target nucleic acids can be treated in a common reaction vessel or on a surface of a particular substrate. This allows convenient delivery of sequencing reagents, removal of unreacted reagents and detection of incorporation events in a multiplex manner. In embodiments using surface-bound target nucleic acids, the target nucleic acids can be in an array format. In an array format, the target nucleic acids can be typically bound to a surface in a spatially distinguishable manner. The target nucleic acids can be bound by direct covalent attachment, attachment to a bead or other particle or binding to a polymerase or other molecule that is attached to the surface. The array can include a single copy of a target nucleic acid at each site (also referred to as a feature) or multiple copies having the same sequence can be present at each site or feature. Multiple copies can be produced by amplification methods such as, bridge amplification or emulsion PCR as described in further detail below.

The methods set forth herein can use arrays having features at any of a variety of densities including, for example, at least about 10 features/cm2, 100 features/cm2, 500 features/cm2, 1,000 features/cm2, 5,000 features/cm2, 10,000 features/cm2, 50,000 features/cm2, 100,000 features/cm2, 1,000,000 features/cm2, 5,000,000 features/cm2, or higher.

An advantage of the methods set forth herein is that they provide for rapid and efficient detection of a plurality of target nucleic acid in parallel. Accordingly the present disclosure provides integrated systems capable of preparing and detecting nucleic acids using techniques known in the art such as those exemplified above. Thus, an integrated system of the present disclosure can include fluidic components capable of delivering amplification reagents and/or sequencing reagents to one or more immobilized DNA fragments, the system comprising components such as pumps, valves, reservoirs, fluidic lines and the like. A flow cell can be configured and/or used in an integrated system for detection of target nucleic acids. Exemplary flow cells are described, for example, in US 2010/0111768 A1 and US Ser. No. 13/273,666, each of which is incorporated herein by reference. As exemplified for flow cells, one or more of the fluidic components of an integrated system can be used for an amplification method and for a detection method. Taking a nucleic acid sequencing embodiment as an example, one or more of the fluidic components of an integrated system can be used for an amplification method set forth herein and for the delivery of sequencing reagents in a sequencing method such as those exemplified above. Alternatively, an integrated system can include separate fluidic systems to carry out amplification methods and to carry out detection methods. Examples of integrated sequencing systems that are capable of creating amplified nucleic acids and also determining the sequence of the nucleic acids include, without limitation, the MiSeqTM platform (Illumina, Inc., San Diego, CA) and devices described in US Ser. No. 13/273,666, which is incorporated herein by reference.

The sequencing system described above sequences nucleic acid polymers present in samples received by a sequencing device. As defined herein, "sample" and its derivatives, is used in its broadest sense and includes any specimen, culture and the like that is suspected of including a target. In some embodiments, the sample comprises DNA, RNA, PNA, LNA, chimeric or hybrid forms of nucleic acids. The sample can include any biological, clinical, surgical, agricultural, atmospheric or aquatic-based specimen containing one or more nucleic acids. The term also includes any isolated nucleic acid sample such a genomic DNA, fresh-frozen or formalin-fixed paraffin-embedded nucleic acid specimen. It is also envisioned that the sample can be from a single individual, a collection of nucleic acid samples from genetically related members, nucleic acid samples from genetically unrelated members, nucleic acid samples (matched) from a single individual such as a tumor sample and normal tissue sample, or sample from a single source that contains two distinct forms of genetic material such as maternal and fetal DNA obtained from a maternal subject, or the presence of contaminating bacterial DNA in a sample that contains plant or animal DNA. In some embodiments, the source of nucleic acid material can include nucleic acids obtained from a newborn, for example as typically used for newborn screening.

The nucleic acid sample can include high molecular weight material such as genomic DNA (gDNA). The sample can include low molecular weight material such as nucleic acid molecules obtained from FFPE or archived DNA samples. In another embodiment, low molecular weight material includes enzymatically or mechanically fragmented DNA. The sample can include cell-free circulating DNA. In some embodiments, the sample can include nucleic acid molecules obtained from biopsies, tumors, scrapings, swabs, blood, mucus, urine, plasma, semen, hair, laser capture micro-dissections, surgical resections, and other clinical or laboratory obtained samples. In some embodiments, the sample can be an epidemiological, agricultural, forensic or pathogenic sample. In some embodiments, the sample can include nucleic acid molecules obtained from an animal such as a human or mammalian source. In another embodiment, the sample can include nucleic acid molecules obtained from a non-mammalian source such as a plant, bacteria, virus or fungus. In some embodiments, the source of the nucleic acid molecules may be an archived or extinct sample or species.

Further, the methods and compositions disclosed herein may be useful to amplify a nucleic acid sample having low-quality nucleic acid molecules, such as degraded and/or fragmented genomic DNA from a forensic sample. In one embodiment, forensic samples can include nucleic acids obtained from a crime scene, nucleic acids obtained from a missing persons DNA database, nucleic acids obtained from a laboratory associated with a forensic investigation or include forensic samples obtained by law enforcement agencies, one or more military services or any such personnel. The nucleic acid sample may be a purified sample or a crude DNA containing lysate, for example derived from a buccal swab, paper, fabric or other substrate that may be impregnated with saliva, blood, or other bodily fluids. As such, in some embodiments, the nucleic acid sample may comprise low amounts of, or fragmented portions of DNA, such as genomic DNA. In some embodiments, target sequences can be present in one or more bodily fluids including but not limited to, blood, sputum, plasma, semen, urine and serum. In some embodiments, target sequences can be obtained from hair, skin, tissue samples, autopsy or remains of a victim. In some embodiments, nucleic acids including one or more target sequences can be obtained from a deceased animal or human. In some embodiments, target sequences can include nucleic acids obtained from non-human DNA such a microbial, plant or entomological DNA. In some embodiments, target sequences or amplified target sequences are directed to purposes of human identification. In some embodiments, the disclosure relates generally to methods for identifying characteristics of a forensic sample. In some embodiments, the disclosure relates generally to human identification methods using one or more target specific primers disclosed herein or one or more target specific primers designed using the primer design criteria outlined herein. In one embodiment, a forensic or human identification sample containing at least one target sequence can be amplified using any one or more of the target-specific primers disclosed herein or using the primer criteria outlined herein.

The components of the call recalibration system 106 can include software, hardware, or both. For example, the components of the call recalibration system 106 can include one or more instructions stored on a computer-readable storage medium and executable by processors of one or more computing devices (e.g., the client device 108). When executed by the one or more processors, the computer-executable instructions of the call recalibration system 106 can cause the computing devices to perform the bubble detection methods described herein. Alternatively, the components of the call recalibration system 106 can comprise hardware, such as special purpose processing devices to perform a certain function or group of functions. Additionally, or alternatively, the components of the call recalibration system 106 can include a combination of computer-executable instructions and hardware.

Furthermore, the components of the call recalibration system 106 performing the functions described herein with respect to the call recalibration system 106 may, for example, be implemented as part of a stand-alone application, as a module of an application, as a plug-in for applications, as a library function or functions that may be called by other applications, and/or as a cloud-computing model. Thus, components of the call recalibration system 106 may be implemented as part of a stand-alone application on a personal computing device or a mobile device. Additionally, or alternatively, the components of the call recalibration system 106 may be implemented in any application that provides sequencing services including, but not limited to Illumina BaseSpace, Illumina DRAGEN, or Illumina TruSight software. "Illumina," "BaseSpace," "DRAGEN," and "TruSight," are either registered trademarks or trademarks of Illumina, Inc. in the United States and/or other countries.

Embodiments of the present disclosure may comprise or utilize a special purpose or general-purpose computer including computer hardware, such as, for example, one or more processors and system memory, as discussed in greater detail below. Embodiments within the scope of the present disclosure also include physical and other computer-readable media for carrying or storing computer-executable instructions and/or data structures. In particular, one or more of the processes described herein may be implemented at least in part as instructions embodied in a non-transitory computer-readable medium and executable by one or more computing devices (e.g., any of the media content access devices described herein). In general, a processor (e.g., a microprocessor) receives instructions, from a non-transitory computer-readable medium, (e.g., a memory, etc.), and executes those instructions, thereby performing one or more processes, including one or more of the processes described herein.

Computer-readable media can be any available media that can be accessed by a general purpose or special purpose computer system. Computer-readable media that store computer-executable instructions are non-transitory computer-readable storage media (devices). Computer-readable media that carry computer-executable instructions are transmission media. Thus, by way of example, and not limitation, embodiments of the disclosure can comprise at least two distinctly different kinds of computer-readable media: non-transitory computer-readable storage media (devices) and transmission media.

Non-transitory computer-readable storage media (devices) includes RAM, ROM, EEPROM, CD-ROM, solid state drives (SSDs) (e.g., based on RAM), Flash memory, phasechange memory (PCM), other types of memory, other optical disk storage, magnetic disk storage or other magnetic storage devices, or any other medium which can be used to store desired program code means in the form of computer-executable instructions or data structures and which can be accessed by a general purpose or special purpose computer.

A "network" is defined as one or more data links that enable the transport of electronic data between computer systems and/or modules and/or other electronic devices. When information is transferred or provided over a network or another communications connection (either hardwired, wireless, or a combination of hardwired or wireless) to a computer, the computer properly views the connection as a transmission medium. Transmissions media can include a network and/or data links which can be used to carry desired program code means in the form of computer-executable instructions or data structures and which can be accessed by a general purpose or special purpose computer. Combinations of the above should also be included within the scope of computer-readable media.

Further, upon reaching various computer system components, program code means in the form of computer-executable instructions or data structures can be transferred automatically from transmission media to non-transitory computer-readable storage media (devices) (or vice versa). For example, computer-executable instructions or data structures received over a network or data link can be buffered in RAM within a network interface module (e.g., a NIC), and then eventually transferred to computer system RAM and/or to less volatile computer storage media (devices) at a computer system. Thus, it should be understood that non-transitory computer-readable storage media (devices) can be included in computer system components that also (or even primarily) utilize transmission media.

Computer-executable instructions comprise, for example, instructions and data which, when executed at a processor, cause a general purpose computer, special purpose computer, or special purpose processing device to perform a certain function or group of functions. In some embodiments, computer-executable instructions are executed on a general-purpose computer to turn the general-purpose computer into a special purpose computer implementing elements of the disclosure. The computer executable instructions may be, for example, binaries, intermediate format instructions such as assembly language, or even source code. Although the subject matter has been described in language specific to structural features and/or methodological acts, it is to be understood that the subject matter defined in the appended claims is not necessarily limited to the described features or acts described above. Rather, the described features and acts are disclosed as example forms of implementing the claims.

Those skilled in the art will appreciate that the disclosure may be practiced in network computing environments with many types of computer system configurations, including, personal computers, desktop computers, laptop computers, message processors, hand-held devices, multiprocessor systems, microprocessor-based or programmable consumer electronics, network PCs, minicomputers, mainframe computers, mobile telephones, PDAs, tablets, pagers, routers, switches, and the like. The disclosure may also be practiced in distributed system environments where local and remote computer systems, which are linked (either by hardwired data links, wireless data links, or by a combination of hardwired and wireless data links) through a network, both perform tasks. In a distributed system environment, program modules may be located in both local and remote memory storage devices.

Embodiments of the present disclosure can also be implemented in cloud computing environments. In this description, "cloud computing" is defined as a model for enabling on-demand network access to a shared pool of configurable computing resources. For example, cloud computing can be employed in the marketplace to offer ubiquitous and convenient on-demand access to the shared pool of configurable computing resources. The shared pool of configurable computing resources can be rapidly provisioned via virtualization and released with low management effort or service provider interaction, and then scaled accordingly.

A cloud-computing model can be composed of various characteristics such as, for example, on-demand self-service, broad network access, resource pooling, rapid elasticity, measured service, and so forth. A cloud-computing model can also expose various service models, such as, for example, Software as a Service (SaaS), Platform as a Service (PaaS), and Infrastructure as a Service (IaaS). A cloud-computing model can also be deployed using different deployment models such as private cloud, community cloud, public cloud, hybrid cloud, and so forth. In this description and in the claims, a "cloud-computing environment" is an environment in which cloud computing is employed.

FIG. 15 illustrates a block diagram of a computing device 1500 that may be configured to perform one or more of the processes described above. One will appreciate that one or more computing devices such as the computing device 1500 may implement the call recalibration system 106 and the sequencing system 104. As shown by FIG. 15, the computing device 1500 can comprise a processor 1502, a memory 1504, a storage device 1506, an I/O interface 1508, and a communication interface 1510, which may be communicatively coupled by way of a communication infrastructure 1512. In certain embodiments, the computing device 1500 can include fewer or more components than those shown in FIG. 15. The following paragraphs describe components of the computing device 1500 shown in FIG. 15 in additional detail.

In one or more embodiments, the processor 1502 includes hardware for executing instructions, such as those making up a computer program. As an example, and not by way of limitation, to execute instructions for dynamically modifying workflows, the processor 1502 may retrieve (or fetch) the instructions from an internal register, an internal cache, the memory 1504, or the storage device 1506 and decode and execute them. The memory 1504 may be a volatile or nonvolatile memory used for storing data, metadata, and programs for execution by the processor(s). The storage device 1506 includes storage, such as a hard disk, flash disk drive, or other digital storage device, for storing data or instructions for performing the methods described herein.

The I/O interface 1508 allows a user to provide input to, receive output from, and otherwise transfer data to and receive data from computing device 1500. The I/O interface 1508 may include a mouse, a keypad or a keyboard, a touch screen, a camera, an optical scanner, network interface, modem, other known I/O devices or a combination of such I/O interfaces. The I/O interface 1508 may include one or more devices for presenting output to a user, including, but not limited to, a graphics engine, a display (e.g., a display screen), one or more output drivers (e.g., display drivers), one or more audio speakers, and one or more audio drivers. In certain embodiments, the I/O interface 1508 is configured to provide graphical data to a display for presentation to a user. The graphical data may be representative of one or more graphical user interfaces and/or any other graphical content as may serve a particular implementation.

The communication interface 1510 can include hardware, software, or both. In any event, the communication interface 1510 can provide one or more interfaces for communication (such as, for example, packet-based communication) between the computing device 1500 and one or more other computing devices or networks. As an example, and not by way of limitation, the communication interface 1510 may include a network interface controller (NIC) or network adapter for communicating with an Ethernet or other wire-based network or a wireless NIC (WNIC) or wireless adapter for communicating with a wireless network, such as a WI-FI.

Additionally, the communication interface 1510 may facilitate communications with various types of wired or wireless networks. The communication interface 1510 may also facilitate communications using various communication protocols. The communication infrastructure 1512 may also include hardware, software, or both that couples components of the computing device 1500 to each other. For example, the communication interface 1510 may use one or more networks and/or protocols to enable a plurality of computing devices connected by a particular infrastructure to communicate with each other to perform one or more aspects of the processes described herein. To illustrate, the sequencing process can allow a plurality of devices (e.g., a client device, sequencing device, and server device(s)) to exchange information such as sequencing data and error notifications.

In the foregoing specification, the present disclosure has been described with reference to specific exemplary embodiments thereof. Various embodiments and aspects of the present disclosure(s) are described with reference to details discussed herein, and the accompanying drawings illustrate the various embodiments. The description above and drawings are illustrative of the disclosure and are not to be construed as limiting the disclosure. Numerous specific details are described to provide a thorough understanding of various embodiments of the present disclosure.

The present disclosure may be embodied in other specific forms without departing from its spirit or essential characteristics. The described embodiments are to be considered in all respects only as illustrative and not restrictive. For example, the methods described herein may be performed with less or more steps/acts or the steps/acts may be performed in differing orders. Additionally, the steps/acts described herein may be repeated or performed in parallel with one another or in parallel with different instances of the same or similar steps/acts. The scope of the present application is, therefore, indicated by the appended claims rather than by the foregoing description. All changes that come within the meaning and range of equivalency of the claims are to be embraced within their scope.

Aspects can be understood from the following numbered paragraphs:
1. A system comprising:
   at least one processor; and
   a non-transitory computer readable medium comprising instructions that, when executed by the at least one processor, cause the system to:
      determine sequencing metrics for nucleotide base calls of nucleotide reads corresponding to a multiallelic genomic coordinate of a sample nucleotide sequence;
      generate, utilizing a call recalibration machine learning model and based on the sequencing metrics, a set of variant call classifications comprising a reference probability of a homozygous reference genotype at the multiallelic genomic coordinate, a differing genotype probability of a genotype error at the multiallelic genomic coordinate, and a correct variant probability of a correct variant call genotype at the multiallelic genomic coordinate; and
      determine final nucleotide base calls for the multiallelic genomic coordinate based on the set of variant call classifications.
2. The system of paragraph 1, further comprising instructions that, when executed by
   the at least one processor, cause the system to:
   modify a base call quality metric or a genotype quality metric based on the set of variant call classifications; and
   generate a variant call file that includes the modified base call quality metric or the modified genotype quality metric.
3. The system of paragraph 1, further comprising instructions that, when executed by
   the at least one processor, cause the system to:
   generate updated genotype likelihoods for candidate nucleotide base calls of alleles at the multiallelic genomic coordinate; and
   generate a variant call file that includes the updated genotype likelihoods.
4. The system of paragraph 1, further comprising instructions that, when executed by the at least one processor, cause the system to determine the final nucleotide base calls for the multiallelic genomic coordinate by predicting two nucleotide bases from three or more candidate alleles at the multiallelic genomic coordinate.
5. The system of paragraph 1, further comprising instructions that, when executed by the at least one processor, cause the system to generate the reference probability by determining a probability that a genotype at the multiallelic genomic coordinate is a homozygous genotype with respect to a reference genome.
6. The system of paragraph 1, further comprising instructions that, when executed by the at least one processor, cause the system to generate the differing genotype probability by determining a probability that a predicted genotype for the multiallelic genomic coordinate is an incorrect genotype or an incorrect allele in the predicted genotype.
7. The system of paragraph 1, further comprising instructions that, when executed by the at least one processor, cause the system to generate the correct variant probability by determining a probability that a predicted genotype for the multiallelic genomic coordinate is correct as initially determined by a call generation model.
8. A computer-implemented method comprising:
   determining sequencing metrics for nucleotide base calls of nucleotide reads corresponding to a genomic coordinate of a haploid nucleotide sequence from a sample;
   generating, utilizing a call recalibration machine learning model and based on the sequencing metrics, a first genotype probability of a first genotype at the genomic coordinate and a second genotype probability of a second genotype at the genomic coordinate; and
   determining a final nucleotide base call indicating a haploid genotype for the genomic coordinate based on the first genotype probability and the second genotype probability.
9. The computer-implemented method of paragraph 8, wherein:
   generating the first genotype probability comprises utilizing a layer of the call recalibration machine learning model to modify a homozygous reference probability of a homozygous reference genotype at the genomic coordinate to generate a haploid reference probability of a reference genotype at the genomic coordinate; and
   generating the second genotype probability comprises utilizing the layer of the call recalibration machine learning model to modify a homozygous alternate probability of a homozygous alternate genotype at the genomic coordinate to generate a haploid alternate probability of an alternate genotype at the genomic coordinate.
10. The computer-implemented method of paragraph 8, wherein generating the first genotype probability and the second genotype probability comprises:
   generating, for the genomic coordinate utilizing one or more layers of the call recalibration machine learning model, a first confidence score corresponding to a first genotype, a second confidence score corresponding to a second genotype, and a third confidence score corresponding to a third genotype;
   excluding the second confidence score corresponding to the second genotype; and
   normalizing the first confidence score and the third confidence score utilizing a softmax model to generate the first genotype probability and the second genotype probability.
11. The computer-implemented method of paragraph 8, wherein determining the final nucleotide base call indicating the haploid genotype for the genomic coordinate comprises determining one of:
   a haploid alternate genotype for the genomic coordinate, a modified base call quality metric, a modified genotype metric, and a modified genotype quality metric based on determining that the second genotype probability exceeds the first genotype probability; or
   a haploid reference genotype for the genomic coordinate, a modified base call quality metric, and a modified genotype quality metric based on determining that the first genotype probability exceeds the second genotype probability.
12. The computer-implemented method of paragraph 8, further comprising:
   converting a haploid reference genotype call generated by a call generation model to a diploid homozygous reference genotype call as an input for the call recalibration machine learning model; or
   converting a haploid alternate genotype call generated by the call generation model to a diploid homozygous alternate genotype call as an input for the call recalibration machine learning model; and
   generating, utilizing the call recalibration machine learning model, the first genotype probability and the second genotype probability based further on the diploid homozygous reference genotype call or the diploid homozygous alternate genotype call.
13. The computer-implemented method of paragraph 8, further comprising downsampling diploid sequencing metrics to simulate haploid sequencing metrics corresponding to the haploid nucleotide sequence by:
   selecting a subset of diploid nucleotide reads from the sample to simulate haploid nucleotide reads; and
   selecting, based on nucleotide base calls of the subset of diploid nucleotide reads, a subset of genomic coordinates exhibiting homozygous reference genotypes or homozygous alternate genotypes as indicated by a call generation model or as indicated by a ground-truth base-call dataset.
14. The computer-implemented method of paragraph 8, wherein:
   generating the first genotype probability comprises generating a probability that the first genotype at the genomic coordinate is a haploid reference genotype; and
   generating the second genotype probability comprises generating a probability that the second genotype at the genomic coordinate is a haploid alternate genotype.
15. A non-transitory computer readable medium comprising instructions that, when executed by at least one processor, cause a computing device to:
   determine, for one or more nucleotide reads, one or more nucleotide base calls indicating a homozygous reference genotype at a genomic coordinate of a sample nucleotide sequence;
   determine sequencing metrics for the one or more nucleotide base calls corresponding to the genomic coordinate;
   generate, utilizing a call recalibration machine learning model and based on the sequencing metrics from the one or more nucleotide base calls, one or more variant call classifications indicating an accuracy of identifying a variant at the genomic coordinate; and
   determine a variant call for the genomic coordinate based on the one or more variant call classifications.
16. The non-transitory computer readable medium of paragraph 15, further comprising instructions that, when executed by the at least one processor, cause the computing device to:
   receive, from a call generation model, an indication of the homozygous reference genotype at the genomic coordinate; and
   determine the variant call for the genomic coordinate by modifying the homozygous reference genotype to a different genotype based on the one or more variant call classifications.
17. The non-transitory computer readable medium of paragraph 15, further comprising instructions that, when executed by the at least one processor, cause the computing device to determine the sequencing metrics by determining one or more of read-based sequencing metrics, externally sourced sequencing metrics, or call model generated sequencing metrics for the genomic coordinate indicated as having a homozygous reference genotype.
18. The non-transitory computer readable medium of paragraph 15, further comprising instructions that, when executed by the at least one processor, cause the computing device to:
   identify a previous homozygous reference genotype call from a call generation model for the sample nucleotide sequence at the genomic coordinate;
   identify a ground truth base call for the sample nucleotide sequence at the genomic coordinate; and
   modify the call recalibration machine learning model based on a comparison of the variant call for the genomic coordinate and the ground truth base call for the genomic coordinate.
19. The non-transitory computer readable medium of paragraph 15, further comprising instructions that, when executed by the at least one processor, cause the computing device to determine, for the genomic coordinate, one of:
   a homozygous alternate genotype based on determining that a homozygous alternate classification has a highest probability from among the one or more variant call classifications;
   a heterozygous genotype based on determining that a heterozygous genotype classification has the highest probability from among the one or more variant call classifications; or
   a homozygous reference genotype based on determining that neither the homozygous alternate classification nor the heterozygous genotype classification has the highest probability from among the one or more variant call classifications.
20. The non-transitory computer readable medium of paragraph 15, further comprising instructions that, when executed by the at least one processor, cause the computing device to update one or more of a call quality field, a genotype field, or a genotype quality field corresponding to a variant call file based on the one or more variant call classifications.

## Claims

1. A computer-implemented method comprising:
determining sequencing metrics for nucleotide base calls of nucleotide reads corresponding to a genomic coordinate of a haploid nucleotide sequence from a sample;
generating, utilizing a call recalibration machine learning model to process the sequencing metrics, a set of variant call classifications comprising:
a first genotype probability of a haploid reference genotype, with respect to a reference genome, at the genomic coordinate; and
a second genotype probability of a haploid alternate genotype, with respect to the reference genome, at the genomic coordinate; and
determining a final nucleotide base call indicating a haploid genotype for the genomic coordinate based on the first genotype probability and the second genotype probability.

2. The computer-implemented method of claim 1, wherein:
generating the first genotype probability comprises utilizing a layer of the call recalibration machine learning model to modify a homozygous reference probability of a homozygous reference genotype at the genomic coordinate to generate a haploid reference probability of the haploid reference genotype at the genomic coordinate; and
generating the second genotype probability comprises utilizing the layer of the call recalibration machine learning model to modify a homozygous alternate probability of a homozygous alternate genotype at the genomic coordinate to generate a haploid alternate probability of the haploid alternate genotype at the genomic coordinate.

3. The computer-implemented method of claim 1 or 2, wherein generating the first genotype probability and the second genotype probability comprises:
generating, for the genomic coordinate utilizing one or more layers of the call recalibration machine learning model, a first confidence score corresponding to a first genotype, a second confidence score corresponding to a second genotype, and a third confidence score corresponding to a third genotype;
excluding the second confidence score corresponding to the second genotype; and
normalizing the first confidence score and the third confidence score utilizing a softmax layer to generate the first genotype probability and the second genotype probability.

4. The computer-implemented method of any one of claims 1-3, wherein determining the final nucleotide base call indicating the haploid genotype for the genomic coordinate comprises determining one of:
the haploid alternate genotype for the genomic coordinate, a modified base call quality metric, a modified genotype metric, and a modified genotype quality metric based on determining that the second genotype probability exceeds the first genotype probability; or
the haploid reference genotype for the genomic coordinate, a modified base call quality metric, and a modified genotype quality metric based on determining that the first genotype probability exceeds the second genotype probability.

5. The computer-implemented method of any one of claims 1-4, further comprising:
converting a haploid reference genotype call generated by a call generation model to a diploid homozygous reference genotype call as an input for the call recalibration machine learning model; or
converting a haploid alternate genotype call generated by the call generation model to a diploid homozygous alternate genotype call as an input for the call recalibration machine learning model; and
generating, utilizing the call recalibration machine learning model, the first genotype probability and the second genotype probability based further on the diploid homozygous reference genotype call or the diploid homozygous alternate genotype call.

6. The computer-implemented method of any one of claims 1-5, further comprising downsampling diploid sequencing metrics to simulate haploid sequencing metrics corresponding to the haploid nucleotide sequence by:
selecting a subset of diploid nucleotide reads from the sample to simulate haploid nucleotide reads; and
selecting, based on nucleotide base calls of the subset of diploid nucleotide reads, a subset of genomic coordinates exhibiting homozygous reference genotypes or homozygous alternate genotypes as indicated by a call generation model or as indicated by a ground-truth base-call dataset.

7. The computer-implemented method of claim 6, further comprising selecting, to simulate the haploid sequencing metrics corresponding to the haploid nucleotide sequence, the diploid sequencing metrics corresponding to the subset of genomic coordinates exhibiting homozygous reference genotypes or homozygous alternate genotypes.

8. The computer-implemented method of any one of claims 1-7, wherein the haploid nucleotide sequence comprises a sequence of one or more nucleotide bases from a haploid chromosome or a single chromosome without a counterpart chromosome.

9. The computer-implemented method of any one of claims 1-8, wherein the haploid nucleotide sequence comprises a sequence of one or more nucleotide bases from a sex chromosome.

10. The computer-implemented method of any one of claims 1-9, wherein:
determining the sequencing metrics comprises determining one or more of read-based sequencing metrics, externally sourced sequencing metrics, or call model generated sequencing metrics for the nucleotide base calls of nucleotide reads corresponding to the genomic coordinate of the haploid nucleotide sequence; and
generating the set of variant call classifications comprises utilizing the call recalibration machine learning model to process the one or more of the read-based sequencing metrics, the externally sourced sequencing metrics, or the call model generated sequencing metrics to generate the set of variant call classifications.

11. The computer-implemented method of claim 10, wherein:
the read-based sequencing metrics comprise sequencing metrics derived from nucleotide reads of the sample;
the externally sourced sequencing metrics comprise sequencing metrics stored in one or more databases external to a system executing the computer-implemented method; and
the call model generated sequencing metrics comprise sequencing metrics generated by a call generation model.

12. The computer-implemented method of any one of claims 1-11, wherein to determine the final nucleotide base call by modifying an initial nucleotide base call generated by a call generation model for a variant call file.

13. The computer-implemented method of any one of claims 1-12, wherein:
generating the first genotype probability comprises utilizing a layer of the call recalibration machine learning model to modify a homozygous reference probability of a homozygous reference genotype at the genomic coordinate to generate a haploid reference probability of a reference genotype at the genomic coordinate; and
generating the second genotype probability comprises utilizing the layer of the call recalibration machine learning model to modify a homozygous alternate probability of a homozygous alternate genotype at the genomic coordinate to generate a haploid alternate probability of an alternate genotype at the genomic coordinate.

14. A system comprising at least one processor and a non-transitory computer readable medium comprising instructions that, when executed by the at least one processor, cause the system to perform a method according to any one of claims 1-13.

15. A non-transitory computer-readable medium storing instructions that, when executed by at least one processor, cause a system to perform a method according to any one of claims 1-13.
